# EUROPEAN PATENT SPECIFICATION

(11) **EP 3 079 679 B1**
(45) Date of publication and mention of the grant of the patent: **14.08.2019**
(21) Application number: 14869892.1
(22) Date of filing: 12.12.2014
(51) Int. Cl.: A61K 31/122, A61K 31/045, A61K 31/22, A61P 17/00, A61P 17/14, A61Q 7/00, A61K 8/34, A61K 8/35, A61K 8/37

(54) **METHOD OF TREATMENT OF ALOPECIA WITH MONOTERPENOIDS**
VERFAHREN ZUR BEHANDLUNG VON ALOPECIA MIT MONOTERPENOIDEN
PROCÉDÉ DE TRAITEMENT DE L'ALOPÉCIE À L'AIDE DE MONOTERPÉNOÏDES

(30) Priority: 12.12.2013 AU 2013904859
(43) Date of publication of application: 19.10.2016
(73) Proprietor: Cellmid Limited, Sydney, New South Wales 2000 (AU)
(72) Inventor: YAMAMOTO, Masakuni, Kashiwa, Chiba 2770882 (JP); NAMEKATA, Masato, Kashiwa, Chiba 2770882 (JP); YAMAUCHI, Koichi, Kashiwa, Chiba 2770882 (JP); MATSUMOTO, Takeo, Kashiwa, Chiba 2770882 (JP); JONES, Darren, Ross, Avalon Beach, New South Wales 2107 (AU); HALASZ, Maria, Sydney, New South Wales 2000 (AU)
(74) Representative: Leonard, Thomas Charles
(86) International application number: PCT/AU2014/050421
(87) International publication number: WO 2015/085373

(56) References cited:
- EP-A1- 0 495 684
- EP-A2- 0 380 047
- EP-B1- 0 211 392
- WO-A1-99/20232
- WO-A2-2008/036054
- WO-A2-2011/082235
- FR-A1- 2 875 403
- US-A- 5 308 873
- US-A1- 2013 018 109
- DATABASE WPI Week 200409 Thomson Scientific, London, GB; AN 2004-085311 XP002769603, -& JP 2004 002258 A (NOEVIR KK) 8 January 2004 (2004-01-08)
- TETSUO MAEDA ET AL: "Sanguisorba Officinalis Root Extract Has FGF-5 Inhibitory Activity and Reduces Hair Loss by Causing Prolongation of the Anagen Period", NISHI NIHON HIFUKA - NISHINIHON JOURNAL OF DERMATOLOGY, vol. 69, no. 1, 27 September 2007 (2007-09-27), pages 81-86, XP55367322, JP ISSN: 0386-9784, DOI: 10.2336/nishinihonhifu.69.81
- DOMINIC BURG ET AL: "Promotion of anagen, increased hair density and reduction of hair fall in a clinical setting following identification of FGF5-inhibiting compounds via a novel 2-stage process", CLINICAL, COSMETIC AND INVESTIGATIONAL DERMATOLOGY, vol. Volume 10, 1 February 2017 (2017-02-01), pages 71-85, XP55367253, DOI: 10.2147/CCID.S123401
- DATABASE WPI Derwent Publications Ltd., London, GB; AN 2006-265891, XP055349760 'COMPOSITION, USEFUL IN PHARMACEUTICS/COSMETICS, COMPRISES E.G. ETHANOL, ETHYL ACETATE, EUCALYPTOL, GLYCOL DIPROPYLENE, LINALOL (ACETATE), ITACONIC/METHYL SUCCINIC ANYDRIDE, CAMPHOR, GERANYL FORMATE, COUMARIN AND DIETHYL PHTHALATE' & FR 2 875 403 A1 (BASRI, F. AND KADRI, A.) 24 March 2006
- RUSTY BALLS: 'Natural Australian Soaps and Oils - Tallawang Plantation :: Our Oils' BROAD LEAF PEPPERMINT OIL 12 August 2013, XP008183829 Retrieved from the Internet: <URL:http://web.archive.org/web/20130812080 352/ http://rustyballs_com.au/ouroils.html> [retrieved on 2015-02-16]

## Description

### RELATED APPLICATION DATA

This application claims priority from Australian Provisional Application No. 2013904859 filed on 12 December 2013.

### FIELD OF THE INVENTION

The present application relates to the field of hair loss and/or hair growth, such as the production and use of cosmetics for prevention or treatment of hair loss or the promotion or enhancement of hair growth, and the production and use of medicaments for therapy of alopecia.

### BACKGROUND TO THE INVENTION

### Hair and hair development

Hair is integral to our body image and can have a profound influence on our self-esteem and self-confidence. The appearance altering effects of hair loss and/or hair thinning can have a significant impact on an individual's psychological well-being and quality of life. In this regard, alopecia, and particularly androgenic alopecia, is a source of low self-esteem and anxiety for many sufferers. Hair is an important facet of the human appearance that is commonly used for recognition and is one determinant of physical attractiveness. In both Western and Asian cultures, voluminous thick hair is a symbol of health, youthfulness and virility. As a consequence, the loss of one's hair can diminish body image satisfaction and have deleterious effects on self-esteem *e.g.,* Cash TF, British Journal of Dermatology, 141:398-405, 1999. Those suffering from hair loss often experience embarrassment and fear being ridiculed by others because they look different. In some subjects, alopecia may lead to depression. These factors, coupled with society's current emphasis on youthfulness, have only served to strengthen the value of abundant hair, and, as a result, products and services promoting hair growth, replacement or fuller appearance of hair have proliferated.

The hair of non-human mammals is commonly referred to as "fur". Unless specifically stated otherwise, or the context requires otherwise, the term "hair" as used herein shall be taken to include "fur". The term "hair" shall also be taken to include hair on any part of a mammalian body, including the eyebrow, edge of the eyelid, armpit, and inside of the nostril, unless the context requires otherwise. Thus, hair may include head hair, eyebrow hair, eyelash, cilia, or other body hair.

Each hair comprises two structures: the shaft and the follicle. The primary component of the hair shaft is keratin. The hair shaft contains three layers of keratin, however the inner layer *i.e.,* the medulla, may not be present. The middle layer *i.e.,* the cortex, makes up the majority of the hair shaft. The outer layer *i.e.,* the cuticle, is formed by tightly-packed scales in an overlapping structure. Pigment cells are distributed throughout the cortex and medulla giving the hair its characteristic colour. The follicle contains several layers. At the base of the follicle is a projection called a papilla, which contains capillaries, or tiny blood vessels, that feed the cells. The living part of the hair, the area surrounding the papilla called the bulb, is the only part fed by the capillaries. The cells in the bulb divide every 23 to 72 hours, faster than any other cells in the body. The follicle is surrounded by an inner root sheath and an outer root sheath. These two sheaths protect and mould the growing hair shaft. The inner root sheath follows the hair shaft and ends below the opening of a sebaceous (oil) gland, which produces sebum, and sometimes an apocrine (scent) gland. The outer root sheath continues all the way up to the sebaceous gland. An erector pili muscle attaches below the sebaceous gland to a fibrous layer around the outer sheath. When this muscle contracts, it causes the hair to stand up.

Human skin comprises two types of hair: vellus hair and terminal hair. Vellus hair is short, fine, "peach fuzz" body hair. It is a very soft, generally pale, and short hair that grows in most places on the human body in both sexes. Vellus hair is generally less than two centimetres in length, and the follicles from which vellus hair grows are not connected to sebaceous glands. It is observed most easily in those having less terminal hair to obscure it, such as women and children. It is also found in pre-adolescents and in males exhibiting male-pattern baldness. Terminal or "androgenic" hair is developed hair, which is generally longer, coarser, thicker and darker than vellus hair. Phases of growth for terminal hair are also more apparent than for vellus hair, by virtue of a generally-longer anagen phase. Terminal hair has associated sebaceous glands. In puberty, some vellus hair may develop into terminal hair. Under other conditions, such as male pattern baldness, terminal hair may revert to a vellus-like state.

The hair growth cycle in mammals is composed of three sequential phases: anagen, catagen and telogen.

Anagen is the active growth phase of the cycle during which time cells in the root of the hair are dividing rapidly, follicles grow, hair synthesis takes place and skin thickness increases. Anagen can be further divided into six short subphases *i.e*., anagen I-VI. During the anagen phase, hairs are anchored deeply into the subcutaneous fat and therefore cannot be pulled out easily. As new hair is synthesized, the club hair is pushed up the follicle, and eventually out from the skin. During this phase active growth phase, the hair grows at a rate of about 1 cm every 28 days. Scalp hair stays in this active phase of growth for approximately 2-6 years. Human subjects that have difficulty growing their hair beyond a certain length may have a shortened anagen phase, whereas those having an ability to grow longer hair quickly may have a longer anagen phase. In humans, the hair on the arms, legs, eyelashes, and eyebrows generally has a short anagen compared to head or scalp hair.

The catagen phase is a transitional stage that lasts for about 2-3 weeks in humans, during which time growth stops, thereby forming "club" hair, follicles regress and skin thickness decreases.

Telogen is the final phase of the hair growth cycle during which both follicles and skin are at rest, lasting for about 100 days for scalp hair and much longer for other body hair. During telogen, the hair follicle is at rest, the club hair is formed, and compared to hair in anagen, the hair in telogen is located higher in the skin and can be pulled out more readily. The root of telogen hair comprises a visible solid, hard, dry, and white material. Shedding of hair in the telogen phase is normal, and up to 75 hairs in telogen are shed from a normal human scalp daily. However, hairs are typically replaced at a rate similar to that at which they are shed, because about the same number of follicles enter the anagen phase daily. At any given time, approximately 80% to 90% of follicles in a normal scalp are in the anagen phase, about 1% to 3% are in the catagen phase *i.e.,* undergoing involution, and about 5% to 10% are in the telogen phase.

The hair growth cycle is known to be regulated by a variety of mediators, including several members of the fibroblast growth factor (FGF) family. Of the 22 known members of the FGF gene family, FGF-1, FGF-2, FGF-5, FGF-7, FGF-13, FGF-18 and FGF-22 are expressed in the epithelium of the hair follicle and are thought to be involved in the hair growth cycle. For example, FGF-1, FGF-2 and FGF-7 are reported to be involved in cell proliferation in the hair follicle and agonists of these molecules have been proposed for used in hair growth products.

### Conditions of hair loss and/or reduced hair growth

As used herein, the term "alopecia" refers to a medical condition or pathology in which hair loss or hair thinning occurs by virtue of a reduced ability to replace shed hairs or as a result of a medical condition or pathology that results in enhanced hair shedding without concomitant or subsequent replacement thereof *e.g.,* brittle hair growth, thin hair growth, short hair growth, sparse hair growth, alopecia, or hair de-pigmentation. For example, the hair cycle can become uncontrolled leading to accelerated hair loss, which may be temporary or permanent.

Alopecia can have various causes, including androgenic alopecia (also referred to as male or female pattern hair loss), acute alopecia, and alopecia areata including alopecia totalis and alopecia universalis.

Androgenic alopecia is the most common form of alopecia. Androgenic alopecia is a hereditary hair-loss condition affecting men and women of, for example, Caucasion or Asian descent. Androgenic alopecia is characterised by a progressive decrease in hair volume, or even baldness. Without treatment, the number of hairs on a sufferer of androgenic alopecia will decrease at a rate of approximately 5% per year after onset *e.g.,* Ellis et al, Expert Reviews in Molecular Medicine, 4:1-11, 2002. Androgenic alopecia is so common it is reported to affect up to 70% of the general population, with an estimated 30% of men developing androgenic alopecia by the age of 30, and 50% of men affected by the age of 50. In fact, it is reported that fewer than 15% of the male population have little or no baldness by the age of 70 *e.g.,* Sinclair R, JMHG, 1(4):319-327, 2004; Lee and Lee, Ann. Dermatol., 24(3):243-252, 2012. As many as 10% of pre-menopausal women are reported to exhibit some evidence of androgenic alopecia (also referred to as female pattern hair loss), and the incidence of androgenic alopecia in women increases significantly as women enter menopause. For instance, as many as 50-75% of women aged 65 years or older are or will be affected by androgenic alopecia *e.g.,* Norwood OT, Dermatol Surg., 27(1):53-4, 2001.

Whilst the onset and physical manifestation of androgenic alopecia varies quite significantly among individuals, at least in males, its pathogenesis is thought to commence after puberty when there are sufficient circulating androgens. For example, dihydrotestosterone (DHT) is produced by the action of 5α-reductase on testosterone, and binds to androgen receptors (AR) in the dermal papilla of sensitive hair follicle of the scalp inducing growth factor beta (TGF-β), and results in cyclical miniaturization (shrinkage) of the entire hair follicle. Hair produced from miniaturized hair follicles is generally short and fin compared to hair produced from normal hair follicles and therefore provides less complete scalp coverage.

In contrast, androgen stimulation of facial dermal papillae cells produces insulin-like growth factor-2 (IGF-2), resulting in cyclical enlargement of the entire hair follicle. As a consequence, hair produced from follicles that have undergone cyclical enlargement is generally longer and thicker compared to hair produced from normal hair follicles and provides more complete facial skin coverage.

Acute alopecia is hair loss associated with an acute event, such as pregnancy, severe illness, treatment *e.g.,* such as by chemotherapy, stress, severe malnutrition, iron deficiency, hormonal disorders, AIDS, or acute irradiation. For example, treatment with chemotherapeutic agents, radiotherapeutic agents, and other medicinal products may induce necrosis or apoptosis of the follicle as a side-effect of the therapy, thereby preventing the follicle from entering anagen. Examples of agents which are known to induce temporary or permanent alopecia include alkylating agents *e.g.,* temozolomide, busulfan, ifosamide, melphalan hydrochloride, carmustine, lomustine or cyclophosphamide, and antimetabolites *e.g.,* 5-fluorouracil, capecitabine, gemcitabine, floxuridine, decitabine, mercaptopurine, pemetrexed disodium, methotrexate or dacarbazine, and natural products *e.g.,* vincristine, vinblastine, vinorelbine tartrate, paclitaxel, docetaxel, ixabepilone, daunorubicin, epirubicin, doxorubicin, idarubicin, mitoxantrone, mitomycin, dactinomycin, irinotecan, topotecan, etoposide, teniposide, etoposide phosphate, or bleomycin sulfate, and biologics *e.g.,* filgrastim, pegfilgrastim, bevacizumab, sargramostim or panitumumab, and hormones or hormone-related agents *e.g.,* megestrol acetate, fluoxymesterone, leuprolide, octreotide acetate, tamoxifen citrate or fluxymesterone, and other therapeutic agents *e.g.,* sorafenib, erlotinib, oxaliplatin, dexrazoxane, anagrelide, isotretinoin, bexarotene, vorinostat. adriamycin, cytoxan, taxol, leucovorin, oxaliplatin, and combinations of the foregoing agents.

Alopecia areata is a common cause of non-scarring alopecia that occurs in a patchy, confluent or diffuse pattern on one or more sites of the body. Alopecia areata is thought to be T-cell mediated autoimmune condition and has a reported incidence of 0.1-0.2% in the general population with a lifetime risk of 1.7% in both men and women alike *e.g.,* Amin SS and Sachdeva S, JSSDDS, 17(2):37-45, 2013. In approximately 1-2% of cases, the condition can spread to the entire scalp (Alopecia totalis) or to the entire epidermis (Alopecia universalis).

Mechanistically, in all forms of alopecia, hair loss is directly-related to a reduced ability, slowing or failure of the follicle to enter the anagen phase, or a failure to maintain the follicle in the anagen phase, such that formation of a hair shaft reduces, is slowed or ceases altogether. Hair may move into the catagen phase before sufficient growth is achieved in the anagen phase, thus becoming in a sustained manner short and thin (i.e. "hair thinning"). Chemotherapeutic agents, radiotherapeutic agents, and other medicinal products may induce necrosis or apoptosis of the follicle as a side-effect of the therapy, also preventing the follicle from entering anagen. For example, alkylating agents, antimetabolites, natural products, biologics, hormones or hormone-related agents, other therapeutic agents, and combinations thereof are known to induce temporary or permanent acute alopecia.

### Animal models of alopecia

There are several models of alopecia in humans that have been acknowledged in the art for use in testing efficacy of alopecia remedies and other hair growth-promoting therapies.

For example, the stumptailed macaque possesses hereditary balding characteristics similar in many respects to that of androgenic alopecia in humans, is used to obtain a morphometric assessment of the rate of cyclic change of the hair follicle, including rates of cyclic progression (resting to regrowing phase, and regrowing to late anagen phase) and overall changes in follicular size. These primates are also reasonably good predictors of compound efficacy, and for example, have been employed to test efficacy of minoxidil on androgenic alopecia. Cessation of topical minoxidil treatment resulted in a renewal of the balding process, with folliculograms demonstrating increases in the proportion of resting follicles. This withdrawal from treatment apparently had no effect on hair regrowth during subsequent reapplications of minoxidil. Such treatment resulted in regrowth similar to that in the first treatment phase. Continuous treatment of topical minoxidil for 4 years has not resulted in systemic or local side effects in these animals. *See e.g.,* Brigham et al., Clin. Dermatol. 6, 177-187,1998; Sundberg et al., Exp. Mol. Pathol. 67, 118-130 (1999)).

In addition to the stumptailed macaque, Crabtree and colleagues recently reported the first rodent model of androgenic alopecia *e.g.,* Crabtree et al., Endocrinology, 151(5):2373-2380, 2010. In this study, transgenic mice overexpressing human AR in the skin under control of the keratin 5 promoter were exposed to high levels of 5-alpha-dihydrotestosterone and showed delayed hair regeneration, mimicking the androgenic alopecia scalp. Crabtree and colleagues also demonstrated that the androgenic alopecia of the scalp was androgen receptor (AR) mediated, because treatment of the mice with the AR antagonist hydroxyflutamide inhibited the effect of dihydrotestosterone on hair growth.

Collectively, the findings obtained from studies on mouse models support the concept of alopecia areata as an autoimmune disease, and several rodent models with spontaneous and induced alopecia areata have been identified. For example, the Dundee Experimental Bald Rat (DEBR) was the first rodent model validated that developed spontaneous alopecia areata and is utilized to identify candidate alopecia areata susceptibility gene loci (Michie et al., Br J Dermatol., 125, 94-100, 1991). The most extensively-characterized and readily-accessible alopecia areata model is the C3H/HeJ mouse model (Sundberg et al., J Invest Dermatol., 102, 847-56, 1994). Aging C3H/HeJ mice (females at 3-5 months of age or older and males at more than 6 months of age) develop histopathological and immunohistochemical features of human alopecia areata. Alopecia develops diffusely or in circular areas on the dorsal surface of sufficiently-aged animals. Histologically, the changes in this non-scarring alopecia appear limited to anagen follicles surrounded by mononuclear cells composed primarily of cytotoxic or cytostatic (CD8+) and helper (CD4+) T cells, this is associated with follicular and hair shaft dystrophy. Pedigree tracing of affected C3H/HeJ mice suggests that this non-scarring alopecia may be an inherited and complex polygenic disease with a female predominance at younger ages. C3H/HeJ mice with alopecia areata can be used to study the efficacy of current treatments of alopecia areata, to study the effectiveness and safety profile of new treatment forms in established alopecia areata, and to assess the influence of various factors on the development of alopecia areata in order to prevent the onset of the disease.

Paus et al., Am. J. Pathol. 144, 719-734 (1994) disclose a rodent model of acute alopecia. In this model, alopecia is induced by a single intraperitoneal injection of cyclophosphamide to C57 BL/6 mice. In depilated C57 BL/6 mice, the hair follicles are synchronized to anagen. By day 9 after depilation, all follicles are mature anagen VI follicles, and the skin is characterized by grey-to-black coloured hair shafts. Histologically, macroscopically, and functionally, depilation-induced anagen VI follicles are indistinguishable from spontaneously-developing anagen follicles. Around day 16 after depilation, follicle regression occurs without loss of hair shafts in the depilated animals, and skin colour converts from black to pink, indicating both induction of catagen and cessation of melanogenesis. The development of catagen follicles is indicated macroscopically by a change in skin color from black to light grey, and occurs in large waves appearing in the neck region first and then the flanks and tail regions. At day 20 after depilation, all follicles enter telogen again, characterized by change in skin color from grey to pink. When cyclophosphamide is administered to C57 BL/6 mice on day 9 after depilation, the animals show rapid and reproducible visible signs of acute alopecia dose-dependent, including significant loss of fur and premature termination of anagen characterized by depigmentation leading to a grey skin appearance by day 12-14. Thus, follicles of the neck region are generally in catagen 3-5 days after cyclophosphamide treatment. Hair shafts on the backs of animals are also removed easily by rubbing at days 12-14, and by day 15, as much as 60% of the dorsal surface may be exhibit alopecia. The color change and alopecia induced by cyclophosphamide reflect the induction of dystrophic forms of anagen and catagen in anagen VI follicles. In cyclophosphamide-treated animals, follicles also progress to telogen rapidly, as evidenced by pink skin, and rapid loss of fur due to damage of the hair follicle. Telogen is shortened following cyclophosphamide treatment, and normal telogen hair follicles enter the next hair cycle, so that animals develop new hair shafts on days 16-20 *i.e.,* within about 7-10 days following treatment. These new hair shafts are often de-pigmented due to the presence of dystrophic anagen follicles that have not had time to produce new, normally-pigmented hair shafts. Later, pigmented hair shafts develop.

### Therapy for alopecia

Products that claim to be useful for treating hair loss target a steadily growing, multi-billion dollar market worldwide. Existing therapies for alopecia include topical minoxidil and derivatives thereof *e.g.,* U.S. Pat. Nos. 4,139,619 and 4,596,812, and European Pat. Nos. EP-0353123, EP-0356271, EP-0408442, EP-0522964, EP-0420707, EP-0459890 and EP-0519819, spironolactone, cyproterone acetate, flutamide, finasteride, progesterone or estrogen. However, none of those agents are broadly applicable for all forms of alopecia, nor are they uniformly dependable for all patients.

Based on the fact that androgenic alopecia is the most commonly reported form of hair loss, there have been many attempts to discover effective agents for treatment of this condition. Notwithstanding the large number of advertised 'anti-hair loss' agents on the market, convincing evidence-based medicine is still the exception rather than the rule in this field, and currently, only monoxidil and finasteride are known to be effective for treating androgenic alopecia, and only topical monoxidil and oral finasteride formulations (for males only) have been approved by the United States Food and Drug Association (FDA). However, even these agents have their own shortcomings.

Minoxidil is a vasodilator which was originally used to treat hypertension. However, following observations that patients treated with minoxidil showed increased hair growth, a topical formulation was developed for treatment of hair loss. Although a mechanism of action of minoxidil is not fully understood, minoxidil has is postulated to (i) arrest hair loss by prolonging the anagen growth phase of terminal hair leading to a decrease in hair shedding, and (ii) stimulate hair growth by increasing cutaneous blood flow to the scalp *e.g.,* Kwack et al., Journal of Dermatological Science, 62(3):154-159, 2011; Buhl et al., The Journal of Investigative Dermatology, 92(3):315-320, 1989. Whilst minoxidil has demonstrated some efficacy in promoting hair growth, it does not inhibit the biological process of hair loss, and upon cessation of topical minoxidil treatment, hair shedding rapidly resumes, including the loss of any minoxidil-stimulated hair. For this reason, patients treated with topical minoxidil require frequent dosing to achieve an effective outcome *e.g.,* twice-daily at 2% concentration. Minoxidil is also considered to be effective in less than 60% of patients, and there is currently no indication as to which patients are most likely to respond. Minoxidil also has a number of undesirable side-effects. For example, irritation of the scalp, including dryness, scaling, itching, and redness, is reported to occur in approximately 7% of patients using the 2% solution and in more of those using the 5% solution because of its higher content of propylene glycol. Minoxidil may also cause allergic contact dermatitis or photoallergic contact dermatitis. Hypertrichosis is another dermatologic adverse effect reported in subjects using minoxidil which is thought to be caused by increase cutaneous blood flow thereby increasing nutrients, blood and oxygen to the follicles *e.g.,* Price VH, New England Journal of Medicine, 341(13):964-973, 1999; Rossie et al., Recent Patents on Inflammation & Allergy Drug Discovery, 6(2):130-136, 2012.

Finasteride is a selective inhibitor of 5-alpha reductase of type II, which reduces conversion of testosterone into DHT. Notwithstanding that finasteride provides an advance over minoxidil in beingdeliverable orally, approximately 35% or more of balding male recipients show poor or no response to finasteride treatment. Furthermore, because finasteride is used for systemic therapy in males, DHT production is reduced systematically in tissues and serum. As a consequence, systemic inhibition of 5-alpha reductase during finasteride treatment can produce significant side-effects in some users, including erectile dysfunction, impotence, low libido, or gynecomestica after using that drug *e.g.,* Price VH, New England Journal of Medicine, 341(13):964-973, 1999. In those males suffering such side-effects, the side effects may not disappear after ceasing finasteride. Finasteride is also costly to produce.

Other experimental agents, including various prostaglandin analogs, have also been disclosed for use in treatment of alopecia *e.g.,* travoprost and voprostol. However, most of these drug require frequent administration *e.g.,* at least daily. For example, prostaglandin analogues, which are have been used to treat eyelash hypotrichosis with some success, have been proposed for treating alopecia. However, topical applications of prostaglandins have not proved efficacious *e.g.,* Atanaskova et al., Dermatologic Clinics, 31(1):119-127, 2013. Botulinum toxins have also been introduced for treatment of hair loss with some success, resulting in reduced hair loss, and in some cases, increased hair growth. However, little data on the effectiveness of these emerging agents in treating hair loss are currently available.

Herbal cosmetics are also finding increasing popularity, and approximately 1000 types of plant extracts are reported to have been examined with respect to hair growth *e.g.,* Rathi et al., Pharmacognosy Reviews, 2:185-187, 2008. For example, procanthocyanidins extracted from grape seeds have been reported to induce hair growth *e.g.,* Takahashi et al., Acta Dermato-Venereologica, 78:428-432, 1998.

There is a need for cosmetic and medical products for treatment and prevention of hair loss and for the treatment of pathological conditions of hair loss such as alopecia.

The following publications provide conventional techniques of molecular biology. Such procedures are described, for example, in the following texts:
1. Remington 's Pharmaceutical Sciences, 21th Ed. Philadelphia, PA: Lippincott Williams & Wilkins, 2005

### SUMMARY OF THE INVENTION

The subject matter for which protection is sought is as defined by the claims.
In work leading up to the present invention, the inventor sought to identify compounds *e.g.,* for topical administration to a subject, capable of reducing FGF-5-dependent signalling in a hair follicle or part thereof, and/or which are capable of preventing and/or reducing and/or inhibiting FGF-5 binding to its cognate receptor, FGFR1. The inventors hypothesized that such compounds would be useful for reducing and/or preventing loss or thinning of terminal hair associated with FGF-5 signalling in the hair follicle. This work was based on the recognition by the inventors that FGF-5 is important for transition of a hair follicle from anagen to catagen during the normal growth cycle and that FGF-5-signalling in the hair follicle can cause hair loss or hair thinning by decreasing proliferation of outer root sheath cells, suppressing dermal papillae cell activation during anagen and inducing onset of catagen.

The inventors reasoned that compounds identified as being capable of reducing FGF-5-dependent signalling in a hair follicle or part thereof and/or which are capable of preventing and/or reducing and/or inhibiting FGF-5 binding to its cognate receptor, FGFR1, could be administered as part of a topical formulation to a reduce, delay or prevent loss of terminal hair caused by FGF-5 signalling in the hair follicle, such as in subjects suffering from, or having a propensity to, develop alopecia.

The inventors screened synthetic and naturally-occurring compounds using a FR-Ba/F3 cell-based screening assay, *e.g.,* Ito et al., Journal of Cellular Physiology, 197:273-283, 2003. The inventors also used a dermal papilla Alkaline Phosphatase (DP-ALP) cell-based screening assay as disclosed in WO2013/105417 to validate the FGF-5-inhibitory activity of monoterpenoids identified in the primary FR-Ba/F3 cell-based screening assay as inhibiting FGF-5-dependent signalling.

The data provided herein show that certain monoterpenoid compounds derived from plant extracts exhibit an inhibitory activity on proliferation and/or viability of FGF-5-dependent FR-BaF3 cells cultured in the presence of FGF-5. Because FR-BaF3 cells are dependent on FGF-5 for viability and proliferation, the observed reduction in cell proliferation and viability indicates the ability of the monoterpenoid compound(s) to inhibit and/or prevent and/or reduce FGF-5 dependent signalling in those cells. The data provided herein also show that a subset of monoterpenoid compounds that modulate FGF-5-dependent signalling in the FR-BaF3 cell assay are also capable of increasing or enhancing alkaline phosphatase (ALP) activity in dermal papilla (DP) cells treated with FGF-5. Collectively, these data support the conclusion that monoterpenoid compounds of the invention are effective inhibitors of FGF-5-dependent signalling in hair follicles or parts thereof, and useful to reduce and/or delay and/or inhibit hair loss or hair thinning caused by FGF-5 signalling in the hair follicle. The monoterpenoids compounds are formulated for topical application to the skin. Such topical formulations are administered topically to subjects to reduce FGF5-dependent signalling in a hair follicle cell or part thereof and/or delay FGF5-dependent signalling in a hair follicle cell or part thereof and/or prevent FGF5-dependent signalling in a hair follicle cell or part thereof, to thereby reduce loss of terminal hair and/or reduce thinning of terminal hair and/or prevent loss of terminal hair and/or prevent thinning of terminal hair and/or delay loss of terminal hair and/or delay thinning of terminal hair in a subject *e.g.,* such as in an aging subject or a subject wishing or a subject suffering from alopecia, such as androgenic alopecia and/or alopecia areata and/or acute alopecia.

Accordingly, the present invention provides a topical formulation for use in a method of treating or preventing hair loss in a subject, said topical formulation comprising 2-Methyl-5-(1-methylethenyl)-2-cyclohexen-1-ol (carveol) or an isolated enantiomer thereof or an isolated ester thereof with a carboxylic acid in an amount sufficient to reduce or inhibit fibroblast growth factor 5 (FGF5)-dependent signalling in a hair follicle cell of a subject to which the formulation is applied.

The present invention also provides the topical formulation for use according to the invention comprising a carboxylic acid monoester of 2-Methyl-5-(1-methylethenyl)-2-cyclohexen-1-ol (carveol). The carboxylic acid monoester of 2-Methyl-5-(1-methylethenyl)-2-cyclohexen-1-ol (carveol) may be a monoester with a carboxylic acid selected from acetic acid, propionic acid and formic acid, or may be 5-Isopropenyl-2-methyl-2-cyclohexen-1-yl acetate (carvyl acetate).

The present invention provides the topical formulation for use according to the present invention comprising an isolated enantiomer of 2-Methyl-5-(1-methylethenyl)-2-cyclohexen-1-ol (carveol). The isolated enantiomer of 2-Methyl-5-(1-methylethenyl)-2-cyclohexen-1-ol (carveol) may be selected from the group consisting of:
(*1R,5R*)-2-Methyl-5-(1-methylethenyl)-2-cyclohexen-1-ol [(-)-*cis*-carveol];
(*1S,5S*)-2-Methyl-5-(1-methylethenyl)-2-cyclohexen-1-ol [(+)-*cis*-carveol];
(*1R,5S*)-2-Methyl-5-(1-methylethenyl)-2-cyclohexen-1-ol [(+)-*trans*-carveol]; and
(*1S,5R*)-2-Methyl-5-(1-methylethenyl)-2-cyclohexen-1-ol [(-)-*trans*-carveol].

The present invention provides the topical formulation for use according to the present invention comprising a topical carrier, excipient or emollient and/or further comprising one or more adjunctive agents effective for treatment or prevention of hair loss.

The present invention also provides the topical formulation for use according to the invention, wherein the method of treating or preventing hair loss comprises administering the topical formulation to an area of the dermis or skin of the subject comprising one or more hair follicle cells comprising terminal hair(s), or an area of dermis adjacent or surrounding thereto, to thereby extend an anagen phase of a hair follicle cell comprising the hair and/or delay the hair follicle cell comprising the hair from entering a catagen phase. The hair may be scalp hair and the method of treating or preventing hair loss comprises administering the topical formulation to the scalp of the subject. The hair may be eyelash hair and the method of treating or preventing hair loss comprises administering the topical formulation to the eyelid or eyelash of the subject; and/or the hair may be eyebrow hair and the method of treating or preventing hair loss comprises administering the topical formulation to the face or forehead or eyebrow of the subject. Also provided is the topical formulation for use according to the invention, wherein hair growth is promoted or enhanced.

The present invention also provides the topical formulation for use according to the present invention, wherein the hair loss is associated with alopecia. The alopecia may involve scalp hair and the method comprises administering the topical formulation to the scalp of the subject. The alopecia may involve eyelash hair and the method comprises administering the topical formulation to the eyelid or eyelash of the subject; and/or the alopecia may involve eyebrow hair and the method comprises administering the topical formulation to the face or forehead or eyebrow of the subject.

The present invention also provides 2-Methyl-5-(1-methylethenyl)-2-cyclohexen-1-ol (carveol) or an isolated enantiomer thereof or an isolated ester thereof with a carboxylic acid of the invention for use in the treatment or prevention of alopecia in a subject. The 2-Methyl-5-(1-methylethenyl)-2-cyclohexen-1-ol (carveol) or isolated enantiomer thereof or isolated ester thereof with a carboxylic acid may be provided an amount sufficient to reduce or inhibit FGF-5 activity in the hair follicle or part thereof, and/or may be provided in an amount sufficient to reduce or inhibit FGF-5 binding to a cognate fibroblast growth factor receptor (FGFR) in the hair follicle or part thereof; and/or may be provided with one or more adjunctive agents effective for treatment or prevention of hair loss.

The present invention also provides a cosmetic method for treating or preventing hair loss comprising administering a topical formulation to a subject, wherein said topical formulation comprises 2-Methyl-5-(1-methylethenyl)-2-cyclohexen-1-ol (carveol) or an isolated enantiomer thereof or an isolated ester thereof with a carboxylic acid in an amount sufficient to reduce or inhibit fibroblast growth factor 5 (FGF5)-dependent signalling in a hair follicle cell of a subject to which the formulation is applied. The topical formulation may be a topical formulation of the invention.

The present disclosure provides a topical formulation comprising an amount of an isolated C₁₀-monoterpenoid or isolated enantiomer thereof or an isolated ester thereof with a carboxylic acid in an amount sufficient to reduce fibroblast growth factor 5 (FGF5)-dependent signalling in a hair follicle cell, wherein the C₁₀-monoterpenoid is of formula (I): 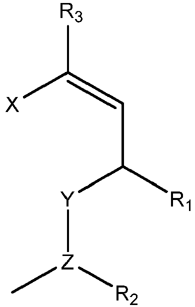 wherein:
R₁ is hydrogen, hydroxyl or oxygen;
R₂ is absent or hydrogen or hydroxyl;
R₃ is CH₃;
X is CH₃ or CH₂OH, or
X is CH₂CH₂ or CHOHCH₂ and X and Y together form a single bond within a 6-membered ring;
Y is CH₂ when X is CH₃ or CH₂OH, or
Y is CH or COH when X is CH₂CH₂ or CHOHCH₂; and
Z is a saturated or unsaturated C₂-C₅ alkyl or alkyl ester.

Topical formulations used in the inventions comprise 2-Methyl-5-(1-methylethenyl)-2-cyclohexen-1-ol (carveol) or an isolated enantiomer thereof or an isolated ester thereof with a carboxylic acid in an amount sufficient to reduce or inhibit fibroblast growth factor 5 (FGF5)-dependent signalling in a hair follicle cell of a subject to which the formulation is applied.

By "topical formulation" is meant that the formulation is capable of being applied externally to the dermis of a mammal *e.g.,* a human, or is applied to the dermis.

As used herein, the term "FGF5-dependent signalling" shall be understood to mean any signalling within and/or between cells in a signal transduction pathway that is dependent, either directly or indirectly, on the presence of FGF-5 and/or the presence of an amount of FGF-5 above a specific threshold.

As used herein, the term "C₂₋C₅ alkyl" refers to monovalent straight chain or branched hydrocarbon groups, having 2 to 5 carbon atoms. It is to be understood that the term "C₂₋C₅ alkyl" includes an alkyl chain having 2, 3, 4 or 5 carbon atoms. Suitable alkyl groups include, but are not limited to, ethyl, propyl, isopropyl, n-butyl, sec-butyl, tert-butyl, n-pentyl or 2,2-dimethylpropyl. The C₂-C₅ alkyl may be optionally substituted with one or more substituents. The substituents may be in any position of the carbon chain. Hydroxyl groups of the C₂-C₅ alkyl may be esterified with a lower alkyl carboxylic acid, such as, for example, acetic acid, propionic acid or formic acid.

The topical formulation of the present disclosure may comprise a C₁₀-monoterpenoid which is monohydroxylated or non-hydroxylated. In one example, the C₁₀-monoterpenoid is monohydroxylated. In one example, the C₁₀-monoterpenoid is non-hydroxylated.

In one example, the topical formulation comprises a C₁₀-monoterpenoid of formula (I) wherein R₁ is hydrogen. Alternatively, the topical formulation comprises a C₁₀-monoterpenoid of formula (I) wherein R₁ is oxygen.

In another example, the topical formulation comprises a C₁₀-monoterpenoid of formula (I) wherein X is CH₃ and Y is CH₂. Alternatively, the topical formulation comprises a C₁₀-monoterpenoid of formula (I) wherein X is CH₂OH and Y is CH₂.

In another example, the topical formulation comprises a C₁₀-monoterpenoid of formula (I) wherein X is CH₂CH₂. For example, the topical formulation may comprise a C₁₀-monoterpenoid of formula (I) wherein X is CH₂CH₂ and Y is CH. Alternatively, the topical formulation may comprise a C₁₀-monoterpenoid of formula (I) wherein X is CH₂CH₂ and Y is COH.

In another example, the topical formulation comprises a C₁₀-monoterpenoid of formula (I) wherein X is CHOHCH₂. For example, the topical formulation may comprise a C₁₀-monoterpenoid of formula (I) wherein X is CHOHCH₂ and Y is CH. Alternatively, the topical formulation may comprise a C₁₀-monoterpenoid of formula (I) wherein X is CHOHCH₂ and Y is COH.

In another example, the topical formulation comprises a C₁₀-monoterpenoid of formula (I) wherein R₂ is hydrogen. Alternatively, the topical formulation comprises a C₁₀-monoterpenoid of formula (I) wherein R₂ is hydroxyl. Alternatively, the topical formulation comprises a C₁₀-monoterpenoid of formula (I) wherein R₂ is absent.

In another example, the topical formulation comprises a C₁₀-monoterpenoid of formula (I) wherein Z is a saturated C₂ alkyl, such as, for example, CCH₃. Alternatively, the topical formulation comprises a C₁₀-monoterpenoid of formula (I) wherein Z is an unsaturated C₂-C₃ alkyl, such as, for example, CCH₂ or CCHCH₂. In one embodiment, Z is CCH₂. In another embodiment, Z is CCHCH₂.

In another example, the topical formulation comprises a C₁₀-monoterpenoid of formula (I) wherein Z is an unsaturated C₂-C₃ alkyl and R₂ is absent. Alternatively, the topical formulation comprises a C₁₀-monoterpenoid of formula (I) wherein Z is an unsaturated C₂-C₃ alkyl and R₂ is hydroxyl. Alternatively, the topical formulation comprises a C₁₀-monoterpenoid of formula (I) wherein Z is an unsaturated C₂-C₃ alkyl and R₂ is hydrogen.

In yet another example, the topical formulation comprises a C₁₀-monoterpenoid of formula (I) wherein Z is CCHCH₂OCOCH₃. In a preferred embodiment, Z is CCHCH₂OCOCH₃, and the C₁₀-monoterpenoid or enantiomer thereof is non-hydroxylated.

In a further example, the topical formulation comprises a C₁₀-monoterpenoid or enantiomer thereof which is monohydroxylated, wherein R₁ is hydrogen, R₂ is hydroxyl, X is CH₃, Y is CH₂, and Z is an unsaturated C₂-C₃ alkyl, such as CCHCH₂.

In another example, the topical formulation comprises a C₁₀-monoterpenoid or enantiomer thereof which is monohydroxylated, wherein R₁ is hydrogen or oxygen, R₂ is absent or hydrogen or hydroxyl, X is CH₂CH₂ or CHOHCH₂, Y is CH or COH, and Z is a saturated or unsaturated C₂ alkyl. For example, the topical formulation may comprise a C₁₀-monoterpenoid of formula (I) wherein R₁ is oxygen, R₂ is hydrogen or hydroxyl, X is CH₂CH₂, Y is CH, and Z is a saturated C₂ alkyl, preferably, wherein R₂ is hydrogen. Alternatively, the topical formulation may comprise a C₁₀-monoterpenoid of formula (I) which is monohydroxylated, wherein R₁ is hydrogen, R₂ is hydrogen or hydroxyl, X is CH₂CH₂, Y is CH or COH, and Z is a saturated C₂ alkyl, preferably wherein Y is CH and/or R₂ is hydroxyl. Alternatively, the topical formulation may comprise a C₁₀-monoterpenoid of formula (I) which is monohydroxylated, wherein R₁ is hydrogen, R₂ is hydrogen or hydroxyl, X is CH₂CH₂, Y is CH or COH, and Z is a saturated C₂ alkyl, preferably wherein Y is COH and/or R₂ is hydrogen. Alternatively, the topical formulation comprises a C₁₀-monoterpenoid or enantiomer thereof which is monohydroxylated, wherein R₁ is hydrogen or oxygen, R₂ is absent, X is CHOHCH₂, Y is CH, and Z is an unsaturated C₂ alkyl.

In another example, the topical formulation comprises a C₁₀-monoterpenoid or enantiomer thereof which is non-hydroxylated, and wherein R₁ is hydrogen, R₂ is absent, X is CH₃, Y is CH₂, and Z is CCHCH₂OCOCH₃.

In one example, the C₁₀-monoterpenoid is selected from the group consisting of 3-Methyl-6-(propan-2-yl)cyclohex-2-en-1-one (piperitone), 1-Isopropyl-4-methyl-3-cyclohexen-1-ol (terpinen-4-ol), 2-(4-Methyl-3-cyclohexen-1-yl)-2-propanol (alpha-terpineol), 2-Methyl-5-(1-methylethenyl)-2-cyclohexen-1-ol (carveol), 6-Isopropyl-3-methyl-2-cyclohexen-1-one (3-carvomenthenone), and 3,7-Dimethyl-1,6-octadien-3-ol (linalool). Preferably, the C₁₀-monoterpenoid is 3-Methyl-6-(propan-2-yl)cyclohex-2-en-1-one (piperitone) or 1-Isopropyl-4-methyl-3-cyclohexen-1-ol (terpinen-4-ol). More preferably, the C₁₀-monoterpenoid is 3-Methyl-6-(propan-2-yl)cyclohex-2-en-1-one (piperitone).

In another example, the topical formulation comprises a carboxylic acid monoester of a C₁₀-monoterpenoid of formula (I) as described herein. For example, the carboxylic acid monoester may be a monoester with a carboxylic acid selected from acetic acid, propionic acid and formic acid. For example, the carboxylic acid is acetic acid In another example, the carboxylic acid is acetic acid and/or the the C₁₀-monoterpenoid carboxylic acid ester is selected from the group consisting of (*2E*)-3,7-Dimethyl-2,6-octadien-1-yl acetate (geranyl acetate), 3,7-Dimethyl-1,6-octadien-3-yl acetate (linalyl acetate); 2-(4-Methyl-3-cyclohexen-1-yl)-2-propanyl acetate (terpinyl acetate); and 5-Isopropenyl-2-methyl-2-cyclohexen-1-yl acetate (carvyl acetate). More preferably, the C₁₀-monoterpenoid carboxylic acid ester is (*2E*)-3,7-Dimethyl-2,6-octadien-1-yl acetate (geranyl acetate) or 3,7-Dimethyl-1,6-octadien-3-yl acetate (linalyl acetate).

In a further example, the topical formulation of the present disclosure comprises an isolated enantiomer of a C₁₀-monoterpenoid of formula (I) as described herein, such as, for example, an isolated enantiomer selected from the group consisting of (*R*)-1-Isopropyl-4-methyl-3-cyclohexen-1-ol [(-)-terpinen-4-ol], (*1S*)-1-Isopropyl-4-methyl-3-cyclohexen-1-ol [(+)-terpinen-4-ol], 2-[(*1R*)-4-Methylcyclohex-3-en-1-yl]propan-2-ol [(+)-alpha-terpineol], (6*R*)-3-methyl-6-(propan-2-yl)cyclohex-2-en-1-one *syn.* (*6R*)-Isopropyl-3 -methyl-2-cyclohexen-1 -one [(-)-piperitone], (6*S*)-3-Methyl-6-(propan-2-yl)cyclohex-2-en-1-one [(+)-piperitone], (*3S*)-3,7-Dimethyl-1,6-octadien-3-ol [(+)-Linalool], (*3R*)-3,7-Dimethyl-1,6-octadien-3-ol [(-)-Linalool], (*1R,5R*)-2-Methyl-5-(I-methylethenyl)-2-cyclohexen-1-ol [(-)-*cis*-carveol], (*1S*,*5S*)-2-Methyl-5-(1-methylethenyl)-2-cyclohexen-1-ol [(+)-*cis*-carveol], (*1R*,*5S*)-2-Methyl-5-(1-methylethenyl)-2-cyclohexen-1-ol [(+)-*trans*-carveol], and (*1S*,*5R*)-2-Methyl-5-(1-methylethenyl)-2-cyclohexen-1-ol [(-)-*trans*-carveol]. In one example, the isolated enantiomer of the C₁₀-monoterpenoid is (*R*)-1-Isopropyl-4-methyl-3-cyclohexen-1-ol [(-)-terpinen-4-ol]. In one example, the isolated enantiomer of the C₁₀-monoterpenoid is (*1S*)-1-Isopropyl-4-methyl-3-cyclohexen-1-ol [(+)-terpinen-4-ol]. In one example, the isolated enantiomer of the C₁₀-monoterpenoid is 2-[(*1R*)-4-Methylcyclohex-3-en-1-yl]propan-2-ol [(+)-alpha-terpineol]. In one example, the isolated enantiomer of the C₁₀-monoterpenoid is (6*R*)-3-methyl-6-(propan-2-yl)cyclohex-2-en-1-one *syn.* (*6R*)-Isopropyl-3-methyl-2-cyclohexen-1-one [(-)-piperitone]. In one example, the isolated enantiomer of the C₁₀-monoterpenoid is (6*S*)-3-Methyl-6-(propan-2-yl)cyclohex-2-en-1-one [(+)-piperitone]. In one example, the isolated enantiomer of the C₁₀-monoterpenoid is (*3S*)-3,7-Dimethyl-1,6-octadien-3-ol [(+)-Linalool]. In one example, the isolated enantiomer of the C₁₀-monoterpenoid is (*3R*)-3,7-Dimethyl-1,6-octadien-3-ol [(-)-Linalool]. In one example, the isolated enantiomer of the C₁₀-monoterpenoid is (*1R,5R*)-2-Methyl-5-(1-methylethenyl)-2-cyclohexen-1-ol [(-)-*cis*-carveol]. In one example, the isolated enantiomer of the C₁₀-monoterpenoid is (*1S,5S*)-2-Methyl-5-(1-methylethenyl)-2-cyclohexen-1-ol [(+)-*cis*-carveol]. In one example, the isolated enantiomer of the C₁₀-monoterpenoid is (*1R,5S*)-2-Methyl-5-(1-methylethenyl)-2-cyclohexen-1-ol [(+)-*trans*-carveol]. In one example, the isolated enantiomer of the C₁₀-monoterpenoid is (*1S,5R*)-2-Methyl-5-(1-methylethenyl)-2-cyclohexen-1-ol [(-)-*trans*-carveol]. Preferably, the isolated enantiomer of the C₁₀-monoterpenoid is selected from the group consisting of (*R*)-1-Isopropyl-4-methyl-3-cyclohexen-1-ol [(-)-terpinen-4-ol], (*1S*)-1-Isopropyl-4-methyl-3-cyclohexen-1-ol [(+)-terpinen-4-ol], (6*R*)-3-methyl-6-(propan-2-yl)cyclohex-2-en-1-one or (6*R*)-Isopropyl-3-methyl-2-cyclohexen-1-one [(-)-piperitone], (6*S*)-3-Methyl-6-(propan-2-yl)cyclohex-2-en-1-one [(+)-piperitone], (*1S,5S*)-2-Methyl-5-(1-methylethenyl)-2-cyclohexen-1-ol [(+)-*cis*-carveol], and (*1R,5S*)-2-Methyl-5-(1-methylethenyl)-2-cyclohexen-1-ol [(+)-*trans*-carveol]. Preferably, an isolated enantiomer of the C₁₀-monoterpenoid is (*R*)-1-Isopropyl-4-methyl-3-cyclohexen-1-ol [(-)-terpinen-4-ol].

Alternatively, or in addition, an isolated enantiomer of the C₁₀-monoterpenoid is (-)-piperitone or (+)-piperitone. For example, a topical formulation of the present disclosure comprises isolated piperitone or enantiomer thereof such as (-)-piperitone or (+)-piperitone in combination with terpinen-4-ol or enantiomer thereof such as (-)-terpinen-4-ol.

A further particularly preferred embodiment of the present disclosure provides a topical formulation comprising a combination of (i) isolated piperitone or an isolated enantiomer or carboxylic acid ester thereof and (ii) isolated terpinen-4-ol or an isolated enantiomer or carboxylic acid ester thereof in the preparation of a topical medicament for the treatment and/or prevention of alopecia in a subject in need thereof. This combination includes a combination selected from the following: (i) piperitone and terpinene-4-ol; (ii) piperitone and (-)-terpinene-4-ol; (iii) piperitone and (+)-terpinen-4-ol; (iv) (-)-piperitone and terpinene-4-ol; (v) (-)-piperitone and (-)-terpinen-4-ol; (vi) (-)-piperitone and (+)-terpinen-4-ol; (vii) (+)-piperitone and terpinene-4-ol; (viii) (+)-piperitone and (-)-terpinen-4-ol; and (ix) (+)-piperitone and (+)-terpinen-4-ol. In one example, the combination is piperitone and terpinene-4-ol. In one example, the combination is piperitone and (-)-terpinene-4-ol. In one example, the combination is piperitone and (+)-terpinen-4-ol. In one example, the combination is (-)-piperitone and terpinene-4-ol. In one example, the combination is (-)-piperitone and (-)-terpinen-4-ol. In one example, the combination is (-)-piperitone and (+)-terpinen-4-ol. In one example, the combination is (+)-piperitone and terpinene-4-ol. In one example, the combination is (+)-piperitone and (-)-terpinen-4-ol. In one example, the combination is (+)-piperitone and (+)-terpinen-4-ol. Of these combinations, the combination of piperitone and (-)-terpinene-4-ol is particularly preferred.
The total amount of the C₁₀-monoterpenoid or ester or enantiomer thereof in the topical formulation is an amount sufficient to reduce or inhibit FGF-5 activity in the hair follicle or part thereof. For example, the total amount of the C₁₀-monoterpenoid or ester or enantiomer thereof is an amount sufficient to reduce or inhibit FGF-5 binding to a cognate fibroblast growth factor receptor (FGFR) *e.g.,* FGFR1, in the hair follicle or part thereof.

It is to be understood that isolated C₁₀-monoterpenoids or esters or enantiomers thereof comprised in the topical formulation(s) for use in the present invention may be isolated from various sources. For example, the C₁₀-monoterpenoids or esters or enantiomers thereof may be a natural product or isolated from a natural product or natural source *e.g.,* such as from plants, plant parts and/or essential oils by conventional procedures. Alternatively, the isolated C₁₀-monoterpenoids or esters or enantiomers thereof may be synthetic compounds. Alternatively, the monoterpenoids may be produced recombinantly, such as by expression of genes required for monoterpenoid production in yeast cells. Preferably, the compound is isolated as an essential oil, perfume oil, or perfume.

In a preferred example, the topical formulation used in the invention consists of or comprises a fragrance oil or perfume oil or essential oil or combination thereof or a perfume derived from a fragrance oil or perfume oil or essential oil or combination thereof, wherein the fragrance oil or perfume oil or essential oil or perfume comprises an amount of carveol or enantiomer or carboxylic acid derivative thereof in an amount sufficient to reduce binding of FGF-5 to FGFR1 or to treat or prevent hair loss in a subject in need thereof, especially in treatment or prevention of alopecia, as described in any example hereof.

As used herein the term "fragrance oil" or "perfume oil" shall be taken to refer to an extract such as a solution comprising alcohol, e.g., ethanol, comprising one or more synthetic monoterpenoids used in the invention, whether or not the extract also comprises a natural compound.

As used herein, the term "essential oil" shall be taken to mean a concentrated hydrophobic liquid derived by distillation or cold pressing of plant material and comprising one or more natural monoterpenoids of the. An oil is termed "essential" because it carries a distinctive scent or essence of the plant from which it derives.

For example, the topical formulation of the present disclosure may comprise the isolated C₁₀-monoterpenoid or ester or enantiomer thereof in the form of an essential oil, such as an essential oil from *Eucalyptus dives.* An essential oil from *E. dives* may comprise piperitone or enantiomer thereof such as (-)-piperitone or (+)-piperitone and/or terpinen-4-ol or enantiomer thereof such as (-)-terpinen-4-ol.

As used herein, the term "perfume" shall be taken to mean an oil e.g., a perfume oil or essential oil or combination thereof as defined herein wherein the oil comprises up to about 25% of the essential oil or perfume oil or combination thereof, generally diluted in ethanol and/or water or other diluent known in the art.

It is to be understood that an oil or perfume need only have a sufficient concentration of a monoterpenoid described herein to perform the invention. Notwithstanding that an oil or perfume is generally used in liquid form, quantitation of the active compound *e.g.,* monoterpenoid or carboxylic acid ester or enantiomer thereof, may be determined employing the liquid or a powder prepared therefrom. The skilled artisan will be aware various methods known in the art for quantifying such active compounds. For example, a powder may be prepared from a predetermined volume of oil or perfume, resuspending the powder in a suitable solvent to produce a sample solution, and subjecting the sample solution to one or more gas chromatography (GC) and/or mass spectrometry (MS) processes to thereby determine an amount of monoterpenoid in the powder. Exemplary means for drying an oil or perfume include drying over anhydrous sodium sulphate. Exemplary solvents for dissolving powders comprising monoterpenoids include any solvent suitable for GS-MS, *e.g.,* diethyl ether. Exemplary GS-MS systems for quantitation of monterpenoids include fast-GC and/or fast-GC-qMSs and/or enantioselective GC and/or multidimensional GC and/or GC-isotopic ratio mass spectrometry (GC-IRMS) and/or gas chromatography with flame ionization detection (GC-FID). Thus, based on the concentration of monoterpenoid in a powder prepared from a known sample volume of oil or perfume, the amount of the active compound in any other volume of the oil or perfume may be determined without undue effort. Similarly, if such quantitation is performed on a liquid aliquot of the oil or perfume, the amount of the active compound in any other volume of the oil or perfume may be determined without undue effort. Similarly, quantitation of formulations of the invention other than oils or perfumes *e.g.,* a tonic or shampoo or lotion, may be determined readily based on the percentage volume of oil or perfume (v/v) in the formulation.

Exemplary concentrations of monoterpenoids in essential oils are set forth in Table 1 hereof, and amounts of the monoterpenoids in any fragrance oils may be determined readily based on the known amount of the active compound(s).

It is within the ken of a skilled formulation chemist to produce an oil or perfume or other formulation used in the invention having a reproducible amount of a given monoterpenoid or carboxylic acid ester or enantiomer thereof. In general, a suitable concentration of such active comound(s) is prepared readily by evaporation of an oil or perfume comprising one or more non-volatile active compounds, or by dilution of an oil or perfume comprising the active compound(s) described herein, *e.g.,* using ethanol or other suitable diluent known in the art.

Topical formulation(s) used in the present invention may be presented in unit dose forms containing a predetermined amount of the carveol or ester or enantiomer thereof per unit dose sufficient to reduce FGF5-dependent signalling in a hair follicle cell. It is to be understood that the concentration of monoterpenoid compound may vary depending upon a range of parameters *e.g.,* including whether or not the formulation is for use in prevention or therapy, the site to which the topical formulation is to be applied, the half-life of the compound
following administration of the topical formulation, the age, sex and weight of the subject, and the type of hair loss condition, if any, to which the subject is predispose or which is to be treated.

It is also to be understood that the topical formulation(s) used in the present invention may comprise a plurality of isolated C₁₀-monoterpenoids or esters or enantiomers thereof as described herein, *e.g.,* such as 2, 3, 4, 5, 6, 7, 8, 9, 10 or more compounds. The skilled artisan will be aware that it is possible to combine monoterpenoids that are active in performing the invention by combining one or more perfume oils and/or one or more essential oils to achieve optimum concentrations of active monoterpenoids as determined by the activity profile(s) of the constituent monoterpenoid(s) described herein.

The topical formulations used in the present invention may also comprise one or more carriers, excipients or emollients suitable for topical administration *e.g.,* such as to the dermis or skin of a subject. For example, a carrier suitable for topical administration may be selected from the group consisting of a transdermal patch, lotion, ointment, paste, foam, emulsion, cream, serum, aerosol, spray, roll-on formulation, masque, cleanser, shampoo, conditioner, gel, oil or moisturizer, A suitable carrier can be a lubricating formulation, water-based formulation, silicone-based formulation, petroleum- based formulation, natural-oil based formulation, and/or massage formulation.

The topical formulation used in the present invention may further comprise one or more adjunctive therapeutic agents. For example, the adjunctive agent may be selected from the group consisting of estradiol, oxandrolone, minoxidil, *Sanguisorba officinalis* root extract, *Rosa multiflora* extract, Brown algae extract, loquat leaf extract, Pecan shell extract, squill extract, sodium phytate, Fucus vesiculosus extract, phytic acid, nonanal, and Lipidure-C. Combinations of the monoterpenoids used in the invention are not excluded from such adjunctive formulations.

The topical formulations used in the invention as described in any example hereof are useful for delaying and/or reducing loss of terminal hair in a subject. Such utility may be non-therapeutic or therapeutic. By "non-therapeutic" is meant that the subject to whom the formulation is administered does not suffer from a pre-existing medical condition that causes hair loss or hair thinning, *e.g.,* alopecia, however may be predisposed to such a condition. Accordingly, a non-therapeutic use may be a cosmetic treatment or a prophylactic treatment in the present context. Such cosmetic treatments include treatment of hair loss that is of non-medical aetiology *e.g.,* as a consequence of age and/or sex of the subject. In contrast, a therapeutic use is for treatment of a pre-existing medical condition that causes hair loss or hair thinning *e.g.,* alopecia arising from any one or more factors responsible for the condition *e.g.,* stress, chemotherapy, *etc.*

For example, a non-therapeutic or cosmetic use may comprise administering a formulation of the invention as described herein to a non-alopecic subject who wishes to maintain full, voluminous hair. A non-therapeutic formulation is also suitable for reducing or delaying hair loss in a subject who is not suffering from alopecia, but who is suffering from loss of terminal hair *e.g.,* natural hair loss. Alternatively, or in addition, the non-therapeutic formulations are suitable for prevention of terminal hair loss in a subject having no visible symptoms of alopecia, however suffers from a genetic condition that predisposes him/her to future onset of alopecia including androgenic alopecia. Alternatively, or in addition, the non-therapeutic formulations are suitable for prevention of terminal hair loss in a non-alopecic subject about to undergo therapy with a cytotoxic or cytostatic agent or antiviral compound that will induce loss of terminal hair.

Accordingly, the present disclosure also provides a method of reducing and/or delaying and/or preventing loss of terminal hair in a human or mammalian subject who is not suffering from alopecia. Such a non-therapeutic method may comprise administering a topical formulation of the invention as described in any example hereof to an area of the dermis or skin of the human or mammalian subject in which loss of terminal hair is to be reduced and/or delayed and/or prevented, or to an area of dermis adjacent or surrounding an area of the dermis or skin of the human or mammalian subject. The administration is generally for a time and under conditions sufficient to reduce or delay or prevent the loss of terminal hair in the subject. In one example, the subject to whom the topical formulation is administered is a subject who wishes to maintain full, voluminous hair by reducing and/or delaying and/or preventing hair loss not caused by alopecia. Alternatively, the subject is not suffering from alopecia, but suffering from loss of terminal hair. Alternatively, the subject may have no visible symptoms of alopecia, however suffer from a genetic condition that predisposes the subject to alopecia *e.g.,* a genetic predisposition to hair loss or familial history of hair loss. Alternatively, the subject may be about to undergo therapy with a cytotoxic or cytostatic agent or antiviral compound that induces loss of terminal hair.

It is to be understood that the frequency of dosage and the total amount of C₁₀-monoterpenoid or ester or enantiomer thereof in a unit dosage of the topical formulation for a non-therapeutic use may vary. Factors affecting frequency and amount of dosage include *e.g.,* the site to which the topical formulation is to be applied and/or the half-life of the specific C₁₀-monoterpenoid compound in the topical formulation following administration thereof, and/or the age and/or sex and/or weight of the subject.

The topical formulations used in the invention are useful for delaying or reducing or preventing loss of any terminal hair including, for example, scalp hair and/or eyelash hair and/or eyebrow hair. The method may comprise administering the topical formulation used in the invention to the scalp of a human or mammalian subject not suffering from alopecia to reduce and/or delay and/or prevent loss of scalp hair in that subject. Alternatively, or in addition, the method may comprise administering the topical formulation of the invention to the eyelid or eyelash of a human or mammalian subject not suffering from alopecia to reduce and/or delay and/or prevent loss of eyelash hair in that subject. Alternatively, or in addition, the method may comprise administering the topical formulation used in the invention to the face or forehead or eyebrow of a human or mammalian subject not suffering from alopecia to reduce and/or delay and/or prevent loss of eyebrow hair in that subject.

The non-therapeutic method of the invention may also comprise promoting or enhancing growth of terminal hair of the subject. In addition, topical formulation(s) used in the invention as described in any example hereof may promote or enhance growth of the terminal hair in a subject.

In another example, the topical formulation(s) used in the present invention as described in any example hereof are useful for treating alopecia *e.g.,* an acute form of alopecia or alopecia areata or androgenic alopecia, in a human or other mammalian subject. An acute form of alopecia may be induced by an acute event selected from pregnancy, stress, illness, treatment with a cytotoxic agent, treatment with a cytostatic agent, and treatment with an agent that induces necrosis or apoptosis of hair follicles as a side-effect of therapy. Accordingly, the topical formulation used in the invention is suitable for a human or mammalian subject undergoing treatment with a cytotoxic agent or cytostatic agent, or to whom treatment with a cytotoxic agent or cytostatic agent has been prescribed. Alternatively, or in addition, the topical formulation is suitable for be a human or mammalian subject suffering from androgenic alopecia.

For example, the present invention also provides a topical formulation of the present invention as described in any example hereof for administration to an affected area of the dermis or skin of the human or mammalian subject for use in a method of treating alopecia *e.g.,* an acute form of alopecia or alopecia areata or androgenic alopecia, in a human or mammalian subject in need thereof. Alternatively, or in addition, the formulation is administered to an area of dermis adjacent or surrounding an affected area. The administration is generally for a time and under conditions sufficient to reduce or delay or prevent loss of terminal hair in the subject.

An acute form of alopecia may be induced by an acute event selected from pregnancy, stress, illness, treatment with a cytotoxic agent, treatment with a cytostatic agent, and treatment with an agent which induces necrosis or apoptosis of hair follicles as a side-effect of therapy. For example, the subject to whom the topical formulation is administered may be a human or mammalian subject undergoing treatment with a cytotoxic agent or cytostatic agent or to whom treatment with a cytotoxic agent or cytostatic agent has been prescribed. In one example, the topical formulation used in the invention is co-administered with a cytotoxic or cytostatic compound that causes hair loss *e.g.,* in the case of a subject undergoing chemotherapy or radiation therapy or treatment for HIV-1 infection or AIDS. In such circumstances, the efficacy of the C₁₀-monoterpenoid or ester or enantiomer thereof in the topical formulation counteracts the hair-loss effect of the cytotoxic or cytostatic compound.

The frequency and dosage amount of C₁₀-monoterpenoid or ester or enantiomer thereof in a topical formulation administered to the subject to treat alopecia may vary depending upon a range of parameters *e.g.,* the type of alopecia and/or the severity of the alopecia and/or the site to which the topical formulation is to be applied and/or the half-life of the specific C₁₀-monoterpenoid compound in the topical formulation following administration thereof and/or the age and/or sex and/or weight of the subject.

The topical formulations used in the invention are useful for delaying or reducing or preventing loss of any terminal hair in an alopectic patient or subject including, for example, scalp hair and/or eyelash hair and/or eyebrow hair. For example, the topical formulation may be for delaying or reducing or preventing loss of scalp hair in an alopectic patient or subject. Alternatively, or in addition, the topical formulation may be for delaying or reducing or preventing loss of eyelash hair in an alopectic patient or subject. Alternatively, or in addition, the topical formulation may be for delaying or reducing or preventing loss of eyebrow hair in an alopectic patient or subject.

The topical formulation(s) used in the invention for therapeutic and/or non-therapeutic application may delay or reduce or prevent loss of terminal hair by delaying hair follicles comprising the terminal hair from entering catagen phase. Alternatively, or in addition, an anagen phase of hair follicles comprising the terminal hair may be extended to thereby delay or reduce or prevent loss of terminal hair. In addition, topical formulation(s) used in the invention as described in any example hereof may promote or enhance growth of the terminal hair in a subject.

The present disclosure also provides for use of at least one isolated C₁₀-monoterpenoid or isolated enantiomer thereof or an isolated ester thereof with a carboxylic acid in the preparation of a topical medicament for the treatment of hair loss in a subject suffering from alopecia, wherein the C₁₀-monoterpenoid is of formula (I): 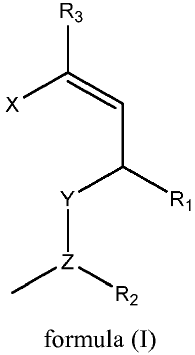 wherein:
R₁ is hydrogen, hydroxyl or oxygen;
R₂ is absent or hydrogen or hydroxyl;
R₃ is a CH₃;
X is CH₃ or CH₂OH, or
X is CH₂CH₂ or CHOHCH₂ and X and Y together form a single bond within a 6-membered ring;
Y is CH₂ when X is CH₃ or CH₂OH, or
Y is CH or COH when X is CH₂CH₂ or CHOHCH₂; and
Z is a saturated or unsaturated C₂-C₅ alkyl or alkyl ester.

By "topical medicament" is meant that the isolated C₁₀-monoterpenoid or isolated enantiomer thereof or an isolated ester thereof with a carboxylic acid is formulated for application to the dermis of a mammal.

The C₁₀-monoterpenoid for use in the preparation of the topical medicament may be monohydroxylated or non-hydroxylated.

In one example, the topical medicament comprises a C₁₀-monoterpenoid of formula (I) wherein R₁ is hydrogen. Alternatively, the topical medicament comprises a C₁₀-monoterpenoid of formula (I) wherein R₁ is oxygen.

In one example, the topical medicament comprises a C₁₀-monoterpenoid of formula (I) wherein X is CH₃ and Y is CH₂. Alternatively, the topical medicament comprises a C₁₀-monoterpenoid of formula (I) wherein X is CH₂OH and Y is CH₂.

In another example, the topical medicament comprises a C₁₀-monoterpenoid of formula (I) wherein X is CH₂CH₂. For example, the topical medicament may comprise a C₁₀-monoterpenoid of formula (I) wherein X is CH₂CH₂ and Y is CH. Alternatively, the topical medicament may comprise a C₁₀-monoterpenoid of formula (I) wherein X is CH₂CH₂ and Y is COH.

In another example, the topical medicament comprises a C₁₀-monoterpenoid of formula (I) wherein X is CHOHCH₂. For example, the topical medicament may comprise a C₁₀-monoterpenoid of formula (I) wherein X is CHOHCH₂ and Y is CH. Alternatively, the topical medicament may comprise a C₁₀-monoterpenoid of formula (I) wherein X is CHOHCH₂ and Y is COH.

In one example, the topical medicament comprises a C₁₀-monoterpenoid of formula (I) wherein R₂ is hydrogen. Alternatively, the topical medicament comprises a C₁₀-monoterpenoid of formula (I) wherein R₂ is hydroxyl. Alternatively, the topical medicament comprises a C₁₀-monoterpenoid of formula (I) wherein R₂ is absent.

In one example, the topical medicament comprises a C₁₀-monoterpenoid of formula (I) wherein Z is a saturated C₂ alkyl, such as, for example, CCH₃. Alternatively, the topical medicament comprises a C₁₀-monoterpenoid of formula (I) wherein Z is an unsaturated C₂-C₃ alkyl *e.g.,* such as CCH₂ or CCHCH₂. For example, in one embodiment, Z is CCH₂. In another embodiment, Z is CCHCH₂.

In another example, the topical medicament comprises a C₁₀-monoterpenoid of formula (I) wherein Z is an unsaturated C₂-C₃ alkyl and R₂ is absent. Alternatively, the topical medicament comprises a C₁₀-monoterpenoid of formula (I) wherein Z is an unsaturated C₂-C₃ alkyl and R₂ is hydroxyl. Alternatively, the topical medicament comprises a C₁₀-monoterpenoid of formula (I) wherein Z is an unsaturated C₂-C₃ alkyl and R₂ is hydrogen.

In yet another example, the topical medicament comprises a C₁₀-monoterpenoid of formula (I) wherein Z is CCHCH₂OCOCH₃. In a preferred embodiment, Z is CCHCH₂OCOCH₃, and the C₁₀-monoterpenoid or enantiomer thereof is non-hydroxylated.

In one example, the topical medicament comprises a C₁₀-monoterpenoid or enantiomer thereof which is monohydroxylated, wherein R₁ is hydrogen, R₂ is hydroxyl, X is CH₃, Y is CH₂, and Z is an unsaturated C₂-C₃ alkyl, such as CCHCH₂.

In another example, the topical medicament comprises a C₁₀-monoterpenoid or enantiomer thereof which is monohydroxylated, wherein R₁ is hydrogen or oxygen, R₂ is absent or hydrogen or hydroxyl, X is CH₂CH₂ or CHOHCH₂, Y is CH or COH, and Z is a saturated or unsaturated C₂ alkyl. For example, the topical medicament may comprise a C₁₀-monoterpenoid of formula (I) wherein R₁ is oxygen, R₂ is hydrogen or hydroxyl, X is CH₂CH₂, Y is CH, and Z is a saturated C₂ alkyl, preferably, wherein R₂ is hydrogen. Alternatively, the topical medicament may comprise a C₁₀-monoterpenoid of formula (I) which is monohydroxylated, wherein R₁ is hydrogen, R₂ is hydrogen or hydroxyl, X is CH₂CH₂, Y is CH or COH, and Z is a saturated C₂ alkyl, preferably wherein Y is CH and/or R₂ is hydroxyl. Alternatively, the topical medicament may comprise a C₁₀-monoterpenoid of formula (I) which is monohydroxylated, wherein R₁ is hydrogen, R₂ is hydrogen or hydroxyl, X is CH₂CH₂, Y is CH or COH, and Z is a saturated C₂ alkyl, preferably wherein Y is COH and/or R₂ is hydrogen. Alternatively, the topical medicament may comprise a C₁₀-monoterpenoid or enantiomer thereof which is monohydroxylated, wherein R₁ is hydrogen or oxygen, R₂ is absent, X is CHOHCH₂, Y is CH, and Z is an unsaturated C₂ alkyl.

In another example, the topical medicament comprises a C₁₀-monoterpenoid or enantiomer thereof which is non-hydroxylated, and wherein R₁ is hydrogen, R₂ is absent, X is CH₃, Y is CH₂, and Z is CCHCH₂OCOCH₃.

For example, the C₁₀-monoterpenoid for use in the manufacture of the topical medicament is selected from the group consisting of 3-Methyl-6-(propan-2-yl)cyclohex-2-en-1 -one (piperitone), 1 -Isopropyl-4-methyl-3 -cyclohexen-1 -ol (terpinen-4-ol), 2-(4-Methyl-3-cyclohexen-1-yl)-2-propanol (alpha-terpineol), 2-Methyl-5-(1-methylethenyl)-2-cyclohexen-1-ol (carveol), 6-Isopropyl-3-methyl-2-cyclohexen-1-one (3-carvomenthenone); and 3,7-Dimethyl-1,6-octadien-3-ol (linalool). Preferably, the C₁₀-monoterpenoid is 1-Isopropyl-4-methyl-3-cyclohexen-1-ol (terpinen-4-ol) or 3-Methyl-6-(propan-2-yl)cyclohex-2-en-1-one (piperitone).

In another example, the topical medicament comprises a carboxylic acid monoester of a C₁₀-monoterpenoid of formula (I) as hereinbefore described. For example, the carboxylic acid monoester may be a monoester with a carboxylic acid selected from acetic acid, propionic acid and formic acid. Preferably, the carboxylic acid is acetic acid and/or the C₁₀-monoterpenoid carboxylic acid ester for use in the manufacture of the topical medicament is selected from the group consisting of (*2E*)-3,7-Dimethyl-2,6-octadien-1-yl acetate (geranyl acetate), 3,7-Dimethyl-1,6-octadien-3-yl acetate (linalyl acetate); 2-(4-Methyl-3-cyclohexen-1-yl)-2-propanyl acetate (terpinyl acetate); and 5-Isopropenyl-2-methyl-2-cyclohexen-1-yl acetate (carvyl acetate). More preferably, the C₁₀-monoterpenoid carboxylic acid ester for use in the manufacture of the topical medicament is (*2E*)-3,7-Dimethyl-2,6-octadien-1-yl acetate (geranyl acetate) or 3,7-Dimethyl-1,6-octadien-3-yl acetate (linalyl acetate).

In a further example, the topical medicament of the present disclosure comprises an isolated enantiomer of a C₁₀-monoterpenoid of formula (I) as described herein. For example, an isolated enantiomer of a C₁₀-monoterpenoid of formula (I) for use in the manufacture of the topical medicament is selected from the group consisting of (*R*)-1-Isopropyl-4-methyl-3-cyclohexen-1-ol [(-)-terpinen-4-ol], (*1S*)-1-Isopropyl-4-methyl-3-cyclohexen-1-ol [(+)-terpinen-4-ol], 2-[(*1R*)-4-Methylcyclohex-3-en-1-yl]propan-2-ol [(+)-alpha-terpineol], (6*R*)-3-methyl-6-(propan-2-yl)cyclohex-2-en-1-one or (*6R*)-Isopropyl-3 -methyl-2-cyclohexen-1 -one [(-)-piperitone], (6*S*)-3-Methyl-6-(propan-2-yl)cyclohex-2-en-1-one [(+)-piperitone], (*3S*)-3,7-Dimethyl-1,6-octadien-3-ol [(+)-Linalool], (*3R*)-3,7-Dimethyl-1,6-octadien-3-ol [(-)-Linalool], (*1R,5R*)-2-Methyl-5-(1-methylethenyl)-2-cyclohexen-1-ol [(-)-*cis*-carveol], (*1S*,*5S*)-2-Methyl-5-(1-methylethenyl)-2-cyclohexen-1-ol [(+)-*cis*-carveol], (*1R,5S*)-2-Methyl-5-(1-methylethenyl)-2-cyclohexen-1-ol [(+)-*trans*-carveol], and (*1S,5R*)-2-Methyl-5-(1-methylethenyl)-2-cyclohexen-1-ol [(-)-*trans*-carveol]. Preferably, the isolated enantiomer of the C₁₀-monoterpenoid is selected from the group consisting of (*R*)-1-Isopropyl-4-methyl-3-cyclohexen-1-ol [(-)-terpinen-4-ol], (*1S*)-1-Isopropyl-4-methyl-3-cyclohexen-1-ol [(+)-terpinen-4-ol], (6*R*)-3-methyl-6-(propan-2-yl)cyclohex-2-en-1-one or (*6R*)-Isopropyl-3-methyl-2-cyclohexen-1-one [(-)-piperitone], (6*S*)-3-Methyl-6-(propan-2-yl)cyclohex-2-en-1-one [(+)-piperitone], (*1S,5S*)-2-Methyl-5-(1-methylethenyl)-2-cyclohexen-1-ol [(+)-*cis*-carveol], and (*1R,5S*)-2-Methyl-5-(1-methylethenyl)-2-cyclohexen-1-ol [(+)-*trans*-carveol]. More preferably, the isolated enantiomer of the C₁₀-monoterpenoid is (-)-terpinen-4-ol or (-)-piperitone or (+)-piperitone.

A particularly preferred embodiment of the present disclosure provides for use of isolated 1-Isopropyl-4-methyl-3-cyclohexen-1-ol (terpinen-4-ol) or an isolated enantiomer or carboxylic acid ester thereof in the preparation of a topical medicament for the treatment and/or prevention of alopecia in a subject in need thereof.

A further particularly preferred embodiment of the present disclosure provides use of isolated 3-methyl-6-(propan-2-yl)cyclohex-2-en-1-one (piperitone) or an isolated enantiomer or carboxylic acid ester thereof in the preparation of a topical medicament for the treatment and/or prevention of alopecia in a subject in need thereof.

A further particularly preferred embodiment of the present disclosure provides use of a combination of (i) isolated piperitone or an isolated enantiomer or carboxylic acid ester thereof and (ii) isolated terpinen-4-ol or an isolated enantiomer or carboxylic acid ester thereof in the preparation of a topical medicament for the treatment and/or prevention of alopecia in a subject in need thereof. This combination includes a combination selected from the following: (i) piperitone and terpinene-4-ol; (ii) piperitone and (-)-terpinene-4-ol; (iii) piperitone and (+)-terpinen-4-ol; (iv) (-)-piperitone and terpinene-4-ol; (v) (-)-piperitone and (-)-terpinen-4-ol; (vi) (-)-piperitone and (+)-terpinen-4-ol; (vii) (+)-piperitone and terpinene-4-ol; (viii) (+)-piperitone and (-)-terpinen-4-ol; and (ix) (+)-piperitone and (+)-terpinen-4-ol. Of these combinations, the combination of piperitone and (-)-terpinene-4-ol is particularly preferred.

As with the topical formulation used in the invention, a plurality of isolated C₁₀-monoterpenoids or esters or enantiomers thereof as described herein, *e.g.,* 2, 3, 4, 5, 6, 7, 8, 9, 10 or more compounds, may be used in the preparation of a topical medicament.

The integers described herein for the composition and use of topical formulations for therapeutic applications, especially with respect to the concentrations of active monoterpenoids and enantiomers and esters thereof, formulation and dosage, apply *mutatis mutandis* to the use of at least one isolated C₁₀-monoterpenoid or isolated enantiomer thereof or an isolated ester thereof with a carboxylic acid in the preparation of a topical medicament for the treatment of hair loss in a subject suffering from alopecia.

A subject for which the topical medicament is useful may be a human or mammalian subject that has a genetic predisposition for alopecia or familial history of alopecia or is at risk of developing alopecia. Alternatively, or in addition, the subject for which the topical medicament is useful may be a human or mammalian subject that is suffering from alopecia. The alopecia may be an acute form of alopecia and/or alopecia areata and/or androgenic alopecia.

In particularly preferred embodiment, the topical medicament is useful for treatment of androgenic alopecia in a subject suffering from, or at risk of suffering from, androgenic alopecia.

In a further preferred embodiment, the topical medicament is useful for treatment of an acute form of alopecia. The acute form of alopecia may be induced by an acute event selected from pregnancy, stress, illness, treatment with a cytotoxic agent, treatment with a cytostatic agent, and treatment with an agent which induces necrosis or apoptosis of hair follicles as a side-effect of therapy. Accordingly, a subject for which the topical medicament is useful may be a human or mammalian subject undergoing treatment with a cytotoxic agent or cytostatic agent or to whom treatment with a cytotoxic agent or cytostatic agent has been prescribed. For example, the topical medicament may be prepared for co-administration with a cytotoxic or cytostatic compound that causes hair loss *e.g.,* in the case of a subject undergoing chemotherapy or radiation therapy or treatment for HIV-1 infection or AIDS. In such circumstances, the efficacy of the C₁₀-monoterpenoid or ester or enantiomer thereof in the topical medicament counteracts the hair-loss effect of the cytotoxic or cytostatic compound.

As used herein the term "derived from" shall be taken to indicate that a specified integer may be obtained from a particular source albeit not necessarily directly from that source.

Throughout this specification, unless the context requires otherwise, the word "comprise", or variations such as "comprises" or "comprising", is understood to imply the inclusion of a stated step or element or integer or group of steps or elements or integers but not the exclusion of any other step or element or integer or group of elements or integers.

The term "hair" means any hair or fur on the body of a mammal including a human, and includes, for example, head hair, eyebrows, eyelashes, moustaches, beards, chest hair, back hair, arm hair, leg hair, genital hair, nasal hair or ear hair.

As used herein, the term "treat" or "treating" or "treatment" shall be taken to include therapeutic treatment of a pre-existing condition, wherein the aim is to prevent, ameliorate, reduce, slow down (lessen) or arrest progression of hair thinning or hair loss *e.g.,* associated with alopecia. It follows that hair growth, or treatment of hair thinning, refers to normalization of thinned hair, such as caused by alopecia. Treatment preferably extends the anagen phase of a hair follicle, or prevents or delays a follicle in anagen phase from prematurely transitioning to catagen phase.

As used herein, the term "delay" or "delaying" refers to a postponement or deferment of an event *e.g.,* such as loss of hair, until a time which is later that would otherwise be expected, or the act by which something is postponed or deferred, including the slowing of an event or process.

As used herein, the term "reduce" or "reducing" with respect to hair loss shall be taken to mean a decrease or lessening in the loss of hair *e.g.,* terminal hair, than would otherwise be expected in an individual following administration of a formulation or medicament used in the invention.

"Preventing", "prevention", "preventative" or "prophylactic" refers to keeping from occurring, or to hinder, defend from, or protect from the occurrence of a condition, disease, disorder, or phenotype, including an abnormality or symptom. A mammal in need of prevention may be prone to develop the condition.

The term "effective amount" shall be taken to mean an amount of the C₁₀-monoterpenoid compound used in the invention which is capable of preventing and/or reducing and/or delaying progression of hair thinning or hair loss in a mammal to a level which is beneficial to delay and/or reduce and/or treat and/or prevent hair thinning or hair loss, particularly associated with alopecia. A therapeutically effective amount may be determined empirically and in a routine manner in relation to treating hair thinning or hair loss.

Throughout this specification, unless specifically stated otherwise or the context requires otherwise, reference to a single step, composition of matter, group of steps or group of compositions of matter shall be taken to encompass one and a plurality (i.e. one or more) of those steps, compositions of matter, groups of steps or group of compositions of matter.

Each definition or clarifying term described herein shall be taken to apply *mutatis mutandis* to each and every example of the invention unless the context requires otherwise. Each example described herein is to be applied *mutatis mutandis* to each and every other example unless specifically stated otherwise.

### BRIEF DESCRIPTION OF THE DRAWINGS

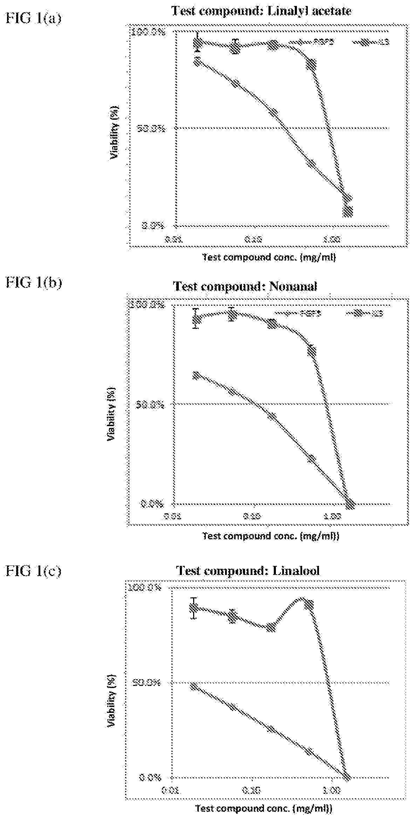

Figure 1(a) is a graphical representation showing the inhibitory activity of linalyl acetate at difference concentrations on proliferation and viability of FR-BaF3 cells in the presence of FGF-5 or IL-3. This figure also illustrates the concentration at which linalyl acetate inhibits viability of FR-BaF3 cells by 50% (IC50) when those cells are cultured in the presence of FGF-5 or IL-3.
Figure 1(b) is a graphical representation showing the inhibitory activity of nonanal at difference concentrations on proliferation and viability of FR-BaF3 cells in the presence of FGF-5 or IL-3. This figure also illustrates the concentration at which nonanal inhibits viability of FR-BaF3 cells by 50% (IC50) when those cells are cultured in the presence of FGF-5 or IL-3.
Figure 1(c) is a graphical representation showing the inhibitory activity of linalool at difference concentrations on proliferation and viability of FR-BaF3 cells in the presence of FGF-5 or IL-3. This figure also illustrates the concentration at which linalool inhibits viability of FR-BaF3 cells by 50% (IC50) when those cells are cultured in the presence of FGF-5 or IL-3.

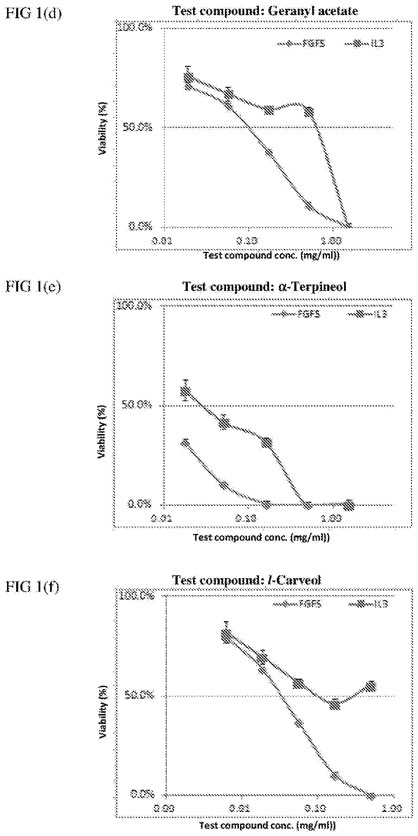

Figure 1(d) is a graphical representation showing the inhibitory activity of geranyl acetate at difference concentrations on proliferation and viability of FR-BaF3 cells in the presence of FGF-5 or IL-3. This figure also illustrates the concentration at which geranyl acetate inhibits viability of FR-BaF3 cells by 50% (IC50) when those cells are cultured in the presence of FGF-5 or IL-3
Figure 1(e) is a graphical representation showing the inhibitory activity of α-terpineol at difference concentrations on proliferation and viability of FR-BaF3 cells in the presence of FGF-5 or IL-3. This figure also illustrates the concentration at which α-terpineol inhibits viability of FR-BaF3 cells by 50% (IC50) when those cells are cultured in the presence of FGF-5 or IL-3.
Figure 1(f) is a graphical representation showing the inhibitory activity of *l*-carveol at difference concentrations on proliferation and viability of FR-BaF3 cells in the presence of FGF-5 or IL-3. This figure also illustrates the concentration at which *l-*carveol inhibits viability of FR-BaF3 cells by 50% (IC50) when those cells are cultured in the presence of FGF-5 or IL-3.

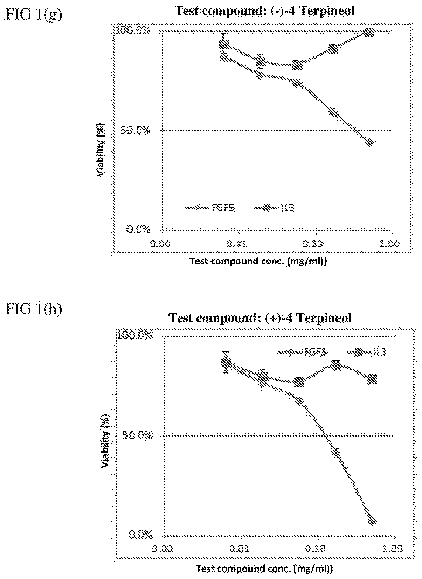

Figure 1(g) is a graphical representation showing the inhibitory activity of (-)-terpinen-4-ol at difference concentrations on proliferation and viability of FR-BaF3 cells in the presence of FGF-5 or IL-3. This figure also illustrates the concentration at which (-)-terpinen-4-ol inhibits viability of FR-BaF3 cells by 50% (IC50) when those cells are cultured in the presence of FGF-5 or IL-3.
Figure 1(h) is a graphical representation showing the inhibitory activity of (+)-terpinen-4-ol at difference concentrations on proliferation and viability of FR-BaF3 cells in the presence of FGF-5 or IL-3. This figure also illustrates the concentration at which (+)-terpinen-4-ol inhibits viability of FR-BaF3 cells by 50% (IC50) when those cells are cultured in the presence of FGF-5 or IL-3.

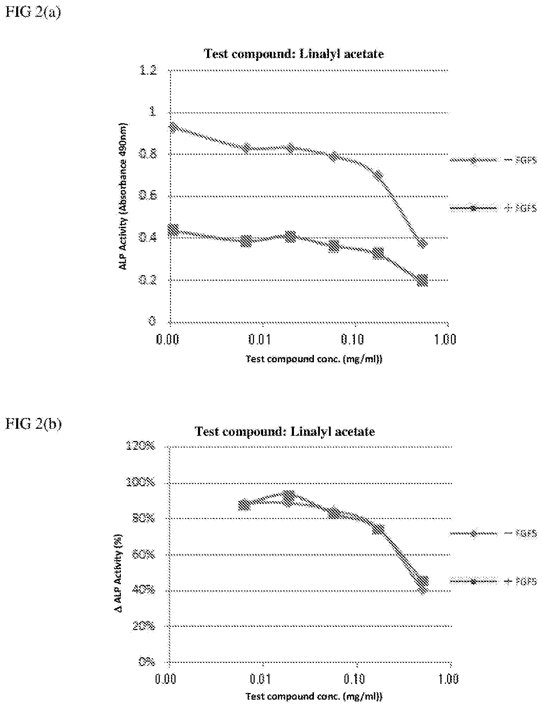

Figure 2(a) is a graphical representation showing the effect of linalyl acetate at difference concentrations on Alkaline Phosphatase (ALP) activity in dermal papilla (DP) cells cultured in the presence of: (i) a GSK3 inhibitor and FGF-5 or (ii) a GSK3 inhibitor only. ALP activity was determined by measuring absorbance at 490nm.
Figure 2(b) is a graphical representation showing the relative difference in Alkaline Phosphatase (ALP) activity (expressed as a percentage) in dermal papilla (DP) cells cultured in the presence of: (i) a GSK3 inhibitor and FGF-5 or (ii) a GSK3 inhibitor only, following treatment with linalyl acetate at difference concentrations. ALP activity was determined by measuring absorbance at 490nm.

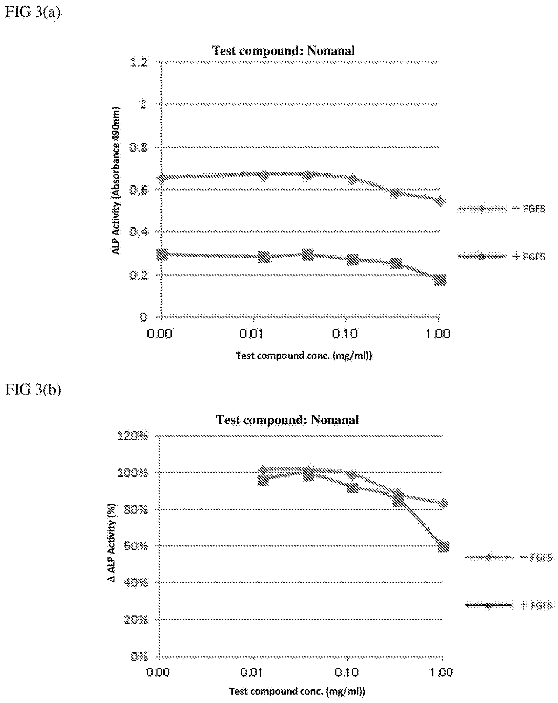

Figure 3(a) is a graphical representation showing the effect of nonanal at difference concentrations on Alkaline Phosphatase (ALP) activity in dermal papilla (DP) cells cultured in the presence of: (i) a GSK3 inhibitor and FGF-5 or (ii) a GSK3 inhibitor only. ALP activity was determined by measuring absorbance at 490nm.
Figure 3(b) is a graphical representation showing the relative difference in Alkaline Phosphatase (ALP) activity (expressed as a percentage) in dermal papilla (DP) cells cultured in the presence of: (i) a GSK3 inhibitor and FGF-5 or (ii) a GSK3 inhibitor only, following treatment with nonanal at difference concentrations. ALP activity was determined by measuring absorbance at 490nm.

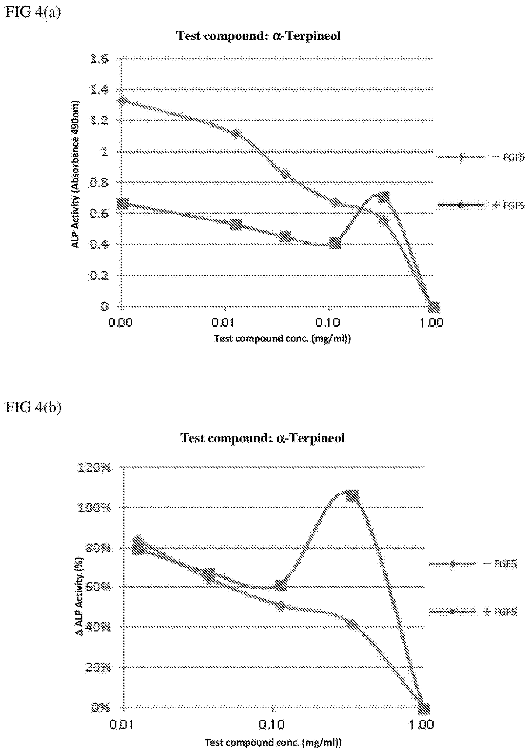

Figure 4(a) is a graphical representation showing the effect of α-Terpineol at difference concentrations on Alkaline Phosphatase (ALP) activity in dermal papilla (DP) cells cultured in the presence of: (i) a GSK3 inhibitor and FGF-5 or (ii) a GSK3 inhibitor only. ALP activity was determined by measuring absorbance at 490nm.
Figure 4(b) is a graphical representation showing the relative difference in Alkaline Phosphatase (ALP) activity (expressed as a percentage) in dermal papilla (DP) cells cultured in the presence of: (i) a GSK3 inhibitor and FGF-5 or (ii) a GSK3 inhibitor only, following treatment with α-Terpineol at difference concentrations. ALP activity was determined by measuring absorbance at 490nm.

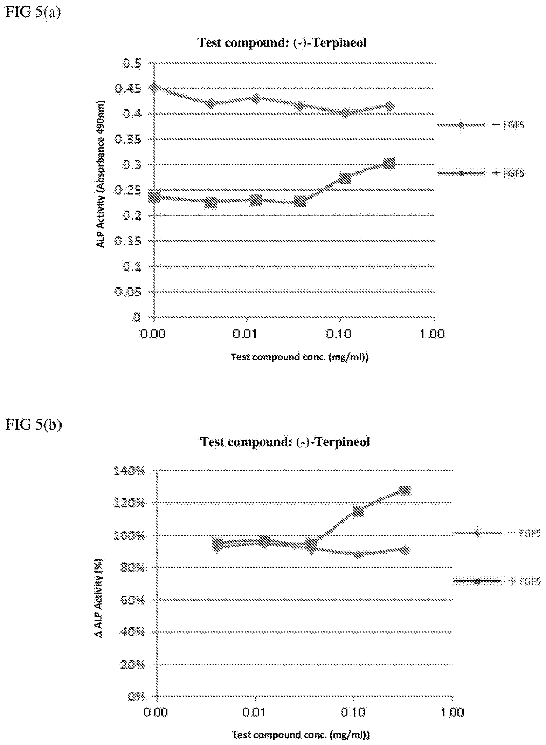

Figure 5(a) is a graphical representation showing the effect of (-)-Terpinen-4-ol at difference concentrations on Alkaline Phosphatase (ALP) activity in dermal papilla (DP) cells cultured in the presence of: (i) a GSK3 inhibitor and FGF-5 or (ii) a GSK3 inhibitor only. ALP activity was determined by measuring absorbance at 490nm.
Figure 5(b) is a graphical representation showing the relative difference in Alkaline Phosphatase (ALP) activity (expressed as a percentage) in dermal papilla (DP) cells cultured in the presence of: (i) a GSK3 inhibitor and FGF-5 or (ii) a GSK3 inhibitor only, following treatment with (-)-terpinen-4-ol at difference concentrations. ALP activity was determined by measuring absorbance at 490nm.

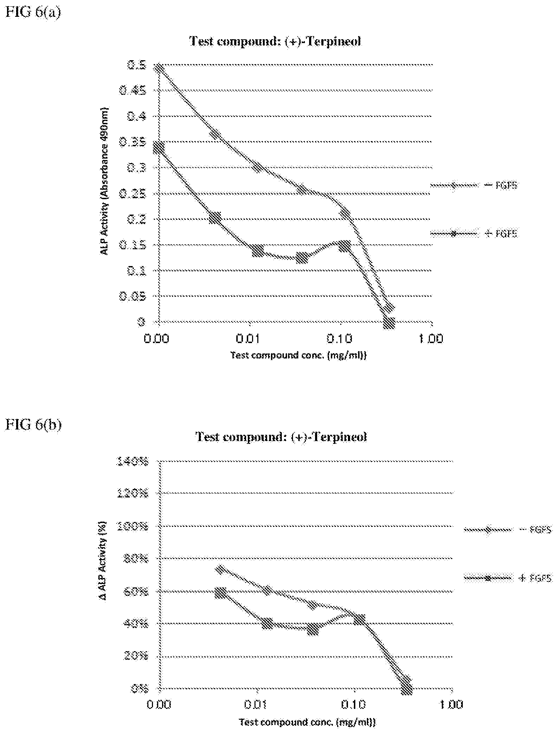

Figure 6(a) is a graphical representation showing the effect of (+)-terpinen-4-ol at difference concentrations on Alkaline Phosphatase (ALP) activity in dermal papilla (DP) cells cultured in the presence of: (i) a GSK3 inhibitor and FGF-5 or (ii) a GSK3 inhibitor only. ALP activity was determined by measuring absorbance at 490nm.
Figure 6(b) is a graphical representation showing the relative difference in Alkaline Phosphatase (ALP) activity (expressed as a percentage) in dermal papilla (DP) cells cultured in the presence of: (i) a GSK3 inhibitor and FGF-5 or (ii) a GSK3 inhibitor only, following treatment with (+)-terpinen-4-ol at difference concentrations. ALP activity was determined by measuring absorbance at 490nm.

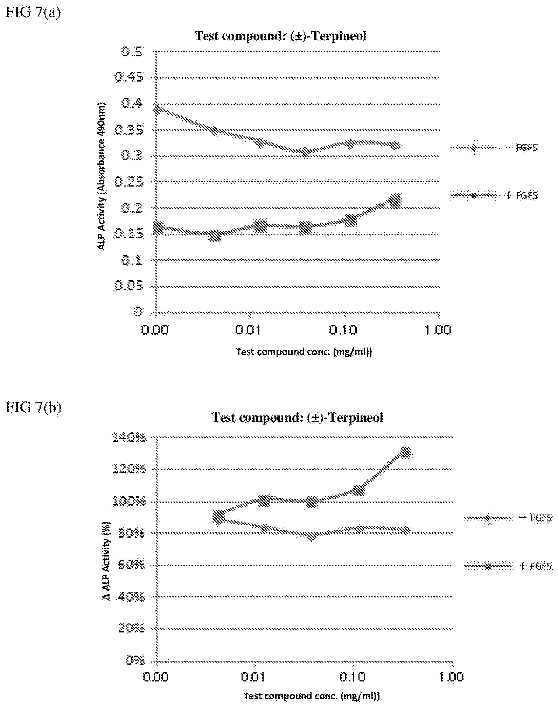

Figure 7(a) is a graphical representation showing the effect of (±)-terpinen-4-ol at difference concentrations on Alkaline Phosphatase (ALP) activity in dermal papilla (DP) cells cultured in the presence of: (i) a GSK3 inhibitor and FGF-5 or (ii) a GSK3 inhibitor only. ALP activity was determined by measuring absorbance at 490nm.
Figure 7(b) is a graphical representation showing the relative difference in Alkaline Phosphatase (ALP) activity (expressed as a percentage) in dermal papilla (DP) cells cultured in the presence of: (i) a GSK3 inhibitor and FGF-5 or (ii) a GSK3 inhibitor only, following treatment with (±)-terpinen-4-ol at difference concentrations. ALP activity was determined by measuring absorbance at 490nm.

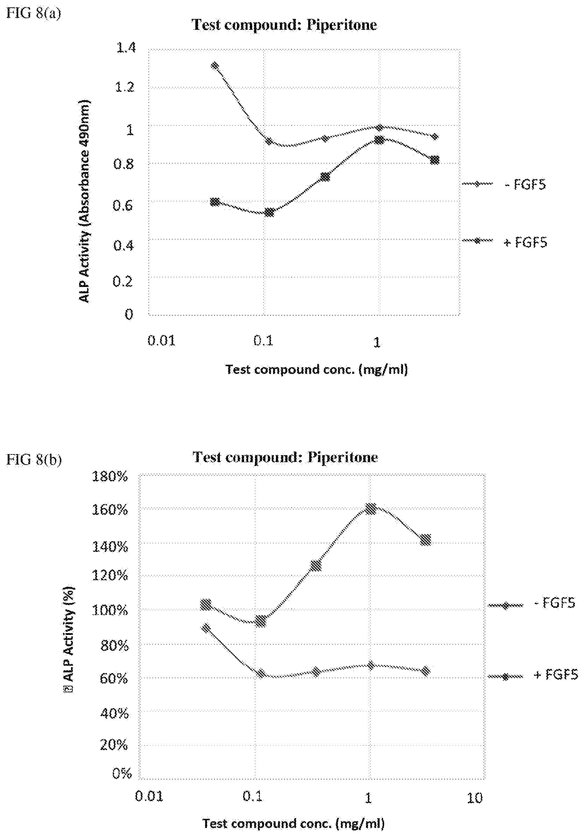

Figure 8(a) is a graphical representation showing the effect of piperitone at difference concentrations on Alkaline Phosphatase (ALP) activity in dermal papilla (DP) cells cultured in the presence of: (i) a GSK3 inhibitor and FGF-5 or (ii) a GSK3 inhibitor only. ALP activity was determined by measuring absorbance at 490nm.
Figure 8(b) is a graphical representation showing the relative difference in Alkaline Phosphatase (ALP) activity (expressed as a percentage) in dermal papilla (DP) cells cultured in the presence of: (i) a GSK3 inhibitor and FGF-5 or (ii) a GSK3 inhibitor only, following treatment with piperitone at difference concentrations. ALP activity was determined by measuring absorbance at 490nm.

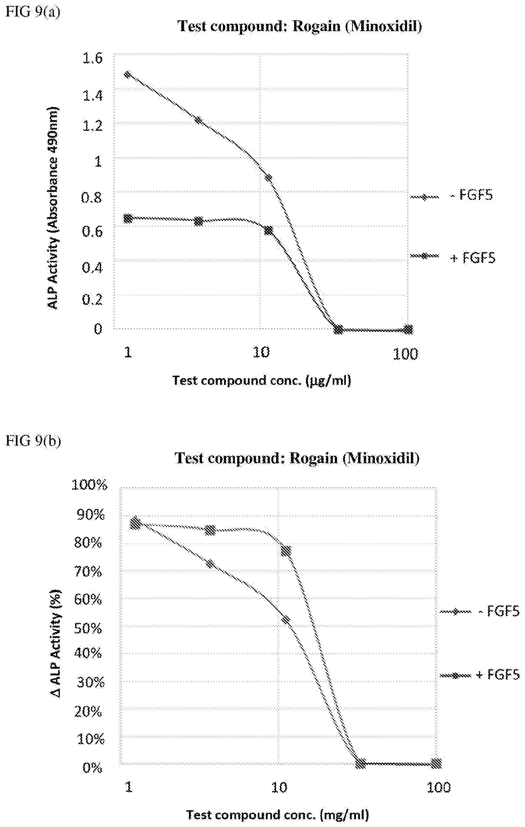

Figure 9(a) is a graphical representation showing the effect of minoxidil at difference concentrations on Alkaline Phosphatase (ALP) activity in dermal papilla (DP) cells cultured in the presence of: (i) a GSK3 inhibitor and FGF-5 or (ii) a GSK3 inhibitor only. ALP activity was determined by measuring absorbance at 490nm.
Figure 9(b) is a graphical representation showing the relative difference in Alkaline Phosphatase (ALP) activity (expressed as a percentage) in dermal papilla (DP) cells cultured in the presence of: (i) a GSK3 inhibitor and FGF-5 or (ii) a GSK3 inhibitor only, following treatment with minoxidil at difference concentrations. ALP activity was determined by measuring absorbance at 490nm.

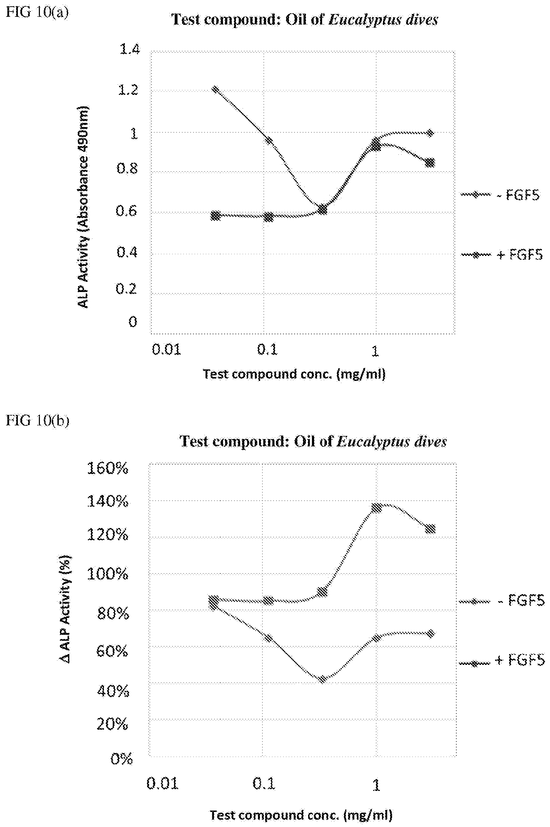

Figure 10(a) is a graphical representation showing the effect of an essential oil from *Eucalyptus dives* at difference concentrations on Alkaline Phosphatase (ALP) activity in dermal papilla (DP) cells cultured in the presence of: (i) a GSK3 inhibitor and FGF-5 or (ii) a GSK3 inhibitor only. ALP activity was determined by measuring absorbance at 490nm.
Figure 10(b) is a graphical representation showing the relative difference in Alkaline Phosphatase (ALP) activity (expressed as a percentage) in dermal papilla (DP) cells cultured in the presence of: (i) a GSK3 inhibitor and FGF-5 or (ii) a GSK3 inhibitor only, following treatment with an essential oil from *Eucalyptus dives* at difference concentrations. ALP activity was determined by measuring absorbance at 490nm.

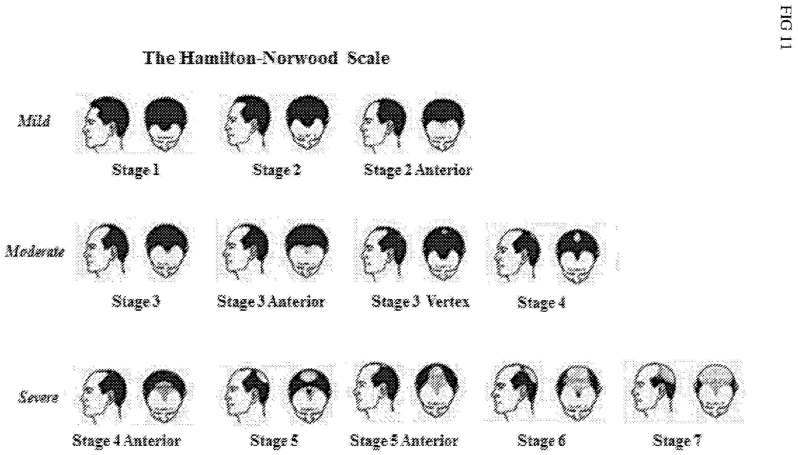

Figure 11 is a graphical representation of the Hamilton-Norwood Scale as used to assess male pattern baldness.

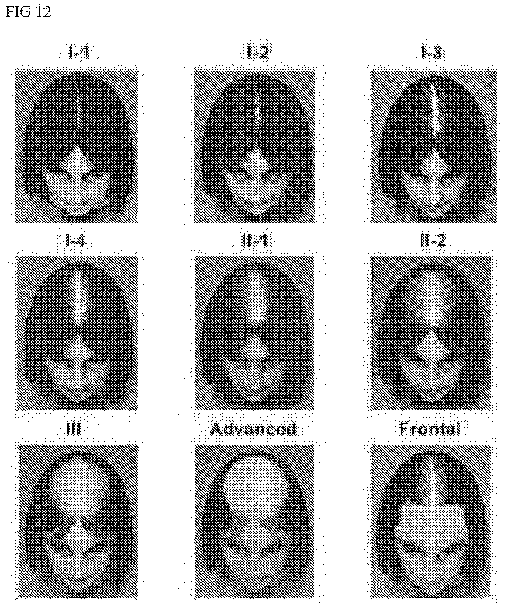

Figure 12 is a graphical representation of the Lugwig Scale as used to assess female pattern baldness.

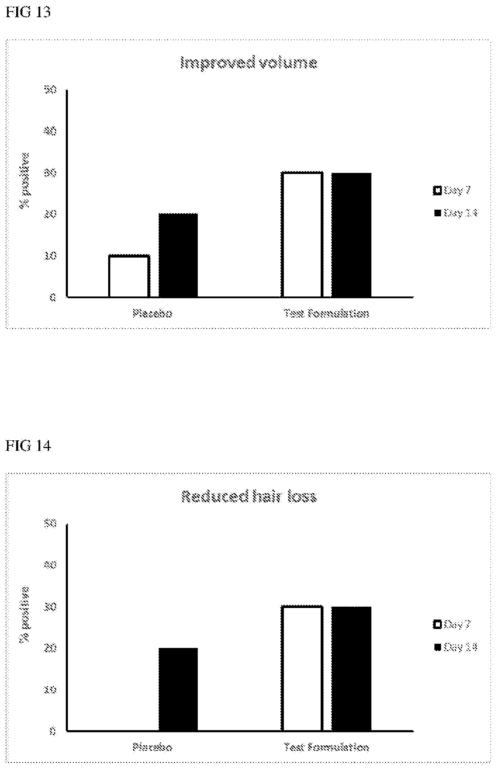

Figure 13 is a graphical representation showing the percentage of subjects receiving the Placebo and Test formulations who perceived a visual improvement in hair volume at days 7 and 14 of trial.
Figure 14 is a graphical representation showing the percentage of subjects receiving the Placebo and Test formulations who perceived a visual reduction in hair loss at days 7 and 14 of trial.

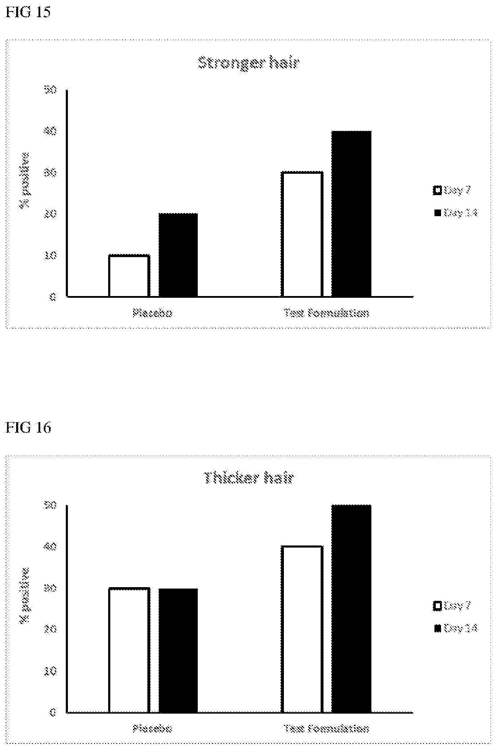

Figure 15 is a graphical representation showing the percentage of subjects receiving the Placebo and Test formulations who perceived that their hair was stronger at days 7 and 14 of trial.
Figure 16 is a graphical representation showing the percentage of subjects receiving the Placebo and Test formulations who perceived that their hair was thicker at days 7 and 14 of trial.

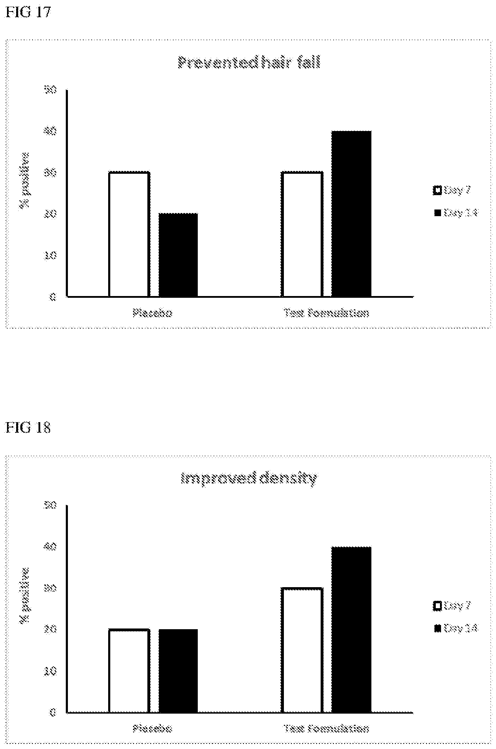

Figure 17 is a graphical representation showing the percentage of subjects receiving the Placebo and Test formulations who perceived that the respective treatment resulted in a reduction in hair fall at days 7 and 14 of the trial.
Figure 18 is a graphical representation showing the percentage of subjects receiving the Placebo and Test formulations who perceived that their hair had improved density at days 7 and 14 of the trial.

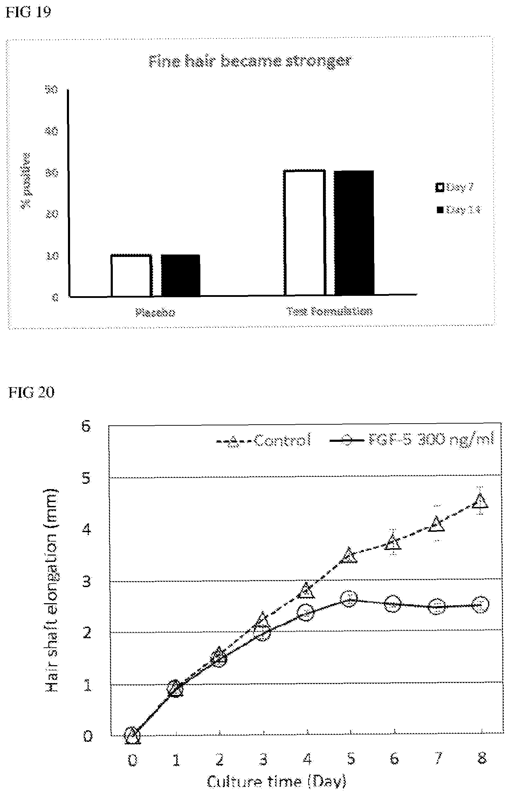

Figure 19 is a graphical representation showing the percentage of subjects receiving the Placebo and Test formulations who perceived that the respective treatment resulted in a strengthening of fine hair at days 7 and 14 of the trial.
Figure 20 is a graphical representation showing hair shaft elongation (mm) over time for hair murine vibrissae follicles cultured in the presence and absence of exogenous FGF-5.

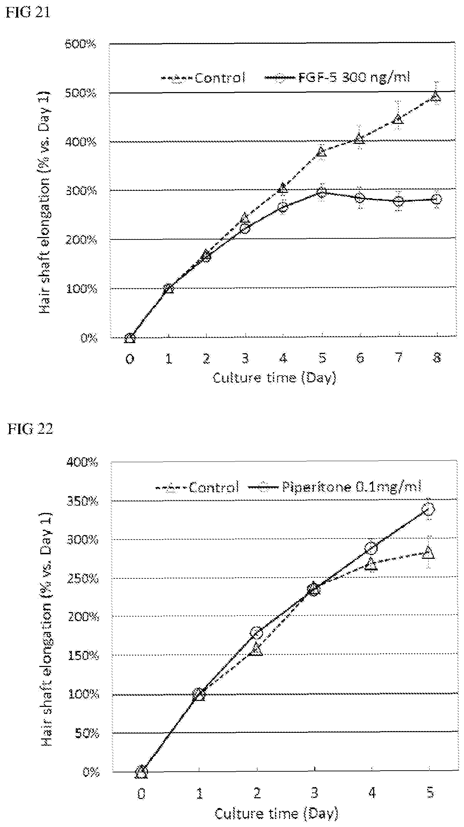

Figure 21 is a graphical representation showing rate of hair shaft elongation over time (measured as percentage growth relative to day 1) for hair murine vibrissae follicles cultured in the presence and absence of exogenous FGF-5.
Figure 22 is a graphical representation showing hair shaft elongation over time (measured as a percentage of growth relative to day 1) for hair murine vibrissae follicles cultured in the presence and absence of piperitone.

### DETAILED DESCRIPTION OF THE INVENTION

### Monoterpenoids

The present disclosure provides topical formulations comprising monoterpenoid compounds which are capable of reducing fibroblast growth factor 5 (FGF5)-dependent signalling in a hair follicle cell or part thereof. The monoterpenoid used in the invention is 2-methyl-5-(1-methylethenyl)-2-cyclohexen-1-ol (carveol) or an isolated enantiomer thereof or an isolated ester thereof with a carboxylic acid.

The term "monoterpenoid" or "monoterpenoid compound" shall be taken to mean a hydrocarbon compound having a monoterpene skeleton formed from two isoprene units *i.e.,* have the molecular formula C₁₀H₁₆, which has undergone biochemical modifications such as oxidation or rearrangement. Monoterpenoids may be acyclic, monocyclic or bicyclic. As used throughout this specification, the term "monoterpenoid compound" shall be understood to include monoterpenoids, enantiomers of monoterpenoids and monoterpenoid esters with a carboxylic acid.

Preferably, the monoterpenoid compound of the disclosure is C₁₀-monoterpenoid, or an enantiomer thereof or an ester thereof with a carboxylic acid, of formula (I): 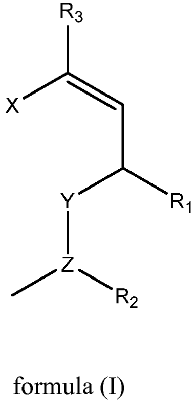 wherein:
R₁ is hydrogen, hydroxyl or oxygen;
R₂ is absent or hydrogen or hydroxyl;
R₃ is CH₃;
X is CH₃ or CH₂OH, or
X is CH₂CH₂ or CHOHCH₂ and X and Y together form a single bond within a 6-membered ring;
Y is CH₂ when X is CH₃ or CH₂OH, or
Y is CH or COH when X is CH₂CH₂ or CHOHCH₂; and
Z is a saturated or unsaturated C₂-C₅ alkyl or alkyl ester.

For example, the C₁₀-monoterpenoid may be selected from the group consisting of 3-Methyl-6-(propan-2-yl)cyclohex-2-en-1-one (piperitone), 1-Isopropyl-4-methyl-3-cyclohexen-1-ol (terpinen-4-ol), 2-(4-Methyl-3-cyclohexen-1-yl)-2-propanol (alpha-terpineol), 2-Methyl-5-(1-methylethenyl)-2-cyclohexen-1-ol (carveol), 6-Isopropyl-3-methyl-2-cyclohexen-1-one (3-carvomenthenone); and 3,7-Dimethyl-1,6-octadien-3-ol (linalool). Preferably, the C₁₀-monoterpenoid is 1-Isopropyl-4-methyl-3-cyclohexen-1-ol (terpinen-4-ol) or Methyl-6-(propan-2-yl)cyclohex-2-en-1-one (piperitone). More preferably, the C₁₀-monoterpenoid is Methyl-6-(propan-2-yl)cyclohex-2-en-1-one (piperitone).

In another example, the carboxylic acid monoester of a C₁₀-monoterpenoid of formula (I) may be a monoester with a carboxylic acid selected from acetic acid, propionic acid and formic acid. Preferably, the carboxylic acid is acetic acid. For example, a C₁₀-monoterpenoid carboxylic acid ester may be selected from the group consisting of (*2E*)-3,7-Dimethyl-2,6-octadien-1-yl acetate (geranyl acetate), 3,7-Dimethyl-1,6-octadien-3-yl acetate (linalyl acetate); 2-(4-Methyl-3-cyclohexen-1-yl)-2-propanyl acetate (terpinyl acetate); and 5-Isopropenyl-2-methyl-2-cyclohexen-1-yl acetate (carvyl acetate).

In another example, the enantiomer of a C₁₀-monoterpenoid of formula (I) may be selected from the group consisting of (*R*)-1-Isopropyl-4-methyl-3-cyclohexen-1-ol [(-)-terpinen-4-ol], (*1S*)-1-Isopropyl-4-methyl-3-cyclohexen-1-ol [(+)-terpinen-4-ol], 2-[(*1R*)-4-Methylcyclohex-3-en-1-yl]propan-2-ol [(+)-alpha-terpineol], (6*R*)-3-methyl-6-(propan-2-yl)cyclohex-2-en-1-one or (*6R*)-Isopropyl-3-methyl-2-cyclohexen-1-one [(-)-piperitone], (6*S*)-3-Methyl-6-(propan-2-yl)cyclohex-2-en-1-one [(+)-piperitone], (*3S*)-3,7-Dimethyl-1,6-octadien-3-ol [(+)-Linalool], (*3R*)-3,7-Dimethyl-1,6-octadien-3-ol [(-)-Linalool], (*1R*,*5R*)-2-Methyl-5-(1-methylethenyl)-2-cyclohexen-1-ol [(-)-*cis-*carveol], (*1S*,*5S*)-2-Methyl-5-(1-methylethenyl)-2-cyclohexen-1-ol [(+)-*cis*-carveol], (*1R,5S*)-2-Methyl-5-(1-methylethenyl)-2-cyclohexen-1-ol [(+)-*trans*-carveol], and (*1S,5R*)-2-Methyl-5-(1-methylethenyl)-2-cyclohexen-1-ol [(-)-*trans*-carveol].

Certain monoterpenoids and carboxylic acid esters thereof may contain chiral centres. It is to be understood that both racemic and diasteromeric mixtures, as well as the individual optical isomers, isolated or synthesized, which are substantially free of their enantiomeric or diastereomeric partners, are within the scope of the invention. Racemic mixtures may be separated into their individual, substantially optically pure isomers through well- known techniques, such as the separation of diastereomeric salts formed with optically active adjuncts *e.g.,* acids or bases followed by conversion back to the optically active substances. The desired optical isomer may be synthesized by means of stereospecific reactions, beginning with the appropriate stereoisomer of the desired starting material.
Monoterpenoid compounds which are capable of reducing fibroblast growth factor 5 (FGF5)-dependent signalling in a hair follicle cell or part thereof may be identified by any method known in the art for doing so. For example, such compounds may be identified by performing an Alkaline Phosphatase Dermal Papilla (ALP-DP) cell assay as described in WO2013/105417. Alternatively, or in addition, a monoterpenoid compound may be screened by one or more of the assays exemplified herein to determine whether or not it is capable of reducing FGF5-dependent signalling.

For example, a C₁₀-monoterpenoid or ester or enantiomer thereof useful in a topical formulation used in the invention will reduce or inhibit FGF-5 activity in the hair follicle or part thereof by about 10-90% or 20-90% or 30-90% or 40-90% or 50-90% or 60-90% or 70-90% or 80-90% or 10-80% or 20-80% or 30-80% or 40-80% or 50-80% or 60-80% or 70-80% or 10-70% or 20-70% or 30-70% or 40-70% or 50-70% or 60-70% or 10-60% or 20-60% or 30-60% or 40-60% or 50-60% or 10-50% or 20-50% or 30-50% or 40-50% or 10-40% or 20-40% or 30-40% or 10-30% or 20-30%. Alternatively, or in addition, a C₁₀-monoterpenoid or ester or enantiomer thereof useful in a topical formulation used in the invention will reduce or inhibit FGF-5 activity in the hair follicle or part by at least 10% or at least 20% or at least 30% or at least 40% or at least 50% or at least 60% or at least 70% or at least 80% or at least 90%.

Alternatively, or in addition, a C₁₀-monoterpenoid or ester or enantiomer thereof useful in a topical formulation used in the invention will reduce or inhibit FGF-5 binding to a cognate fibroblast growth factor receptor (FGFR) in the hair follicle or part thereof. For example, a C₁₀-monoterpenoid or ester or enantiomer thereof useful in a topical formulation used in the invention will reduce or inhibit FGF-5 binding to fibroblast growth factor receptor 1 (FGFR1) by about 10-90% or 20-90% or 30-90% or 40-90% or 50-90% or 60-90% or 70-90% or 80-90% or 10-80% or 20-80% or 30-80% or 40-80% or 50-80% or 60-80% or 70-80% or 10-70% or 20-70% or 30-70% or 40-70% or 50-70% or 60-70% or 10-60% or 20-60% or 30-60% or 40-60% or 50-60% or 10-50% or 20-50% or 30-50% or 40-50% or 10-40% or 20-40% or 30-40% or 10-30% or 20-30%. Alternatively, or in addition, a C₁₀-monoterpenoid or ester or enantiomer thereof useful in a topical formulation used in the invention will reduce or inhibit FGF-5 binding to FGFR1 by at least 10% or at least 20% or at least 30% or at least 40% or at least 50% or at least 60% or at least 70% or at least 80% or at least 90%. The ability of a C₁₀-monoterpenoid or ester or enantiomer thereof to reduce binding of FGF-5 to FGFR1 may be determined by a reduction in viability of a BaF3 cell expressing FGFR1, wherein the BaF3 cell is dependent on FGF-5 signalling for viability.

### Sources of monoterpenoids and enantiomers and carboxylic acid esters thereof

The monoterpenoid compounds and/or enantiomers thereof and/or carboxylic acid esters thereof may be produced in microbial, yeast and/or plant cell culture systems known in the art, including microbial, yeast and/or plant cell culture systems which have been metabolically engineered to increase synthesis/production of monoterpenoids. *See e.g.,* WO2011123576; Grover et al., Plant Cell, Tissue & Organ Culture, 108(2):323-331, 2012; Reiling et al., Biotechnology and Bioengineering, 87(2):200-212, 2004; Albrecht et al., Biotechnology Letters, 21:791-795, 1999.

Alternatively, the monoterpenoid compounds and/or enantiomers thereof and/or carboxylic acid esters thereof may be synthetic compounds. Synthetic monoterpenoid compounds are well known in the art and are readily available from a variety of commercial sources. For example, a topical formulation used in the invention may consist
of or comprise a fragrance oil or perfume oil or a perfume derived from a fragrance oil or a perfume oil.

Alternatively, the monoterpenoid compounds and/or enantiomers thereof and/or carboxylic acid esters thereof may be natural compounds.

### Essential oils

The monoterpenoids and/or enantiomers thereof and/or carboxylic acid esters thereof may be in the form of a natural extract, or comprise a natural extract, such as essential oil or a perfume derived from an essential oil. *See e.g.,* Table 1 below:

**Table 1.**

| **Compound** | **Source of essential oil** |
|---|---|
| Geraniol | Geraniol is found in essential oils of rose (∼15% w/v), palmarosa (∼80% w/v), *Cymbopogon spp* (∼40-65% w/v), citronella java (∼23% w/v), lemon balm (∼20-40% w/v) and geranium (∼10-30% w/v). |
| Nerol | Nerol is an isomeric alcohol found in essential oils from devana (∼10% w/v), neroli and petitgrain of lemon(∼5% w/v). |
| β-Citronellol | Found in citronella oils (10-50% w/v), as well as oils from geraniums (∼10-40% w/v) rose (18-55% w/v). |
| Linalool | Linalool can be found as (+)- and (-)-forms in oil of majoram (∼60-80% w/v), basil (-40-60% w/v), lavender (40-50% w/v), bergamot (∼15% w/v), *Pelargonium* geraniums (∼10-15% w/v), neroli bigarde (∼50% w/v) and Ylang ylang (∼10% w/v). |
| α-Terpineol | Found in essential oils of *Anthemis altissima* L. var. *altissima* (∼25% w/v), clary sage oil (∼47% w/v), lavandin (∼9% w/v), marjoram (∼5-25% w/v), petitgrain (∼5% w/v), cajuput (∼5% w/v), tea tree (1.5-8% w/v). |
| β-Terpineol | Isomeric with α-terpineol, but is not isolated from natural sources in sufficient amounts. Found in commercial terpineol. |
| (±)-Terpinen-4-ol | Synthetic racemate *e.g.,* 50% (w/w) (-)-Terpinen-4-ol and 50% (w/w) (+)-Terpinen-4-ol. |
| (-)-Terpinen-4-ol | Found in *Eucalyptus dives* (∼3-5% w/v)¹. Also found in tea tree oil² and essential oil of sweet marjoram³ and lavender⁴ |
| (+)-Terpinen-4-ol | Found in tea tree oil² and essential oil of sweet marjoram³ and lavender⁴ |
| Nonanal | Found in essential oils from mosses, such as in oil of Tortula muralis (∼18% w/v), *Homalothecium lutescens* (∼36% w/v), *Hypnum cupressiforme* (∼2.5% w/v) *Pohlia nutans* (∼8% w/v). |
| Menthol | A constituent of oil from the *Mentha* genus (∼30-75% w/v). |
| *l*-Carveol | Found in essential oil from caraway seed (>50% w/v), Spearmint (∼65-70% w/v) and dill (∼1.5% w/v). |
| Piperitone | Found in oil from *Eucalyptus dives* (∼35-60% w/v), *Cymbopogon spp* also known as lemon grass (-55-80% w/v), and *Artemisia deserti krasch* (∼10-50% w/v), as well as in oil from plants of *Mentha* genus (∼15-20% w/v). |
| Linalyl acetate | Found in essential oils of bergamot (∼15-55% w/v), bergamot mint (∼40-80% w/v), lavender (∼20-40% w/v), lavandin (∼20-40% w/v), marjoram (∼20-25% w/v), thyme (∼25% w/v), and clary sage (∼15-70% w/v). It is the acetate ester of linalool and the two are often present together. |
| Geranyl acetate | Geranyl acetate is a constituent of numerous essential oils, including oils from carrot seed (∼35-75% w/v), from citronella (∼2-5% w/v), palmarosa (∼10% w/v), thyme (∼25% w/v), clary sage (∼5-10% w/v), lemongrass (∼1-5% w/v) and coriander seed (∼5% w/v). |

| | |
|---|---|
| 1. The Terpinen-4-ol in *E*. *dives* oil is predominantly the (-)-Terpinen-4-ol enantiomeric form. *E*. *dives* oil is a good source of the enantiomer, which is more readily isolated from *E*. *dives* than from tea tree or sweet marjoram or lavender oil. 2, Tea tree oil may comprise about 30-50% (w/v) Terpinen-4-ol, of which about 65% *i.e.* 19.5-32.5% (w/v/) is (+)-Terpinen-4-ol and only 35% *i.e*., 10.5-17.5% (w/v/) is (-)-Terpinen-4-ol. 3. Sweet marjoram oil may comprise about 18-22% (w/v) Terpinen-4-ol, of which about 73% *i.e.* 13-16% (w/v/) is (+)-Terpinen-4-ol and only 27% *i.e*., 5-6% (w/v/) is (-)-Terpinen-4-ol. 4. Lavender oil may comprise about 4.9-9.5% (w/v) Terpinen-4-ol, of which about 98.5% *i.e.* 4.8-9.4% (w/v/) is (+)-Terpinen-4-ol and only about 1.5% *i.e*., 0.1% (w/v/) is (-)-Terpinen-4-ol. | |

Conveniently, an essential oil will be prepared from a plant source that provides an active monoterpenoid at a concentration sufficient to perform the invention, preferably without a need for any concentration of the oil and/or separation of enantiomeric forms.

Techniques for extracting essential oils from natural materials, such as from plants, algae, fungi and yeast, are known and described in the art. A preferred method of extracting essential oils from natural materials in accordance with the present invention is distillation, such as by steam distillation or water distillation (also known as "hydrodistillation").

In steam distillation, the natural material from which the essential oil is to be extracted, such as plant foliage, bark or twigs etc., is introduced into a distilling chamber through which steam is to be passed. Typically, the distilling chamber is configured to support the natural material in a manner which exposes the oil-rich areas of the material to steam when passed through the chamber. In one example, the natural material is suspended or held above water contained in the distilling chamber such that when the water is boiled, "wet steam" produced therefrom rises and contacts the essential oil containing natural material. In another example, steam is produced in a boiler and pumped into the distilling chamber containing the natural material from which the oil is to be extracted. This is sometimes referred to as "dry steam". In either case, steam is typically generated with a temperature between 100-105°C and passed through the distilling chamber containing the essential oil-containing material. As the steam contacts the natural material, the cells and vesicles containing essential oils are disrupted and the essential oils are released in the form of vapour. The vapour flow of essential oil and steam is typically directed to a condenser unit in which the vapour is condensed *e.g.,* by a water cooled jacket surrounding the condenser unit, to form a liquid distillate having an aqueous phase and an oil phase. The liquid distillate is directed into a collection vessel and the essential oil (oil phase) is separated from the hydrosol or aqueous portion (aqueous phase) according to the relative specific densities. The essential oil obtained from the distillate may be collected and used in accordance with the invention.

Although specific reference is made herein to a steam distillation process, it is to be understood that any extraction process that allows for the separation and collection of essential oils from the water soluble components and starting materials e.g. leaves, twigs, sticks, bark, roots, etc., can be used according to the present invention.

Examples of other extraction processes include direct "hydrodistillation" in which the natural material is boiled in an aqueous solution and the vapors produced therefrom are collected and condensed to produce a distillate from which the essential oil may be separated. Other extraction processes that involve partial refluxing, solvent extraction and chromatography to remove essential oils are also contemplated for use in the present invention. For example, physical processes for the isolation of monoterpenoid compounds from naturally-occurring materials, including distillation, solvent extraction, and chromatography are described in Ziegler and Ziegler, Flavourings: production, compositions, applications, regulations. 1st Ed. Wiley-VCH, Weinheim, Germany.

A preferred essential oil will provide an effective amount of an active monoterpenoid or enantiomer or carboxylic acid derivative in downstream processing, such as to produce a perfume or other formulation used in the invention, or to substantially purify the compound for other formulations disclosed herein.

Preferred topical formulations of the disclosure comprise an essential oil or perfume comprising piperitone or enantiomer thereof in an amount useful for performing the invention. For example, the working examples hereof demonstrate that piperitone elicits high alkaline phosphatase activity in dermal papillae. Accordingly, the essential oils of *Eucalyptus dives* and/or *Cymbopogon spp.* and/or lemon grass and/or *Artemisia deserti* krasch and/or *Mentha spp.,* and perfumes and other topical formulations of the disclosure derived there from are useful in performing the invention.

Other preferred topical formulations of the disclosure comprise an essential oil comprising terpinen-4-ol or enantiomer thereof, such as (-)-terpinen-4-ol, in amount(s) useful for performing the present disclosure. For example, the working examples hereof demonstrate that (-)-terpinen-4-ol elicits high alkaline phosphatase activity in dermal papillae and also has high FGF-5 inhibitory activity. Accordingly, the essential oils of *Eucalyptus dives* and/or tea tree and/or sweet marjoram, and perfumes and other topical formulations of the disclosure derived there from are useful in the present disclosure.

Particularly preferred topical formulations of the disclosure comprise an essential oil comprising piperitone or enantiomer thereof and/or terpinen-4-ol or enantiomer thereof, such as, for example piperitone and/or (-)-terpinen-4-ol, in amount(s) useful for performing the disclosure. For example, an essential oil from *Eucalyptus dives* is a suitable source of both and both piperitone and (-)-terpinen-4-ol in amounts for use in performing the present disclosure. The essential oil of *E. dives* is particularly preferred for performing the disclosure because that oil has a high content of both piperitone and (-)-terpinen-4-ol and because, as demonstrated herein, (i) piperitone and (-)-terpinen-4-ol each elicit higher alkaline phosphatase activity in dermal papillae than a racemic mixture of terpinen-4-ol or (+)-Terpinen-4-ol, and (ii) the (-)-terpinen-4-ol enantiomer has higher FGF-5 inhibitory activity than (+)-terpinen-4-ol. Accordingly, the *E. dives* essential oil provides an advantage in having both piperitone and (-)-terpinen-4-ol relative to essential oils that have predominantly (+)-terpinen-4-ol with little or no measurable piperitone such as tea tree or sweet marjoram or lavender, or essential oils that have high levels of piperitone with little or no measurable (-)-terpinen-4-ol such as the essential oils from *Cymbopogon spp* or *Artemisia deserti* krasch or *Mentha spp.* Thus, the essential oils of tea tree, sweet marjoram, lavender, *Cymbopogon spp., Artemisia deserti* krasch or *Mentha spp.* are less desirable in some embdoments than the essential oil of *E. dives.*

The relative amounts of different monoterpenoids, enantiomers and carboxylic acid derivatives that are active in performing this invention may be the same in an essential oil as in a plant extract from which the essential oil is derived, or those relative amounts may be different. The skilled artisan will also be aware that the concentration of a given monoterpenoid compound may vary between different plant extracts. Notwithstanding these variables, it is within the skill of such a person to produce an essential oil having a suitable amount of an active monoterpenoid, enantiomer or carboxylic acid derivative, or suitable amounts of different active monoterpenoids, enantiomers or carboxylic acid derivatives. In this respect, the concentration of a given synthetic monoterpenoid and/or enantiomer and/or carboxylic acid ester in an essential oil or fragrance oil or perfume may be determined without undue burden. For example, as described herein above, headspace sampling permits analysis and quantitation of an amount of constituent monoterpenoid compound(s) and/or enantiomer(s) thereof and/or carboxylic acid ester(s) thereof by gas chromatography (GC) and/or mass spectrometry (MS) processes. Such analysis of oil and/or perfume samples permits determination of an amount of monoterpenoid and/or enantiomer and/or carboxylic acid ester in an oil or perfume or powder produced therefrom on either a weight basis *e.g.,* weight of the active compound relative to weight of powder or dried oil, or alternatively on a volume basis *e.g.,* weight of active compound per unit volume of oil or perfume. Knowledge of a volume of oil from which a powder is obtained also permits calculation of an amount of active monoterpenoid and/or enantiomer and/or carboxylic acid ester on a volume basis *e.g.,* weight of active compound per unit volume of oil or perfume. Knowledge of an amount of plant material that produced an essential oil from which analysed samples were taken also permits determination of an amount of monoterpenoid and/or enantiomer and/or carboxylic acid ester on a weight basis *e.g.,* weight of the active compound per gram dried weight of plant material.

The weight of monoterpenoid and/or enantiomer and/or carboxylic acid ester in an oil or perfume may also be known or readily derived, such as when the oil is prepared using purified compounds or starting material having a known concentration of the active compound(s), to facilitate determination of compound concentrations in the topical formulation.

Depending upon the concentration of active compound(s) in a synthetic preparation, a fragrance oil having a suitable concentration of one or more non-volatile active compounds is prepared readily by evaporation. A fragrance oil having a suitable concentration of one or more active compounds is also prepared readily by dilution using ethanol or other suitable diluent known in the art.

Combinations of oils are especially preferred when it desirable to combine active monoterpenoids or enantiomers or carboxylic acid esters thereof, which are not each represented in sufficiently-high concentrations in a single oil to have a cosmetic or therapeutic effect, or that are not each present in a single oil in amounts that are processed conveniently without combination. The skilled artisan will be aware that it is possible to combine monoterpenoids and/or enantiomers and/or carboxylic acid esters thereof that are active in performing the invention by combining one or more perfume oils and/or one or more essential oils to achieve optimum concentrations of active compounds as determined by the activity profile(s) of the constituents as described herein.

### Perfumes

In another preferred example, the topical formulation used in the invention consists of or comprises a perfume derived from a fragrance oil or perfume oil or essential oil or combination thereof. The perfume may comprise one oil, such as one essential oil or one fragrance oil, or it may comprise a combination of different oils, including a combination of essential oils, a combination of fragrance oils, or a combination of both essential oils and fragrance oils.

A perfume may be classified as "parfum" and comprise an amount of oil in a range from about 15% to about 25% by volume, including 15% or 16% or 17% or 18% or 19% or 20% or 21% or 22% or 23% or 24% or 25% oil by volume, in aqueous solution such as ethanol and/or water. Alternatively, a perfume may be classified as "soie de parfum" and comprise an amount of oil in a range from about 15% to about 18% by volume, including 15% or 16% or 17% or 18% oil by volume, in aqueous solution such as ethanol and/or water. Alternatively, a perfume may be classified as "eau" and comprise an amount of oil in a range not exceeding about 15% by volume, including up to 1% or up to 2% or up to 3% or up to 4% or up to 5% or up to 6% or up to 7% or up to 8% or up to 9% or up to 10% or up to 11% or up to 12% or up to 13% or up to 14% or up to 15% oil by volume, in aqueous solution such as ethanol and/or water. For example, within such a range of concentration values, a perfume classified as "eau fraiche" may comprise about 3% or less oil by volume, a perfume classified as "eau de cologne" may comprise about 2% to about 5% oil by volume, a perfume classified as "eau de toilette may comprise about 4% to about 10% oil by volume, and a perfume classified as "eau de parfum" may comprise about 8% to about 15% oil.

In a preferred example, the topical formulation used in the present invention is a an eau comprising one or more monoterpenoids used in the invention in aqueous ethanol solution, e.g., eau fraiche or eau de cologne or eau de toilette or eau de parfum, and more preferably, an eau fraiche or eau de cologne or eau de toilette.

An exemplary eau fraiche of the disclosure will comprise not more than 3% by volume of an essential oil, such as an essential oil from *E. dives* and/or an essential oil *Cymbopogon spp* and/or an essential oil from A*rtemisia deserti krasch* and/or an essential oil from a plant of the *Mentha* genus and/or an essential oil of tea tree and/or an essential oil of sweet marjoram. Such a perfume will comprise at least effective amounts of piperitone or enantiomer thereof such as (-)-piperitone or (+)-piperitone and terpinen-4-ol or enantiomer thereof such as (-)-terpinen-4-ol piperitone or enantiomer thereof. Alternatively, an exemplary eau fraiche of the disclosure will comprise not more than 3% by volume of fragrance oil comprising piperitone or enantiomer thereof such as (-)-piperitone or (+)-piperitone and not more than 3% by volume of terpinen-4-ol or enantiomer thereof such as (-)-terpinen-4-ol. Alternatively, an exemplary eau fraiche of the disclosure will comprise not more than 3% by volume of fragrance oil comprising piperitone or enantiomer thereof such as (-)-piperitone or (+)-piperitone and/or a fragrance oil comprising not more than 3% by volume of terpinen-4-ol or enantiomer thereof such as (-)-terpinen-4-ol. As used herein, the term "not more than 3%" includes 0.01% or 0.05% or 0.1% or 0.15% or 0.2% or 0.25% or 0.3% or 0.35% or 0.4% or 0.45% or 0.5% or 0.55% or 0.6% or 0.65% or 0.7% or 0.75% or 0.8% or 0.85% or 0.9% or 0.95% or 1.0% or 1.1% or 1.2% or 1.3% or 1.4% or 1.5% or 1.6% or 1.7% or 1.8% or 1.9% or 2.0% or 2.1% or 2.2% or 2.3% or 2.4% or 2.5% or 2.6% or 2.7% or 2.8% or 2.9% or 2.01% or 2.92% or 2.93% or 2.94% or 2.95% or 2.96% or 2.97% or 2.98% or 2.99%.

An exemplary eau de cologne of the disclosure will comprise not less than 2% by volume and not more than 5% by volume of an essential oil, such as an essential oil from *E. dives* and/or an essential oil *Cymbopogon spp* and/or an essential oil from A*rtemisia deserti krasch* and/or an essential oil from a plant of the *Mentha* genus and/or an essential oil of tea tree and/or an essential oil of sweet marjoram. Such a perfume will comprise at least effective amounts of piperitone or enantiomer thereof such as (-)-piperitone or (+)-piperitone and terpinen-4-ol or enantiomer thereof such as (-)-terpinen-4-ol piperitone or enantiomer thereof. Alternatively, an exemplary eau fraiche of the disclosure will comprise not less than 2% by volume and not more than 5% by volume of fragrance oil comprising piperitone or enantiomer thereof such as (-)-piperitone or (+)-piperitone in combination with not less than 2% by volume and not more than 5% by volume of terpinen-4-ol or enantiomer thereof such as (-)-terpinen-4-ol. Alternatively, an exemplary eau fraiche of the disclosure will comprise not less than 2% by volume and not more than 5% by volume of fragrance oil comprising piperitone or enantiomer thereof such as (-)-piperitone or (+)-piperitone and/or a fragrance oil comprising not less than 2% by volume and not more than 5% by volume of terpinen-4-ol or enantiomer thereof such as (-)-terpinen-4-ol. As used herein, the term "not less than 2% and not more than 5%" includes 2.0% or 2.1% or 2.2% or 2.3% or 2.4% or 2.5% or 2.6% or 2.7% or 2.8% or 2.9% or 3.0% or 3.1% or 3.2% or 3.3% or 3.4% or 3.5% or 3.6% or 3.7% or 3.8% or 3.9% or 4.0% or 4.1% or 4.2% or 4.3% or 4.4% or 4.5% or 4.6% or 4.7% or 4.8% or 4.9% or 4.91% or 4.92% or 4.93% or 4.94% or 4.95% or 4.96% or 4.97% or 4.98% or 4.99% or 5%.

An exemplary eau de toilette of the disclosure will comprise not less than 4% by volume and not more than 10% by volume of an essential oil, such as an essential oil from *E. dives* and/or an essential oil *Cymbopogon spp* and/or an essential oil from A*rtemisia deserti krasch* and/or an essential oil from a plant of the *Mentha* genus and/or an essential oil of tea tree and/or an essential oil of sweet marjoram. Such a perfume will comprise at least effective amounts of piperitone or enantiomer thereof such as (-)-piperitone or (+)-piperitone and terpinen-4-ol or enantiomer thereof such as (-)-terpinen-4-ol piperitone or enantiomer thereof. Alternatively, an exemplary eau fraiche of the disclosure will comprise not less than 4% by volume and not more than 10% by volume of fragrance oil comprising piperitone or enantiomer thereof such as (-)-piperitone or (+)-piperitone in combination with not less than 4% by volume and not more than 10% by volume of terpinen-4-ol or enantiomer thereof such as (-)-terpinen-4-ol. Alternatively, an exemplary eau fraiche of the disclosure will comprise not less than 4% by volume and not more than 10% by volume of fragrance oil comprising piperitone or enantiomer thereof such as (-)-piperitone or (+)-piperitone and/or a fragrance oil comprising not less than 4% by volume and not more than 10% by volume of terpinen-4-ol or enantiomer thereof such as (-)-terpinen-4-ol. As used herein, the term "not less than 4% and not more than 10%" includes 4.0% or 4.1% or 4.2% or 4.3% or 4.4% or 4.5% or 4.6% or 4.7% or 4.8% or 4.9% or 5.0% or 5.1% or 5.2% or 5.3% or 5.4% or 5.5% or 5.6% or 5.7% or 5.8% or 5.9% or 6.0% or 6.1% or 6.2% or 6.3% or 6.4% or 6.5% or 6.6% or 6.7% or 6.8% or 6.9% or 7.0% or 7.1% or 7.2% or 7.3% or 7.4% or 7.5% or 7.6% or 7.7% or 7.8% or 7.9% or 8.0% or 8.1% or 8.2% or 8.3% or 8.4% or 8.5% or 8.6% or 8.7% or 8.8% or 8.9% or 9.0% or 9.1% or 9.2% or 9.3% or 9.4% or 9.5% or 9.6% or 9.7% or 9.8% or 9.9% or 10.0%.

### Formulations

A monoterpenoid or enantiomer or carboxylic acid derivative thereof described according to any example hereof may be formulated in any form used in the pharmaceutical, quasi-drug, or cosmetic field, suitable for topical administration to a human or mammal.

Conveniently, topical formulations used in the invention, including pharmaceutical and cosmetic forms, are prepared by dilution of an essential oil or substantially purified compound.

Such pharmaceutical and cosmetic formulations include essential oils, perfume oils, perfumes, ointments, liniments, creams, shampoos, lotions, pastes, jellies, sprays, aerosols, or in patches or impregnated dressings. For example, the topical formulation may be a product for preventing and/or treating hair loss, a product for growing hair, a hair or scalp cosmetic (*e.g.* shampoo, hair conditioner, scalp lotion, scalp cream, hair tonic, etc.), a skincare product (*e.g.* lotion, cream, face cream, face lotion, milk, pack, liquid facial wash, soap, etc.), a body care product (*e.g.* body cream, body lotion, soap, liquid wash, bath additive, etc.), a UV protective agent (e.g. sun block, sunscreen lotion, tanning oil, etc.), or a cosmetic (*e.g.* eyeliner, eyebrow pencil, cream, lotion, etc). The term "ointment" embraces formulations (including creams) having oleaginous, water-soluble and emulsion-type bases, *e.g.,* petrolatum, lanolin, polyethylene glycols, as well as mixtures thereof. These may be applied directly to the skin or an area of dermis comprising hair follicles.

In producing a formulation used in the invention, an essential oil or perfume oil or perfume, or one or more isolated active monoterpenoids and/or enantiomers thereof and/or carboxylic acid esters thereof is/are presented in an amount suitable for performing the invention *i.e.,* producing an efficacious result in a cosmetic or therapeutic context described herein. The amount may vary depending on the nature of the formulation, the purpose, and the duration of treatment or cosmetic application.

In one example, a concentration of each active C₁₀-monoterpenoid or ester or enantiomer thereof is an amount that is present in an essential oil having the desired cosmetic or therapeutic activity, prepared by conventional procedures for a plant material that produces the active compound as a secondary metabolite. Preferred formulations comprise essential oils described herein, including essential oil from *E. dives* and/or an essential oil *Cymbopogon spp* and/or an essential oil from A*rtemisia deserti krasch* and/or an essential oil from a plant of the *Mentha* genus and/or an essential oil of tea tree and/or an essential oil of sweet marjoram. Particularly preferred formulations comprise essential oil from *E. dives.* Alternatively, a concentration of each active C₁₀-monoterpenoid or ester or enantiomer thereof is an amount that is present in a combination of such essential oils *e.g.,* essential oil from *E. dives* in combination with an essential oil from tea tree and/or an essential oil of sweet marjoram. Conveniently, the essential oil is not processed to concentrate the active agent(s), but comprises the active agent(s) in sufficient concentration(s) to provide for use of the essential oil in an undiluted form, or diluted during downstream processing using an aqueous solvent suitable for topical use, *e.g.,* ethanol in water, to an effective concentration of the active agent(s). One or more carriers, excipients, emollients, diluents, fillers, dispersants, stabilisers, preservatives, emulsifying agents, solubilizing agents, anti-crystallization agents, surfactants, cosmetic components, or adjunctive agents, may be added to an essential oil provided that the final concentration of each active C₁₀-monoterpenoid or ester or enantiomer thereof is an amount having the desired cosmetic or therapeutic activity.

In the case of a perfume derived from an essential oil, the concentration of the active C₁₀-monoterpenoid or ester or enantiomer thereof is in an amount that ensures classification of the formulation as a perfume as described, with or without additional carriers, excipients, emollients, diluents, fillers, dispersants, stabilisers, preservatives, emulsifying agents, solubilizing agents, anti-crystallization agents, surfactants, cosmetic components, or adjunctive agents. For example, the essential oil(s) described according to any example hereof is(are) diluted in aqueous ethanol (up to about 50% (v/v) including 10% (v/v) or 20% (v/v) or 30% (v/v) or 40% (v/v) or 50% (v/v) ethanol solution) to provide a final concentration of compound having the desired activity and desired concentration of essential oil. In another example, the essential oil as described according to any example hereof may be diluted in aqueous ethanol up to about 70% (v/v), including 10% (v/v) or 20% (v/v) or 30% (v/v) or 40% (v/v) or 50% (v/v) or 60% (v/v) or 70%(v/v). In another example, the or each C₁₀-monoterpenoid or ester or enantiomer thereof, or a perfume oil comprising same, as described according to any example hereof may be diluted in aqueous ethanol up to about 70% (w/v), including 10% (w/v) or 20% (w/v) or 30% (w/v) or 40% (w/v) or 50% (w/v) or 60% (w/v) or 70%(w/v). In yet another example, the or each C₁₀-monoterpenoid or ester or enantiomer thereof, or a perfume oil comprising same, as described according to any example hereof may be diluted in aqueous ethanol up to about 70% (w/w), including 10% (w/w) or 20% (w/w) or 30% (w/w) or 40% (w/w) or 50% (w/w) or 60% (w/w) or 70%(w/w).

In the case of a perfume oil or perfume derived therefrom, or other topical formulation comprising substantially purified C₁₀-monoterpenoids or esters or enantiomers thereof, the concentration of each isolated active C₁₀-monoterpenoid or ester or enantiomer thereof is formulated in an amount consistent with the activity profile of that compound, with or without additional carriers, excipients, emollients, diluents, fillers, dispersants, stabilisers, preservatives, emulsifying agents, solubilizing agents, anti-crystallization agents, surfactants, cosmetic components, or adjunctive agents. For example, each C₁₀-monoterpenoid or ester or enantiomer thereof, or a perfume oil comprising same, as described according to any example hereof is diluted in aqueous ethanol (up to about 50% (v/v) including 10% (v/v) or 20% (v/v) or 30% (v/v) or 40% (v/v) or 50% (v/v) ethanol solution) to provide a final concentration of compound having the desired activity. This ensures the desired cosmetic or therapeutic activity. In another example, the or each C₁₀-monoterpenoid or ester or enantiomer thereof, or a perfume oil comprising same, as described according to any example hereof may be diluted in aqueous ethanol up to about 70% (v/v), including 10% (v/v) or 20% (v/v) or 30% (v/v) or 40% (v/v) or 50% (v/v) or 60% (v/v) or 70%(v/v). In another example, the or each C₁₀-monoterpenoid or ester or enantiomer thereof, or a perfume oil comprising same, as described according to any example hereof may be diluted in aqueous ethanol up to about 70% (w/v), including 10% (w/v) or 20% (w/v) or 30% (w/v) or 40% (w/v) or 50% (w/v) or 60% (w/v) or 70%(w/v). In yet another example, the or each C₁₀-monoterpenoid or ester or enantiomer thereof, or a perfume oil comprising same, as described according to any example hereof may be diluted in aqueous ethanol up to about 70% (w/w), including 10% (w/w) or 20% (w/w) or 30% (w/w) or 40% (w/w) or 50% (w/w) or 60% (w/w) or 70%(w/w).

The concentration of monoterpenoid compound in a formulation may vary depending upon a range of parameters *e.g.,* including whether or not the formulation is for prevention or therapy, the site to which the topical formulation is to be applied, the half-life of the monoterpenoid compound following administration of the formulation, the age, sex and weight of the subject to which the formulation is to be applied, and type of hair loss condition, if any, to which the subject is predispose or which is to be treated.

Standard procedures are employed to prepare the topical formulations, especially once an oil or paste or powder comprising the active compound(s) has been prepared. *See, e.g.,* Hardman, et al. (2001) Goodman and Gilman's The Pharmacological Basis of Therapeutics, McGraw-Hill, New York, N.Y.; Gennaro (2000) Remington: The Science and Practice of Pharmacy, Lippincott, Williams, and Wilkins, New York, N.Y.; Avis, et al. (eds.) (1993) Pharmaceutical Dosage Forms: Parenteral Medications, Marcel Dekker, NY; Lieberman, et al. (eds.) (1990) Pharmaceutical Dosage Forms: Tablets, Marcel Dekker, NY; Lieberman, et al. (eds.) (1990) Pharmaceutical Dosage Forms: Disperse Systems, Marcel Dekker, NY; Weiner and Kotkoskie (2000) Excipient Toxicity and Safety, Marcel Dekker, Inc., New York, N.Y.).

For example, the topical formulations may include one or more carriers, excipients or emollients suitable for topical administration *e.g.,* such as to the dermis or skin of a subject.

Excipients will typically be included to improve solubility and/or bioadhesion. Suitable excipients include solvents, co-solvents, emulsifiers, plasticizers, surfactants, thickeners, pH modifiers, emollients, antioxidants, and chelating agents, wetting agents, and water absorbing agents. Formulations may also include one or more additives, for example, dyes, colored pigments, pearlescent agents, deodorizers, and odor maskers.

Diluents or fillers increase the bulk of a solid dosage form so that a practical size is provided for compression of tablets or formation of beads and granules. Suitable diluents include, but are not limited to dicalcium phosphate dihydrate, calcium sulfate, lactose, sucrose, mannitol, sorbitol, cellulose, microcrystalline cellulose, kaolin, sodium chloride, dry starch, hydrolyzed starches, pregelatinized starch, silicone dioxide, titanium oxide, magnesium aluminum silicate and powdered sugar.

For topical use on the skin and the scalp, the formulation may comprise ointments, creams, liniments or patches as a carrier. These topical formulations may or may not contain preservatives, depending on the dispenser and nature of use. Suitable preservatives are described above. Various matrices for slow release delivery may also be used.

For topical use on the eyelids or eyebrows, the monoterpenoid or enantiomer or carboxylic acid derivative compound(s) may be formulated in aqueous alcohol solutions, creams, ointments or oils exhibiting physiologically acceptable osmolarity by addition of pharmacologically acceptable buffers and salts. Such topical formulations may or may not, depending on the dispenser, contain preservatives such as benzalkonium chloride, chlorhexidine, chlorobutanol, parahydroxybenzoic acids and phenylmercuric salts such as nitrate, chloride, acetate, and borate, or antioxidants, as well as additives like EDTA, sorbitol, boric acid etc. as additives. Furthermore, particularly aqueous solutions may contain viscosity increasing agents such as polysaccharides *e.g.,* methylcellulose, mucopolysaccharides, e.g., hyaluronic acid and chondroitin sulfate, or polyalcohol *e.g.,* polyvinylalcohol. Various slow releasing gels and matrices may also be employed as well as soluble and insoluble ocular inserts, for instance, based on substances forming *in-situ* gels. Depending on the actual formulation and specific monoterpenoid compound to be used, various amounts of the monoterpenoid compound and different dose regimens may be employed. Particularly preferred topical formulations are essential oils, perfume oils, or perfumes.

Topical formulations may also comprise one or more dispersants *e.g.,* phosphate-buffered saline (PBS), saline, glucose, sodium lauryl sulfate (SLS), polyvinylpyrrolidone (PVP), polyethylene glycol (PEG), and hydroxypropylmethylcellulose (HPMC).

Topical formulations may or may not contain stabilisers and/or preservatives to inhibit or retard drug decomposition reactions *e.g.,* by oxidation or bacterial action, depending on the dispenser and nature of use. Such preservatives include E216, E218, chlorobutanol hemihydrate, methyl-, propyl-, or butyl-parahydroxybenzoic acid, betain, chlorhexidine, benzalkonium chloride, and the like.

Topical formulations used in the invention may also comprise an emulsifying/solubilizing component comprising one or more of metallic alkyl sulfate, quaternary ammonium compounds, salts of fatty acids, sulfosuccinates, taurates, amino acids, lauroyl macrogol glycerides, caprylocaproyl macrogolglycerides, stearoyl macrogol glycerides, linoleoyl macrogol glycerides, oleoyl macrogol glycerides, polyalkylene glycol, polyethylene glycol, polypropylene glycol, polyoxyethylene-polyoxypropylene copolymer, polyoxyethylene fatty alcohol ether, fatty acid, polyethoxylated fatty acid ester, propylene glycol fatty acid ester, polyoxyethylene-glycerol fatty ester, polyglycolized glycerides, polyglycerol fatty acid ester, sorbitan ester, polyethoxylated sorbitan ester, polyethoxylated cholesterol, polyethoxylated castor oil, polyethoxylated sterol, lecithin, or polyethoxylated vegetable oil.

Topical formulations used in the invention may also comprise an anti-crystallization/solubilizing component which, when present, generally comprises one or more of metallic alkyl sulfate, polyvinylpyrrolidone, lauroyl macrogol glycerides, caprylocaproyl macrogolglycerides, stearoyl macrogol glycerides, linoleoyl macrogol glycerides, oleoyl macrogol glycerides, polyalkylene glycol, polyethylene glycol, polypropylene glycol, polyoxyethylene-polyoxypropylene copolymer, fatty alcohol, polyoxyethylene fatty alcohol ether, fatty acid, polyethoxylated fatty acid ester, propylene glycol fatty acid ester, fatty ester, glycerides of fatty acid, polyoxyethylene-glycerol fatty ester, polyglycolized glycerides, polyglycerol fatty acid ester, sorbitan ester, polyethoxylated sorbitan ester, polyethoxylated cholesterol, polyethoxylated castor oil, polyethoxylated sterol, lecithin, or polyethoxylated vegetable oil.

Topical formulations used in the invention may also comprise one or more surfactants. Surfactants may be anionic, cationic, amphoteric or nonionic surface active agents. Suitable anionic surfactants include, but are not limited to, those containing carboxylate, sulfonate and sulfate ions. Examples of anionic surfactants include sodium, potassium, ammonium of long chain alkyl sulfonates and alkyl aryl sulfonates such as sodium dodecylbenzene sulfonate; dialkyl sodium sulfosuccinates, such as sodium dodecylbenzene sulfonate; dialkyl sodium sulfosuccinates, such as sodium bis-(2-ethylthioxyl)-sulfosuccinate; and alkyl sulfates such as sodium lauryl sulfate. Cationic surfactants include, but are not limited to, quaternary ammonium compounds such as benzalkonium chloride, benzethonium chloride, cetrimonium bromide, stearyl dimethylbenzyl ammonium chloride, polyoxyethylene and coconut amine. Examples of nonionic surfactants include ethylene glycol monostearate, propylene glycol myristate, glyceryl monostearate, glyceryl stearate, polyglyceryl-4-oleate, sorbitan acylate, sucrose acylate, PEG-150 laurate, PEG-00 monolaurate, polyoxyethylene monolaurate, polysorbates, polyoxyethylene octylphenylether, PEG- 1000 cetyl ether, polyoxyethylene tridecyl ether, polypropylene glycol butyl ether, stearoyl monoisopropanolamide, and polyoxyethylene hydrogenated tallow amide. Examples of amphoteric surfactants include sodium N-dodecyl-β-alanine, sodium N-lauryl- β-iminodipropionate, myristoamphoacetate, lauryl betaine and lauryl sulfobetaine.

If desired, various matrices for slow release delivery of the monoterpenoid compound may also be used.

The topical formulations compositions described herein may further comprise components which are generally used in cosmetics, for example, oils, detergents, UV absorbers, alcohols, chelating agents, pH modifiers, preservatives, thickeners, pigments, fragrances, and skin nutritional supplements. Specifically, the composition may comprise active ingredients used for skin cosmetics, such as zinc oxide microparticles, titanium oxide, UV absorbers such as Parsol MCX and Parsol 1789, vitamins such as ascorbic acid, moisturising agents such as hyaluronate sodium, petrolatum, glycerin, and urea, hormonal agents, skin-lightening agents such as kojic acid, arbutin, placenta extract, and rucinol, steroid drugs, inhibitors of production or release of a chemical mediator such as arachidonate metabolite and histamine (e.g. indometacin and ibuprofen), anti-inflammatory drugs such as receptor antagonist, anti-androgenic agents, sebum secretion suppressing agents such as vitamin A acid, royal jelly extract, and royal jelly acid, peripheral blood-vessel dilators such as tocopherol nicotinate, alprostadil, isoxsuprine hydrochloride, and tolazoline hydrochloride, carbon dioxide with peripheral blood-vessel dilating activity, blood circulation promoting agents such as minoxidil, carpronium chloride, capsicum tincture, vitamin E variants, ginkgo extract, and Swertia japonica extract, cellular stimulants such as pentadecanoic acid glyceride and nicotinic-aid amide, antimicrobials such as hinokitiol, L-menthol, and isopropylmethylphenol, glycyrrhizinic acid and variants or salts thereof, ceramide and ceramide analogs.

Topical formulations used in the present invention may be compatible with various types of adjunctive agents with which they are combinable or capable of being administered sequentially or simultaneously or concomitantly. For example a topical formulation comprising one or more monoterpenoid compounds capable of reducing fibroblast growth factor 5 (FGF5)-dependent signalling in a hair follicle cell or part thereof may further comprise an adjunctive agent which is effective for treatment or prevention of hair loss. Alternatively, or in addition, a topical formulation comprising a monoterpenoid compound as hereinbefore described may be formulated for coadministration with one or more adjunctive agents effective for treatment or prevention of hair loss. In such circumstances, the efficacy of the monoterpenoid compound is preferably supplemented by the action of the adjunctive agent. For example, the topical formulation may comprise an adjunctive agent selected from the group consisting of estradiol, oxandrolone, minoxidil, *Sanguisorba officinalis* (also known as *Poterium officinale*) extract, *Rosa multiflora* extract, Brown algae extract, loquat leaf extract, Pecan shell extract, squill extract, sodium phytate, Fucus vesiculosus extract, phytic acid, nonanal, and Lipidure-C.

Alternatively, or in addition, a topical formulation used in the invention may further comprise one or more cellular stimulants, blood circulation promoting agents, anti-androgen drugs, sebum secretion suppressing agents, immunosuppressants, antihistamine agents, antimicrobials, focal stimulants, emollients, antiphlogistics, low-molecular anti-apoptotic agents, estradiol, oxandrolone, minoxidil or analog/variant thereof, pantothenic acid or variants thereof, placenta extract, photosensitizers, ginseng extract, biotin, mononitro guaiacol, carpronium chloride or hydrates thereof, vitamin E or variants thereof, *Swertia japonica* (also known as *Swertia chirata*) extract, capsicum tincture, cepharanthine, nicotinic acid or variants thereof, estradiol, ethynylestradiol, randic acid, 5α-reductase inhibitor, 12-tetradecanoylphorbol-13-acetate, herbal medicine such as *Polygonatum* rhizome, *Uncaria, Silybum marianum,* henna, *Glycyrrhiza,* estradiol benzoate, diphenhydramine, resorcin, hinokitiol, 1-menthol, salicylic acid, *Polygonum* root extract, *Panax japonicus* rhizome extract, panthenol, selenium disulfide, pyridoxine hydrochloride, dipyrithione zinc, pyrithione zinc, sulfur, piroctone olamine, pyrithione zinc, sulfur, glycyrrhetinic acid stearyl, glycyrrhizinate dipotassium, allantoin, dialkylmonoamine variants, *Perilla frutescens* extract, *Poria sclerotium* extract, β-glycyrrhetinic acid, miconazole nitrate, benzoic acid, sodium salicylate, phytosterol, wine yeast extract, takanal, ethinyl estradiol, isopropylmethylphenol, cepharanthine biotin, D-pantothenyl alcohol, *Paeonia* extract, *Tilia* extract, *Sophora* extract, *Sophora flavescens* extract, *Zingiber Officinale* (Ginger) root extract, 6-benzylaminoprine, pentadecanoic glyceride, t-flavanone, sweet *Hydrangea* leaf extract, adenosine, and pantothenylethylether.

It will be understood that the topical formulations of the present disclosure may comprise any one or more of the C₁₀-monoterpenoids or ester or enantiomer thereof, or perfume oils or essential oils comprising same as described according to any example hereof, in combination with one or more other components described herein e.g., carriers, excipients, emollients, diluents, fillers, dispersants, stabilisers, preservatives, emulsifying agents, solubilizing agents, anti-crystallization agents, surfactants, cosmetic components, and/or adjunctive agents.

For example, a preferred topical formulation in accordance with the present disclosure may comprise piperitone, *Rosa multiflora* extract, *Poterium officinale* extract, *Swertia chirata* extract, ethanol, 1,3-butylene glycol, panthenyl ethyl ether, glycyrrhetinic acid, citric acid anhydrous, sodium citrate and purified water.

Particularly preferred topical formulations are as follows:

**0.095% (v/v) Piperitone formulation**

| **Ingredient** | **% (w/v)** | **Amount (mg/ml)** |
|---|---|---|
| (-)-piperitone | 0.088 | |
| *Rosa multiflora* fruit extract (dry) | 1.0 (in solution) | 1.67 |
| *Poterium officinale* root extract (dry) | 1.0 (in solution) | 2.50 |
| *Swertia chirata* whole plant extract (dry) | 0.03 (in solution) | 3.60 |
| Ethanol | 60.0 | 600.00 |
| 1,3-Butylene Glycol | 3.0 | 30.00 |
| Panthenyl ethyl ether | 0.3 | 3.00 |
| Glycyrrhetinic acid | 0.1 | 1.00 |
| Citric acid anhydrous | 0.025 | 0.25 |
| Sodium citrate | 0.024 | 0.24 |
| Purified water | q.s. | |

**0.5% (v/v) Piperitone formulation**

| **Ingredient** | **% (w/v)** | **Amount (mg/ml)** |
|---|---|---|
| **(-)-piperitone** | 0.465 | |
| ***Rosa multiflora* fruit extract** | 1.0 (in solution) | 1.67 |
| ***Poterium officinale* root extract** | 1.0 (in solution) | 2.50 |
| ***Swertia chirata* whole plant extract** | 0.03 (in solution) | 3.60 |
| **Ethanol** | 60.0 | 600.00 |
| **1,3-Butylene Glycol** | 3.0 | 30.00 |
| **Panthenyl ethyl ether** | 0.3 | 3.00 |
| **Glycyrrhetinic acid** | 0.1 | 1.00 |
| **Citric acid anhydrous** | 0.025 | 0.25 |
| **Sodium citrate** | 0.024 | 0.24 |
| **Purified water** | q.s. | |

Other exemplary formulations in accordance with the present disclosure are described in the working examples hereof.

### Dosage units and frequency of administration

The dose of monoterpenoid compound in the topical formulation, and frequency of administration thereof, may be appropriately modified depending on the circumstances.

Typically, topical formulations used in the invention are applied repeatedly for a sustained period of time topically on the part of the body to be treated or which is susceptible to hair loss, for example, the eyelids, eyebrows, skin or scalp. The preferred dosage regimen will generally involve regular, such as daily, weekly, twice-weekly, or thrice-weekly, administration for a period of treatment of at least one about one month, more preferably at least three months, and most preferably at least six months as required to reduce and/or delay and/or prevent loss of terminal hair in the subject. For example, the monoterpenoid compound or topical formulation comprising same may be administered 1, 2, 3, 4, 5, 6 or 7 times per week, corresponding with one use per day that the monoterpenoid compound or topical formulation comprising same is applied. Alternatively, the topical formulation used in the invention may be administered to a subject daily or twice daily or every two days or every three days or every four days or every five days or every six days or weekly as required. On any day, the topical formulation may be administered 1, 2, 3, 4 or 5 times per day.

It is to be understood that terminal hair includes scalp hair, eyelash hair and/or eyebrow hair. Accordingly, the topical formulation may be administered to the scalp, eyelid, eyelash, face, forehead and/or eyebrow of a human or mammalian subject on which terminal hair would normally grow to reduce and/or delay and/or prevent loss of terminal hair. Alternatively, or in addition, the topical formulation may be administered to an area adjacent to the scalp, eyelid, eyelash, face, forehead and/or eyebrow of a human or mammalian subject in which terminal hair normally grows to reduce and/or delay and/or prevent loss of terminal hair.

The total amount of monoterpenoid compound in a topical formulation to be administered to the subject to reduce and/or delay and/or prevent loss of terminal hair will vary depending upon a range of parameters *e.g.,* the duration of cosmeticor therapeutic administration, the site to which the topical formulation is to be applied, the half-life of the specific monoterpenoid compound in the topical formulation following administration thereof, the age, sex and weight of the subject to which the topical formulation is to be administered, and the hair loss condition suffered by the subject or to which the subject is susceptible.

For example, a topical formulation in unit dose form may comprise an amount of each active C₁₀-monoterpenoid or ester or enantiomer thereof per unit dose sufficient to reduce FGF5-dependent signalling in a hair follicle cell *e.g.,* by reducing FGF-5 activity in the hair follicle or part thereof and/or by reducing binding of FGF-5 to its cognate receptor in the hair follicle or part thereof.

An amount of C₁₀-monoterpenoid or ester or enantiomer thereof in a unit dose of topical formulation is generally sufficient to reduce or inhibit FGF-5 activity in the hair follicle or part thereof. For example, the amount of C₁₀-monoterpenoid or ester or enantiomer thereof in a unit dose of the topical formulation may be sufficient to reduce or inhibit FGF-5 activity in the hair follicle or part thereof over the course of a treatment by about 10-90% or 20-90% or 30-90% or 40-90% or 50-90% or 60-90% or 70-90% or 80-90% or 10-80% or 20-80% or 30-80% or 40-80% or 50-80% or 60-80% or 70-80% or 10-70% or 20-70% or 30-70% or 40-70% or 50-70% or 60-70% or 10-60% or 20-60% or 30-60% or 40-60% or 50-60% or 10-50% or 20-50% or 30-50% or 40-50% or 10-40% or 20-40% or 30-40% or 10-30% or 20-30%. Preferably, an amount of C₁₀-monoterpenoid or ester or enantiomer thereof in a unit dose of the topical formulation is sufficient to reduce or inhibit FGF-5 activity in the hair follicle or part over the course of a treatment by at least 10% or at least 20% or at least 30% or at least 40% or at least 50% or at least 60% or at least 70% or at least 80% or at least 90%.

Alternatively, or in addition, an amount of C₁₀-monoterpenoid or ester or enantiomer thereof in a unit dose of topical formulation is generally sufficient to reduce or inhibit FGF-5 binding to a cognate fibroblast growth factor receptor (FGFR) in a hair follicle or part thereof. For example, the amount of C₁₀-monoterpenoid or ester or enantiomer thereof in a unit dose of the topical composition may be sufficient to reduce or inhibit FGF-5 binding to fibroblast growth factor receptor 1 (FGFR1) in a hair follicle or part thereof over the course of a treatment by about 10-90% or 20-90% or 30-90% or 40-90% or 50-90% or 60-90% or 70-90% or 80-90% or 10-80% or 20-80% or 30-80% or 40-80% or 50-80% or 60-80% or 70-80% or 10-70% or 20-70% or 30-70% or 40-70% or 50-70% or 60-70% or 10-60% or 20-60% or 30-60% or 40-60% or 50-60% or 10-50% or 20-50% or 30-50% or 40-50% or 10-40% or 20-40% or 30-40% or 10-30% or 20-30%. Preferably, an amount of C₁₀-monoterpenoid or ester or enantiomer thereof in a unit dose of the topical formulation is sufficient to reduce or inhibit FGF-5 binding to FGFR1 in a hair follicle or part thereof over the course of a treatment by at least 10% or at least 20% or at least 30% or at least 40% or at least 50% or at least 60% or at least 70% or at least 80% or at least 90%.

An ability of a C₁₀-monoterpenoid or ester or enantiomer thereof to reduce binding of FGF-5 to FGFR1 may be determined by a reduction in viability of a BaF3 cell expressing FGFR1, wherein the BaF3 cell is dependent on FGF-5 signalling for viability. For example, an amount of a compound required to reduce and/or inhibit and/or prevent binding of FGF-5 to FGFR1 may be determined using FR-BaF3 cell cultured in the presence of FGF-5 *e.g.,* such as described in Ito et al., Journal of Cellular Physiology, 197:273-283, 2003 or in the accompanying working examples.

Alternatively, or in addition, an amount of C₁₀-monoterpenoid or ester or enantiomer thereof in a unit dose of topical formulation is generally sufficient to sufficient to delay a hair follicle comprising terminal hair from entering catagen phase.

Alternatively, or in addition, an amount of C₁₀-monoterpenoid or ester or enantiomer thereof in a unit dose of topical formulation is generally sufficient to sufficient to extend an anagen phase of hair follicles comprising terminal hair.

A topical formulation used in the invention, including a unit dose thereof, may comprise at least about 0.01% (w/v), or at least about 0.05% (w/v), or at least about 0.1% (w/v), or at least about 0.25% (w/v), or at least about 0.5% (w/v), or at least about 0.75% (w/v), or at least about 1.0 % (w/v), or at least about 1.25% (w/v), or at least about 1.5% (w/v), or at least about 1.75% (w/v), or at least about 2.0% (w/v), or at least about 2.25% (w/v), or at least about 2.5% (w/v), or at least about 2.75% (w/v), or at least about 3.0% (w/v), or at least about 3.25% (w/v) or at least about 3.5% (w/v), or at least about 3.75% (w/v), or at least about 4.0% (w/v) or at least about 4.25% (w/v), or at least about 4.5% (w/v), or at least about 4.75% (w/v) or at least about 5.0% (w/v), or at least about 5.25% (w/v), or at least about 5.5% (w/v), or at least about 5.75% (w/v), or at least about 6.0% (w/v) or at least about 6.25% (w/v), or at least about 6.5% (w/v), or at least about 6.75% (w/v), or at least about 7.0% (w/v) of each C₁₀-monoterpenoid or ester or enantiomer thereof.

In a preferred example, a topical formulation used in the invention, including a unit dose thereof, comprises between 0.01-2.5% (w/v), or between 0.05-1.0% (w/v), or between 0.075-0.5% (w/v) of carveol or an ester or enantiomer thereof.

A topical formulation used in the invention, including a unit dose thereof, may comprise at least about 0.01% (v/v), or at least about 0.05% (v/v), or at least about 0.1% (v/v), or at least about 0.25% (v/v), or at least about 0.5% (v/v), or at least about 0.75% (v/v), or at least about 1.0 % (v/v), or at least about 1.25% (v/v), or at least about 1.5% (v/v), or at least about 1.75% (v/v), or at least about 2.0% (v/v), or at least about 2.25% (v/v), or at least about 2.5% (v/v), or at least about 2.75% (v/v), or at least about 3.0% (v/v), or at least about 3.25% (v/v) or at least about 3.5% (v/v), or at least about 3.75% (v/v), or at least about 4.0% (v/v) or at least about 4.25% (v/v), or at least about 4.5% (v/v), or at least about 4.75% (v/v) or at least about 5.0% (v/v), or at least about 5.25% (v/v), or at least about 5.5% (v/v), or at least about 5.75% (v/v), or at least about 6.0% (v/v) or at least about 6.25% (v/v), or at least about 6.5% (v/v), or at least about 6.75% (v/v), or at least about 7.0% (vv) of carveol or an ester or enantiomer thereof.

In a preferred example, a topical formulation used in the invention, including a unit dose thereof, comprises between 0.01-2.5% (v/v), or between 0.05-1.5% (v/v), or between 0.075-1.0% (n/v) or between 0.1-0.5% (v/v) of carveol or an ester or enantiomer thereof. For example, a topical formulation used in the invention comprises about 0.1% (v/v) *e.g.,* such as 0.095% (v/v), of carveol or an ester or enantiomer thereof. In another example, a topical formulation of the invention comprises about 0.5% (v/v) of carveol or an ester or enantiomer thereof.

A unit dosage of the composition will typically have a volume dependent on the formulation. For example, an essential oil or perfume is conveniently administered *e.g.,* as a spray, in an amount not exceeding about 1 or 2 or 3 or 4 or 5 ml per dose. A liquid formulation is conveniently administered in an amount not exceeding about 5 or 6 or 7 or 8 or 9 or 10 ml per dose, whereas a lotion or cream may be administered in a smaller volume *e.g.,* not exceeding about 1 or 2 or 3 or 4 or 5 ml per dose. For application to small areas such as the eyelash or eyebrow or eyelid, a much smaller volume *e.g.,* a 50µL or 100µL or 250µL or 500µL droplet, may be employed.

Exemplary unit dosages of up to about 10 ml volume may comprise each active C₁₀-monoterpenoid or ester or enantiomer thereof in a range from about 1µg to about 10000mg, or in a range from about 2µg to about 10000mg, or in a range from about 3µg to about 10000 mg, or in a range from about 4µg to about 10000 mg, or in a range from about 5µg to about 10000 mg, or in a range from about 6µg to about 10000 mg, or in a range from about 7µg to about 10000 mg, or in a range from about 8µg to about 10000 mg, or in a range from about 9µg to about 60000 mg, or in a range from about 10µg to about 10000 mg.

For example, a unit dose up to about 10 ml volume consisting essentially of a perfume classified as an eau, or a shampoo, conditioner, lotion or cream, may comprise each active C₁₀-monoterpenoid or ester or enantiomer thereof in a range from about 50µg to about 6000mg, or in a range from about 40µg to about 6000mg, or in a range from about 30µg to about 6000mg, or in a range from about 20µg to about 6000mg, or in a range from about 10µg to about 6000 mg, or in a range from about 50µg to about 5000mg, or in a range from about 50µg to about 4000 mg, or in a range from about 50µg to about 3000mg, or in a range from about 50µg to about 2000 mg, or in a range from about 50µg to about 1000mg, or in a range from about 50µg to about 1mg, or in a range from about 50µg to about 2mg, or in a range from about 50µg to about 3mg, or in a range from about 50µg to about 4mg, or in a range from about 50µg to about 5mg, or in a range from about 50µg to about 6mg, or in a range from about 50µg to about 7mg, or in a range from about 50µg to about 8mg, or in a range from about 50µg to about 9mg, or in a range from about 50µg to about 10mg, or in a range from about 500µg to about 10mg, or in a range from about 500µg to about 20mg, or in a range from about 500µg to about 30mg, or in a range from about 500µg to about 40mg, or in a range from about 500µg to about 50mg, or in a range from about 500µg to about 60mg, or in a range from about 500µg to about 70mg, or in a range from about 500µg to about 80mg, or in a range from about 500µg to about 90mg, or in a range from about 500µg to about 100mg, or in a range from about 1mg to about 100mg, or in a range from about 1mg to about 200mg, or in a range from about 1mg to about 300mg, or in a range from about 1mg to about 400mg, or in a range from about 1mg to about 500mg, or in a range from about 1mg to about 600mg, or in a range from about 1mg to about 700mg, or in a range from about 1mg to about 800mg, or in a range from about 1mg to about 900mg, or in a range from about 1mg to about 1000mg, or in a range from about 10mg to about 1000mg, or in a range from about 10mg to about 1000mg, or in a range from about 10mg to about 2000mg, or in a range from about 10mg to about 3000mg, or in a range from about 10mg to about 4000mg, or in a range from about 10mg to about 5000mg, or in a range from about 10mg to about 6000mg, or in a range from about 10mg to about 7000mg, or in a range from about 10mg to about 8000mg, or in a range from about 10mg to about 9000mg, or in a range from about 10mg to about 10000mg.

In general, a concentration of active compound in an essential oil will be about 10-fold to about 100-fold the concentration in an eau, and a concentration of active compound in a parfum will be about 4-fold to about 6.6-fold the concentration in present an eau. For example, a unit dosage of up to about 10 ml volume consisting essentially of essential oil or parfum may comprise each active C₁₀-monoterpenoid or ester or enantiomer thereof in a range from about 1mg to about 6000 mg, or in a range from about 1mg to about 5000 mg, or in a range from about 1mg to about 4000 mg, or in a range from about 1mg to about 3000 mg, or in a range from about 1mg to about 2000 mg, or in a range from about 1mg to about 1000 mg, or in a range from about 1mg to about 500 mg, or in a range from about 1mg to about 100 mg, or in a range from about 1mg to about 50 mg, or in a range from about 1mg to about 30 mg, or in a range from about 1mg to about 20 mg, or in a range from about 1mg to about 10 mg, or in a range from about 100mg to about 6000mg, or in a range from about 100mg to about 5000mg, or in a range from about 100mg to about 4000mg, or in a range from about 100mg to about 3000mg, or in a range from about 100mg to about 2000mg, or in a range from about 100mg to about 1000mg.

A total amount of monoterpenoid compound administered to a subject may be in a range from about 0.1 ng per day to about 100 mg per day or from about 1 ng per day to about 10 mg per day or from about 10 ng per day to about 1 mg per day.

An amount of active compound administered to the subject may be in a range from 0.001 µg/cm²/day to 1,000 µg/cm²/day or from 0.005 µg/cm²/day to 500 µg/cm²/day or from 0.01 µg/cm²/day to 100 µg/cm²/day or from 0.05 µg/cm²/day to 50 µg/cm²/day or from 0.1 µg/cm²/day to 10 µg/cm²/day.

An amount of active compound to be applied topically on the scalp is in the range of about 0.1 ng to about 100 mg per day, more preferably about 1 ng to about 10 mg per day, and most preferably about 10 ng to about 1 mg per day depending on the specific monoterpenoid compound and formulation comprising same.

A topical formulation used in the invention may be for administration alone or in combination with other active ingredients *e.g.,* sequentially or simultaneously or concomitantly with other drug compositions for therapy of the same or a different condition. For example, the other drug may be combined with a topical formulation used in the invention. Such other active ingredients may include *e.g.,* one or more cellular stimulants, blood circulation promoting agents, anti-androgen drugs, sebum secretion suppressing agents, immunosuppressants, antihistamine agents, antimicrobials, focal stimulants, emollients, antiphlogistics, or low-molecular anti-apoptotic agents. Specifically, the other active ingredients may include at least one of estradiol, oxandrolone, minoxidil or analogs/variants thereof, *Sanguisorba officinalis* root extract, *Rosa multiflora* extract, Brown algae extract, loquat leaf extract, Pecan shell extract, squill extract, sodium phytate, Fucus vesiculosus extract, phytic acid, nonanal, Lipidure-C, pantothenic acid or variants thereof, placenta extract, photosensitizers, ginseng extract, biotin, mononitro guaiacol, carpronium chloride or hydrates thereof, vitamin E or variants thereof, *Swertia japonica* extract, capsicum tincture, cepharanthine, nicotinic acid or variants thereof, estradiol, ethynylestradiol, randic acid, 5α-reductase inhibitor, 12-tetradecanoylphorbol-13-acetate, herbal medicine such as *Polygonatum* rhizome, *Uncaria, Silybum marianum,* henna, *Glycyrrhiza,* estradiol benzoate, diphenhydramine, resorcin, hinokitiol, 1-menthol, salicylic acid, *Polygonum* root extract, *Panax japo*nicus rhizome extract, panthenol, selenium disulfide, pyridoxine hydrochloride, dipyrithione zinc, pyrithione zinc, sulfur, piroctone olamine, pyrithione zinc, sulfur, glycyrrhetinic acid stearyl, glycyrrhizinate dipotassium, allantoin, dialkylmonoamine variants, *Perilla frutescens* extract, *Poria sclerotium* extract, β-glycyrrhetinic acid, miconazole nitrate, benzoic acid, sodium salicylate, phytosterol, wine yeast extract, takanal, ethinyl estradiol, isopropylmethylphenol, cepharanthine biotin, D-pantothenyl alcohol, *Paeonia* extract, *Tilia* extract, *Sophora* extract, *Sophora flavescens* extract, *Zingiber Officinale* (Ginger) root extract, 6-benzylaminoprine, pentadecanoic glyceride, t-flavanone, sweet *Hydrangea* leaf extract, adenosine, and pantothenylethylether.

In one example, a topical formulation comprising a monoterpenoid compound is administered sequentially or simultaneously with an adjunctive therapeutic agent for treatment of the same condition *e.g.,* estradiol and/or oxandrolone and/or minoxidil and/or finasteride or an agent that blocks the conversion of testosterone to dihydrotesterone. The adjunctive therapeutic agent is co-administered under conditions and in accordance to a standard treatment regime for that agent. The skilled artisan will appreciate that such treatment regimens provide enhanced therapeutic benefit to the patient, and may be more than additive in their effect.

Alternatively, or in addition, a topical formulation comprising a monoterpenoid compound is administered sequentially or simultaneously with a cytotoxic or cytostatic compound that causes hair loss *e.g.,* in the case of a subject undergoing chemotherapy or radiation therapy or treatment for HIV-1 infection or AIDS. In such circumstances, the efficacy of the monoterpenoid compound counteracts the hair-loss effect of the cytotoxic or cytostatic compound. The cytotoxic or cytostatic compound will generally be administered in accordance to a standard treatment regime for that agent.

### Subjects and medical indications

Topical formulations used in the present invention are suitable for administration to human and other mammalian subjects, including companion animals such as dogs and cats, and domestic animals such as horses, zoo animals such as felids, canids, bovids, ungulates and primates, or laboratory animals such as rodents, lagomorphs and primates.

The subject to which a topical formulation used in the invention is for administration may be a subject who wishes to maintain full, voluminous hair for cosmetic purposes by reducing and/or delaying and/or preventing loss and/or thinning of terminal hair. In such circumstances, the subject may be a subject who does not suffer from alopecia, but may suffer from loss and/or thinning of terminal hair. Alternatively, or in addition, the subject may have no visible symptoms of alopecia, but is genetically predisposed to develop hair thinning, hair loss or alopecia in the future. The topical formulations are also particularly useful for treating alopecia *e.g.,* an acute form of alopecia, alopecia areata or androgenic alopecia. Accordingly, the subject to which the topical formulation is to be administered may be human or other mammalian subject who is suffering from hair loss or hair thinning, or predisposed to alopecia *e.g.,* an acute form of alopecia, alopecia areata or androgenic alopecia, or hair loss.

The topical formulation used in the present invention is particularly suited for administration to a human or mammalian subject suffering from androgenic alopecia or who has a predisposition or familial history of androgenic alopecia. Administration of a topical formulation of the invention to the human or mammalian subject suffering from, or at risk of suffering from, alopecia may delay and/or reduce and/or prevent loss of terminal hair in the subject by delaying hair follicles comprising the terminal hair from entering catagen phase. Alternatively, or in addition, a topical formulation used in the invention for administration to the human or mammalian subject suffering from, or at risk of suffering from, alopecia may delay and/or reduce and/or prevent loss of terminal hair in the subject by extending an anagen phase of hair follicles comprising the terminal hair.

The present invention is also particularly suited for administration to a subject suffering from or at risk of suffering from an acute form of alopecia induced by an acute event selected from pregnancy, stress, illness, a cytotoxic agent, a cytostatic agent, and treatment with an agent which induces necrosis or apoptosis of hair follicles as a side-effect of therapy. The cytotoxic or cytostatic agents may be endogenous *e.g.,* as generated in response to stress, or may be exogenous *e.g.,* as administered during chemotherapy for treatment of cancer. subject undergoing chemotherapy or radiation therapy or treatment for HIV-1 infection or AIDS.

The present invention is particularly suited to treatment and/or prevention of alopecia in subjects that are either undergoing cancer treatment with a cytotoxic or cytostatic compound or with radiation therapy, subjects who are undergoing treatment for HIV-1 infection or AIDS with antiviral compound, or to whom such therapies has been prescribed. The subject may be treated before therapy with a cytotoxic or cytostatic or antiviral compound commences, or before and after such therapy has commenced. The present invention also provides for therapy with a topical formulation as described herein after treatment with a cytotoxic or cytostatic or antiviral compound has commenced. Cytotoxic, cytostatic and/or antiviral compounds which cause hair loss are known in the art.

For example, a subject may apply a topical formulation described herein as a fine line at the skin-eyelash border of each eyelid, and as a cream to the scalp, once a day several weeks *e.g.,* two weeks or three weeks, prior to the initiation of a chemotherapy regimen (*e.g.,* doxorubicin, cyclophosphamide, and paclitaxel, or 5-fluoruracil, leucovorin and oxaliplatin). The patient may continue applying the topical formulation throughout and after cessation of the chemotherapy regimen. The patient would not generally experience the total hair loss normally associated with chemotherapy, and may recover more rapidly when chemotherapy ceases. A few weeks after completion of the chemotherapy, the patient may stop applying the topical formulation. If hair is lost at this stage, treatment is resumed.

### EXAMPLES

### Example 1 -Extraction of essential oil from Eucalyptus dives by steam distillation

This example describes extraction of essential oils from *Eucalyptus dives* by steam distillation and an exemplary chemical composition for an essential oil of *Eucalyptus dives* as determined by gas chromatography.

Fresh *Eucalyptus dives* foliage is separated from a *Eucalyptus dives* plant and introduced into a distilling chamber through which steam is to be passed. The distilling chamber is configured to support the *Eucalyptus dives* foliage in a manner which exposes the leaves to steam when passed through the chamber. Steam is then generated with a temperature between 100-105°C *e.g.,* in a boiler, and passed through the distilling chamber containing the *Eucalyptus dives* leaves. As the steam contacts the leaves, the cells and vesicles containing essential oils are disrupted and the essential oils are released in the form of vapour. The vapour flow of essential oil and steam is directed to a condenser unit in which the vapour is condensed *e.g.,* by a water cooled jacket surrounding the condenser unit, to form a liquid distillate having an aqueous phase and an oil phase. The liquid distillate is directed into a collection vessel and the essential oil (oil phase) is separated from the hydrosol or aqueous portion (aqueous phase) according to the relative specific densities. The essential oil obtained from the steam distillation of *Eucalyptus dives* is collected and used in accordance with the invention.

An essential oil extracted from *Eucalyptus dives* by steam distillation as described *supra* may be tested for chemical composition by gas chromatography *e.g.,* to determine its suitability for use in the present invention. An exemplary chromatographic analysis for an essential oil of *Eucalyptus dives* showing a typical chemical composition thereof is presented below.

**Typical profile of major chemical constituents of essential of from Eucalyptus dives**

| **Chemical constituent** | **Amount (w/v)** |
|---|---|
| Piperitone | 39-55% |
| 1,8 cineole | trace |
| α-phellandrane | 15-25% |
| β-phellandrane | 1-4% |
| Globulol | 2-6% |
| Terpinene-4-ol | 3-5% |
| *para*-cymene | 0.1-10% |
| α-pinene | 0.1-2% |
| δ-terpinene | 0.5-3.5% |
| limonene | trace |

### Example 2 - Test compounds

This example demonstrates availability of exemplary isolated monoterpenoid compounds tested for suitability in performing the invention.

Test compounds were all commercially available. However, compounds derived from natural sources that are in a substantially-purified form are also contemplated.

| Test Compound | Source |
|---|---|
| Linalyl acetate | Tokyo Chemical Industry |
| Nonanal | Tokyo Chemical Industry |
| Linalool | Tokyo Chemical Industry |
| Geranyl acetate | Tokyo Chemical Industry |
| α-Terpineol | Tokyo Chemical Industry |
| 1-Carveol | Tokyo Chemical Industry |
| (-)-Terpinen-4-ol | Tokyo Chemical Industry |
| (+)-Terpinen-4-ol | Tokyo Chemical Industry |
| (±)-Terpinen-4-ol [(+) 50%, (-) 50%] | Tokyo Chemical Industry |
| Nerol | Tokyo Chemical Industry |
| (-)-Menthol | Tokyo Chemical Industry |
| beta-Citronellol | Tokyo Chemical Industry |
| Geraniol | Tokyo Chemical Industry |
| Piperitone ((-)-Piperitone) | Tokyo Chemical Industry |

### Example 3 - Effect of test compounds on FGF-5 signalling

This example demonstrates the preparation and use of a transgenic FR-Ba/F3 cell line for identifying compounds of the invention having FGF-5 modulatory activity.

Ba/F3 is a murine interleukin-3 (IL-3)-dependent pro-B cell line. IL3-independent Ba/F3 clones expressing FGFR-1 or FGFR-1c on their cell surface and exhibiting FGF-5-dependent proliferation (*e.g.,* Smedley et al., Neoplasia, 1:349-355, 1999; Demiroglu et al., Blood, 98:3778-3783, 2001; Ito et al., Journal of Cellular Physiology, 197:273-283, 2003), were employed to assay compounds for their ability to inhibit FGF-5 activity. That is, compounds were tested for their ability to modulate proliferation of FR-Ba/F3 cells in the presence of recombinant human FGF-5 (rhFGF-5) prepared according to Maeda et al., Nishi Nihon Hifuka, 69(1): 81-86, 2007.

Briefly, a suitable plasmid expressing FGFR-1c was introduced into human ER-Ba/F3 cells, and the cells were maintained in RPMI RPMI-1640 with 10% FBS and 1.3x10⁻¹⁷ M of murine IL-3 (Sigma, St. Louis, MI) containing antibiotic G418 sulfate (Promega, Medison, WI). The inventors compared proliferation of FR/BaF3 cells in culture medium containing IL3 or FGF-5 and a different concentration in the range from 0.005% (w/v) to 1.0% (w/v) of each test compound listed in Example 1. The FR-BaF3 cells were seeded at 5 x 10⁴/mL cells per well in 96-well micro-culture plates (Falcon) in RPMI-1640 culture medium containing 10% fetal bovine serum, 5µg/mL heparin. Recombinant human FGF-5 (rhFGF-5; 1µg/mL) was added to one group, and IL-3 (0.2 ng/mL) (Sigma) was added to another group. The test compounds were then introduced to the micro-culture plate wells. Total volume of media per well was 100µl. Control groups lacking test compound were also prepared for each of the IL-3-supplemented and FGF-5-supplemented samples. The plates were then incubated at 37°C in 5% CO₂ for 3 days.

After 3 days, colorimetric assays were performed using a Cell Counting Kit-8 (CCK-8) (Wako) according to the manufacturer's instructions to measure cell proliferation or suppression thereof by the test compound at varying concentrations. Briefly, 10µl (1:2 dilution) of the CCK-8 reagent was added to each micro-culture plate well after which time the plates were incubated at 37°C for 3 hours.. Absorbance at 450nm was then measured using a microplate reader. Cell viability and proliferation for the IL-3-supplemented and FGF-5-supplemented groups was calculated as a measure of the optical density (OD) of cells treated with a test compound relative to the OD of the respective untreated controls, excluding the OD of blank controls (media only). Dose response curves were plotted and the respective inhibitory concentrations (IC50) for the test compounds were determined.

Suppression of FGF-5-dependent cell proliferation and viability was observed in FR-BaF3 cells to which (-)-terpinen-4-ol, (+)-terpinen-4-ol, α-terpineol, linalyl acetate, geranyl acetate, linalool and 1-carveol were added (data presented in Figures 1(a)-1(h)). IC₅₀ values for each compound are presented in Table 2.

**Table 2.**

| Inhibitory activity of test compounds on FGF-5-dependent cell proliferation | | | |
|---|---|---|---|
| | **IC₅₀ (%)** | | **FGF-5 Specificity** |
| **Test Compound** | **FGF-5** | **IL-3** | **IL-1/FGF-5** |
| Linalyl acetate | 0.4 | 0.9 | 2.25 |
| Nonanal | 0.1 | 0.9 | 9 |
| Linalool | <0.03 | 0.9 | 30> (BR) |
| Geranyl acetate | 0.1 | 0.8 | 8 (BR) |
| α-Terpineol | ∼0.01 | 0.05 | 5> |
| 1-Carveol | 0.05 | 0.1 | 2 |
| (-)-4-Terpinen-4-ol | 0.5 | >1.0 | 2> |
| (+)-4-Terpinen-4-ol | 0.11 | >1.0 | 9.1> |
| Piperitone | 0.024 | 0.59 | 25 |
| (±)-4-Terpinen-4-ol | NT | NT | NT |
| Nerol | 1.0> | 1.0> | ND |
| (-)-Menthol | 1.0> | 1.0> | ND |
| beta-Citronellol | 1.0> | 1.0> | ND |
| Geraniol | 1.0> | 1.0> | ND |

| | | | |
|---|---|---|---|
| BR: less reproducible ND: not determined, NT: not tested. | | | |

From this result, the following test compounds were considered as potent FGF-5-inhibitory active substances:
- Linalyl acetate
- Nonanal
- α-Terpineol
- (-)-4-Terpinen-4-ol
- (+)-4-Terpinen-4-ol
- Piperitone,
- (±)-4-Terpinen-4-ol.

### Example 4 - Effect of test compounds on proliferation of DP cells as determined by Alkaline Phosphatase (ALP) activity

This example demonstrates the use of an alkaline phosphatase (ALP) activity assay for identifying compounds of the invention having the ability to modulate proliferation of dermal papillae (DP) cells, and preferably modulate anagen phase of the cell cycle thereof.

Dermal papilla cells were prepared according to the methods described in WO2013/105417. Briefly, hairs were pulled from the scalp of a healthy subject (32 year old male) showing normal non-alopecia hair growth using tweezers. Hair follicles with dermal papilla were thus obtained at a rate of approximately one for every 100 hairs pulled. The hair follicles with dermal papilla were separated from the hair using tweezers and transferred into a saline for inspection under a microscope. Under a microscope, each dermal papilla was isolated from a respective hair follicle using tweezers and a scalpel. Approximately 2000 isolated dermal papilla cells were then transferred to a 35mm cell culture dish together 1-2ml of cell culture medium, after which time the cells were cultured at 37°C to produce a primary cell culture of dermal papilla. The cell culture medium consisted of approximately 90% DMEM medium, 10% fetal bovine serum (final concentration: 10%), FGF2 (final concentration: 10ng/ml), and penicillin-streptomycin solution (final concentration: 1µg/ml) for every 2mL of medium. Cell culture conditions were maintained for more than 14 days until dermal papilla cells covered approximately 40% of the area of the 35mm culture dish, at which time subcultures were performed. To prepare the subcultures, the cell culture medium was removed from the 35mm culture dish and the adherent cells were washed once with 2ml of saline (at room temperature). 1ml Trypsin-EDTA solution (0.1% trypsin and 0.02% EDTA) was then added to the 35mm culture dish and left to stand for 4 minutes at 37°C. Dissociated dermal papilla cells were then recovered from the 35 mm dish, and seeded to a new 100mm dish, and cultured in media under the same conditions as for the primary cell culture described above.

When the dermal papilla cells covered approximately 70-80% of the area of each 100 mm culture dish, the sub-confluent cells were subcultured as before. The process was repeated until sufficient cells were obtained to screen compounds of interest.

Once sufficient DP cells were obtained, the cells were harvested to provide a cell suspension. To harvest the DP cells, the cell culture medium was removed from the culture dish, and the adherent cells were washed with saline at room temperature. Trypsin-EDTA solution was then added under conditions described *supra* to dissociate the cells from the cell culture dish. Approximately 5x10⁵ of the dissociated cells were resuspended in 500ml of assay medium containing 445 ml of DMEM medium, 50ml of 10% fetal calf serum and 5ml of antibiotic.

Aliquots of the cell suspension were transferred to the wells of cell culture dishes, and a single monoterpenoid listed in Example 1 *e.g.,* (-)-4-terpinen-4-ol, (+)-4-terpinen-4-ol, (±)-4-terpinen-4-ol, α-terpineol, nonanal, linalyl acetate or piperitone, is added to each well at a concentration of 0.005 (mg/ml) or 0.01 (mg/ml) or 0.05 (mg/ml) or 0.1 (mg/ml) or 0.5 (mg/ml). Cell suspensions were transferred to wells of a further cell culture dish to which minoxidil was then added at a concentration of 0.005 (mg/ml) or 0.01 (mg/ml) or 0.05 (mg/ml) or 0.1 (mg/ml) or 0.5 (mg/ml). A Wnt/β- catenin pathway activation agent *e.g.,* Wnt3a or GSK-3 Inhibitor IX, was added to each of the cell cultures to produce an "activated cell culture". Control cell cultures containing the same monoterpenoid compound or minoxodil, but lacking the Wnt/β- catenin pathway activation agent, were processed in parallel. These activated and control cell cultures were divided further into duplicate cell culture samples either lacking FGF-5 or to which recombinant human FGF-5 (rhFGF-5; 1µg/mL) was added. Table 3 illustrates the cell culture and sample design matrix as herein described.

The cell culture samples were each cultured at 37 °C for 3 days under atmosphere containing less than 5% CO₂. Colorimetric assays were performed using a Cell Counting Kit-8 (CCK-8) according to the manufacturer's instructions to measure cell proliferation of the DP cells. An assay control contained cell culture media in the absence of DP cells ("medium background"). Absorbances (450 nm) for each activated cell culture sample and the corresponding control cell culture sample were determined using a microplate reader, and the relative absorbance of each activated cell culture sample was determined by the following ratio: $\frac{\left(A450 {nm}_{activated cell culture}-A450{nm}_{medium background}\right)}{\left(A450 {nm}_{control cell culture}-A450{nm}_{medium background}\right)}$

Each activated cell culture sample and each control cell culture sample was then washed twice in saline at room temperature. The cells were lysed by freeze-thawing employing three freeze-thaw cycles. In each freeze-thaw cycle, the cell suspension was frozen for 5 minutes at -80°C and thawed by incubating cells for 5 minutes at room temperature. To the lysed cells, p-nitrophenyl phosphate (1mg/ml) in 1M diethanolamine buffer solution was added, and each lysate was incubated at 37 °C for 30 minutes. The relative absorbance (490 nm) of each activated cell culture sample to each corresponding control cell sample was determined as a relative measure of alkaline phosphatase (ALP) activity. Similar readings were obtained for p-nitrophenyl phosphate (1mg/ml) in 1M diethanolamine buffer solution lacking DP cells ("buffer background").

ALP activity was determined by the following ratio: $\frac{\left(A490 {nm}_{activated cell culture}-A490{nm}_{buffer background}\right)}{\left(A490 {nm}_{control cell culture}-A490{nm}_{medium background}\right) .}$

Table 3 demonstrates sample design for the DP-ALP assay described in the preceding paragraphs:

**Table 3**

| Sample matrix for DP-ALP assays | | | | | | | | | | | | |
|---|---|---|---|---|---|---|---|---|---|---|---|---|
| | **"FGF-5 group"** | | | | | | **"FGF-5-free group"** | | | | | |
| | **DP cell culture + GSK3 inhibitor IX + FGF5** | | | | | | **DP cell culture +GSK3 inhibitor IX** | | | | | |
| **Test compound** | **Approx. concentration (mg/ml)** | | | | | | **Approx. concentration (mg/ml)** | | | | | |
| **Linalyl acetate** | control | 0.005 | 0.01 | 0.05 | 0.1 | 0.5 | control | 0.005 | 0.01 | 0.05 | 0.1 | 0.5 |
| **Nonanal** | control | 0.005 | 0.01 | 0.05 | 0.1 | 0.5 | control | 0.005 | 0.01 | 0.05 | 0.1 | 0.5 |
| **α-Terpeneol** | control | 0.005 | 0.01 | 0.05 | 0.1 | 0.5 | control | 0.005 | 0.01 | 0.05 | 0.1 | 0.5 |
| **(-)-Terpinen-4-ol** | control | 0.005 | 0.01 | 0.05 | 0.1 | 0.5 | control | 0.005 | 0.01 | 0.05 | 0.1 | 0.5 |
| **(+)-Terpinen-4-ol** | control | 0.005 | 0.01 | 0.05 | 0.1 | 0.5 | control | 0.005 | 0.01 | 0.05 | 0.1 | 0.5 |
| **(±)-Terpinen-4-ol** | control | 0.005 | 0.01 | 0.05 | 0.1 | 0.5 | control | 0.005 | 0.01 | 0.05 | 0.1 | 0.5 |
| **Piperitone** | control | 0.005 | 0.01 | 0.05 | 0.1 | 0.5 | control | 0.005 | 0.01 | 0.05 | 0.1 | 0.5 |
| **Minoxidil** | control | 0.005 | 0.01 | 0.05 | 0.1 | 0.5 | control | 0.005 | 0.01 | 0.05 | 0.1 | 0.5 |

ALP activity was plotted as a function of the concentration of each test compound in the presence and absence of rhFGF-5 (Figures 2a, 3a, 4a, 5a, 6a, 7a, 8a and 9a). Normalised dose response curves were also obtained showing relative change in ALP activity following addition of the respective test compounds at different doses in the presence and absence of FGF-5 (Figures 2b, 3b, 4b, 5b, 6b, 7b, 8b and 9b). Data indicate that the test compounds Piperitone, (-)-terpinen-4-ol, (±)-terpinen-4-ol, α-terpineol, and (+)-terpinen-4-ol each increased relative ALP activity of DP cells treated FGF-5. Taken together with data from Example 2 hereof, this induced increase in ALP activity suggests that the respective test compounds inhibit FGF-5-dependent signalling and extend the anagen phase of the DP cell cycle and/or prevent DP cells from entering catagen. In contrast, minoxidil showed a poor ability to increase relative ALP activity in DP cells treated FGF-5 suggesting that minoxidil is a poor inhibitor of FGF-5-dependent signalling in DP cells.

The FGF-5-inhibitory activity of the test compounds, from most active to least active, as determined by DP-ALP assay, may be ranked as follows:

### Most active Least active Piperitone > (-)-terpinen-4-ol > (±)-terpinen-4-ol > α-terpineol > (+)-terpinen-4-ol > linalyl acetate

The inventors then repeated the DP-ALP assay described *supra* employing *Eucalyptus dives* essential oil extract.

ALP activity of DP cells treated with *Eucalyptus dives* extract was plotted as a function of the concentration in the presence and absence of rhFGF-5 (Figure 10a). A normalised dose response curve was also obtained showing relative change in ALP activity following addition of *Eucalyptus dives* extract at different doses in the presence and absence of FGF-5 (Figure 10b). The data indicate that *Eucalyptus dives* extract increased relative ALP activity of DP cells treated FGF-5, suggesting that *Eucalyptus dives* extract is able to inhibit FGF-5-dependent signalling and extend the anagen phase of the DP cell cycle and/or prevent DP cells from entering catagen.

Test compounds, and extracts comprising same, which have been shown herein to increase and/or restore ALP activity in DP cells treated with FGF-5 and inhibit FGF-5-signalling in DP cells are considered by the inventors to be effective for reducing hair loss and/or hair thinning associated with FGF-5 signalling in the dermal papilla, such as by preventing and/or delaying and/or reducing premature onset of catagen in the hair follicle, and even extending the anagen phase of the hair follicle thereby prolonging hair growth. Such compounds are also effective for treatment of alopecia.

### Example 5 - Compound formulations

This example describes formulation of monoterpenoids for use in performing the invention.

One or more test compound(s) identified in Example 2 and/or Example 3 is/are formulated with a topical carrier *e.g.,* as described in Remington 's Pharmaceutical Sciences, 21th Ed. Philadelphia, PA: Lippincott Williams & Wilkins, 2005, to produce a non-therapeutic topical formulation for treatment or prevention of hair loss or a non-therapeutic topical formulation for treatment of alopecia.

The topical formulation may contain the test compound(s) in any dose suitable for reducing and/or delaying and/or treating and/or preventing hair loss or hair thinning *e.g.,* as determined by testing in an animal model described herein.

In one example, the topical formulation contains the test compound in a dose of about 0.01% by weight of active compound per unit volume of topical formulation (w/v) or by volume of oil or perfume per unit volume of the topical formulation (v/v). In one example, the topical formulation contains the test compound in a dose of about 0.1% by weight of active compound per unit volume of topical formulation (w/v) or by volume of oil or perfume per unit volume of the topical formulation (v/v). In another example, the topical formulation contains the test compound in a dose of about 0.5% by weight of active compound per unit volume of topical formulation (w/v) or by volume of oil or perfume per unit volume of the topical formulation (v/v). In another example, the topical formulation contains the test compound in a dose of about 1.0% by weight of active compound per unit volume of topical formulation (w/v) or by volume of oil or perfume per unit volume of the topical formulation (v/v). In another example, the topical formulation contains the test compound in a dose of about 2.0% by weight of active compound per unit volume of topical formulation (w/v) or by volume of oil or perfume per unit volume of the topical formulation (v/v). In another example, the topical formulation contains the test compound in a dose of about 3.0% by weight of active compound per unit volume of topical formulation (w/v) or by volume of oil or perfume per unit volume of the topical formulation (v/v).

The topical formulation may additionally contain one or more other agents effective for reducing and/or delaying and/or treating and/or preventing hair loss or hair thinning *i.e.,* one or more "adjunctive agents". For example, a topical formulation may contain a primary agent identified in Example 2 and/or Example 3 as being capable of inhibiting FGF-5-signalling and/or binding FGF-5 and optionally any one or more of the adjunctive agents as illustrated in Table 4.

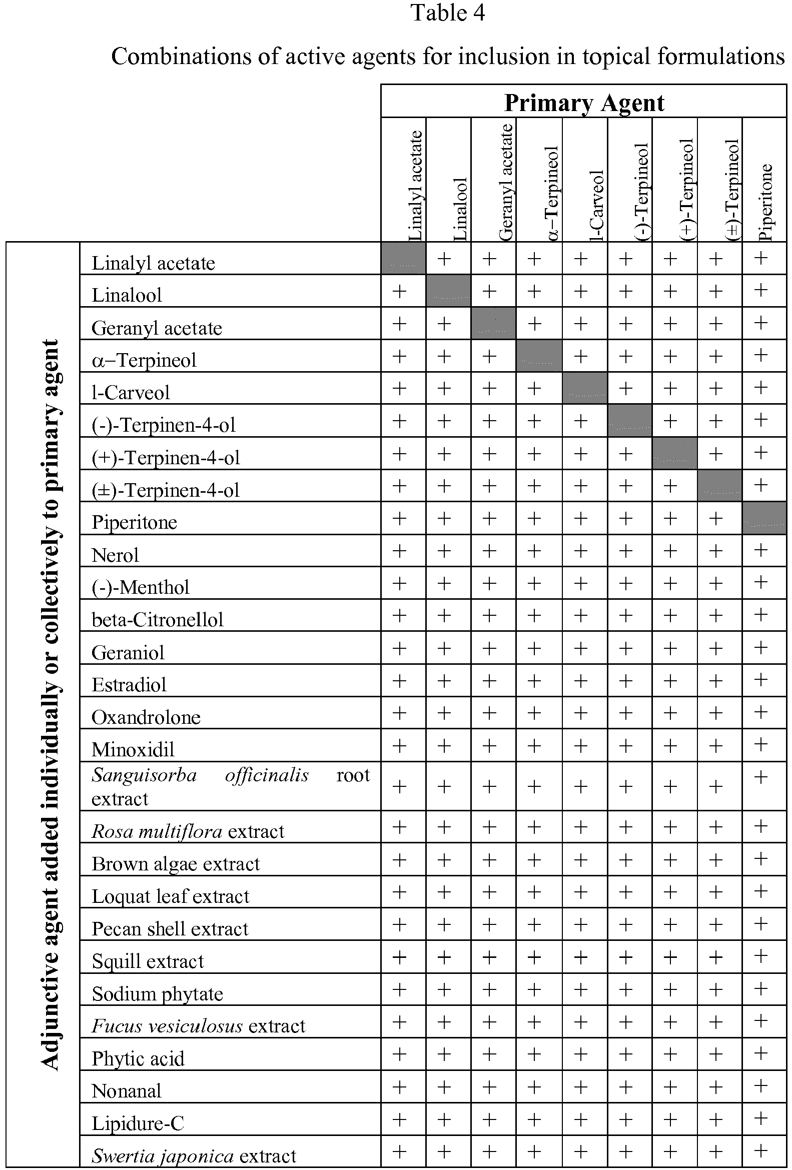

### Reference Example 6 - Shampoo formulation comprising (-)-terpinen-4-ol

This reference example describes a shampoo formulation comprising (-)-terpinen-4-ol.

An exemplary shampoo for use in accordance with the present disclosure comprises (-)-terpinen-4-ol, in a substantially pure form or as a constituent of an essential oil such as *E. dives,* as an active ingredient to inhibit FGF-5-dependent signalling in a hair follicle or part thereof. An exemplary shampoo comprising (-)-terpinen-4-ol may also comprise one or more of the following additional ingredients: purified water, sodium laureth sulfate, lauryl betaine, dipropylene glycol, lauramide DEA, glycol distearate, *Sanguisorba officinalis* root extract, *Rosa multiflora* fruit extract, *Swertia japonica* extract, chlorella vulgaris extract, *Moringa pterygosperma* seed extract, *Eucalyptus globulus* leaf extract, polyquaternium-64, polyquaternium-51, sodium lauroyl methylalanine, glycerol, polyquaternium-10, sorbitan stearate, polysorbate 80, PEG-5 stearate, dimethicone, laureth-2, citric acid, sodium citrate, butylene glycol, laureth-20, methylparaben, propylparaben, sodium salicylate, alcohol (ethanol), and fragrances.

### Reference Example 7 - Shampoo formulation comprising (±)-terpinen-4-ol

This reference example describes a shampoo formulation comprising (±)-terpinen-4-ol.

An exemplary shampoo for use in accordance with the present disclosure comprises (±)-terpinen-4-ol, in a substantially pure form or as a constituent of an essential oil such as from tea tree or sweet marjoram, as an active ingredient to inhibit FGF-5-dependent signalling in a hair follicle or part thereof. An exemplary shampoo comprising (±)-terpinen-4-ol may also comprise one or more of the following additional ingredients: purified water, sodium laureth sulfate, lauryl betaine, dipropylene glycol, lauramide DEA, glycol distearate, *Sanguisorba officinalis* root extract, *Rosa multiflora* fruit extract, *Swertia japonica* extract, chlorella vulgaris extract, *Moringa pterygosperma* seed extract, *Eucalyptus globulus* leaf extract, polyquaternium-64, polyquaternium-51, sodium lauroyl methylalanine, glycerol, polyquaternium-10, sorbitan stearate, polysorbate 80, PEG-5 stearate, dimethicone, laureth-2, citric acid, sodium citrate, butylene glycol, laureth-20, methylparaben, propylparaben, sodium salicylate, alcohol (ethanol), and fragrances.

### Reference Example 8 - Shampoo formulation comprising α-Terpineol

This reference example describes a shampoo formulation comprising α-Terpineol.

An exemplary shampoo for use in accordance with the present disclosure comprises α-Terpineol, in a substantially pure form or as a constituent of an essential oil such as from *Anthemis altissima* or clary sage or lavandin, as an active ingredient to inhibit FGF-5-dependent signalling in a hair follicle or part thereof. An exemplary shampoo comprising (α)-Terpineol may also comprise one or more of the following additional ingredients: purified water, sodium laureth sulfate, lauryl betaine, dipropylene glycol, lauramide DEA, glycol distearate, *Sanguisorba officinalis* root extract, *Rosa multiflora* fruit extract, *Swertia japonica* extract, chlorella vulgaris extract, *Moringa pterygosperma* seed extract, *Eucalyptus globulus* leaf extract, polyquaternium-64, polyquaternium-51, sodium lauroyl methylalanine, glycerol, polyquaternium-10, sorbitan stearate, polysorbate 80, PEG-5 stearate, dimethicone, laureth-2, citric acid, sodium citrate, butylene glycol, laureth-20, methylparaben, propylparaben, sodium salicylate, alcohol (ethanol), and fragrances.

### Reference Example 9 - Shampoo formulation comprising (+)-terpinen-4-ol

This reference example describes a shampoo formulation comprising (+)-terpinen-4-ol.

An exemplary shampoo for use in accordance with the present disclosure comprises (+)-terpinen-4-ol, in a substantially pure form or as a constituent of an essential oil such as from tea tree or marjoram or lavender, as an active ingredient to inhibit FGF-5-dependent signalling in a hair follicle or part thereof. An exemplary shampoo comprising (+)-terpinen-4-ol may also comprise one or more of the following additional ingredients: purified water, sodium laureth sulfate, lauryl betaine, dipropylene glycol, lauramide DEA, glycol distearate, *Sanguisorba officinalis* root extract, *Rosa multiflora* fruit extract, *Swertia japonica* extract, chlorella vulgaris extract, *Moringa pterygosperma* seed extract, *Eucalyptus globulus* leaf extract, polyquaternium-64, polyquaternium-51, sodium lauroyl methylalanine, glycerol, polyquaternium-10, sorbitan stearate, polysorbate 80, PEG-5 stearate, dimethicone, laureth-2, citric acid, sodium citrate, butylene glycol, laureth-20, methylparaben, propylparaben, sodium salicylate, alcohol (ethanol), and fragrances.

### Reference Example 10 - Shampoo formulation comprising linalyl acetate

This reference example describes a shampoo formulation comprising linalyl acetate.

An exemplary shampoo for use in accordance with the present disclosure comprises linalyl acetate, in a substantially pure form or as a constituent of an essential oil such as from bergamot or lavender or marjoram or lavandin or thyme or clary sage, as an active ingredient to inhibit FGF-5-dependent signalling in a hair follicle or part thereof. An exemplary shampoo comprising linalyl acetate may also comprise one or more of the following additional ingredients: purified water, sodium laureth sulfate, lauryl betaine, dipropylene glycol, lauramide DEA, glycol distearate, *Sanguisorba officinalis* root extract, *Rosa multiflora* fruit extract, *Swertia japonica* extract, chlorella vulgaris extract, *Moringa pterygosperma* seed extract, *Eucalyptus globulus* leaf extract, polyquaternium-64, polyquaternium-51, sodium lauroyl methylalanine, glycerol, polyquaternium-10, sorbitan stearate, polysorbate 80, PEG-5 stearate, dimethicone, laureth-2, citric acid, sodium citrate, butylene glycol, laureth-20, methylparaben, propylparaben, sodium salicylate, alcohol (ethanol), and fragrances.

### Reference Example 11 - Shampoo formulation comprising linalool

This reference example describes a shampoo formulation comprising linalool.

An exemplary shampoo for use in accordance with the present disclosure comprises linalool, in a substantially pure form or as a constituent of an essential oil such as from marjoram or lavender or bergamot or *Pelargonium* geranium, or neroli, as an active ingredient to inhibit FGF-5-dependent signalling in a hair follicle or part thereof. An exemplary shampoo comprising linalyl acetate may also comprise one or more of the following additional ingredients: purified water, sodium laureth sulfate, lauryl betaine, dipropylene glycol, lauramide DEA, glycol distearate, *Sanguisorba officinalis* root extract, *Rosa multiflora* fruit extract, *Swertia japonica* extract, chlorella vulgaris extract, *Moringa pterygosperma* seed extract, *Eucalyptus globulus* leaf extract, polyquaternium-64, polyquaternium-51, sodium lauroyl methylalanine, glycerol, polyquaternium-10, sorbitan stearate, polysorbate 80, PEG-5 stearate, dimethicone, laureth-2, citric acid, sodium citrate, butylene glycol, laureth-20, methylparaben, propylparaben, sodium salicylate, alcohol (ethanol), and fragrances.

### Reference Example 12 - Shampoo formulation comprising geranyl acetate

This reference example describes a shampoo formulation comprising geranyl acetate.

An exemplary shampoo for use in accordance with the present disclosure comprises geranyl acetate, in a substantially pure form or as a constituent of an essential oil such as from carrot seed or citronella or palmarosa, as an active ingredient to inhibit FGF-5-dependent signalling in a hair follicle or part thereof. An exemplary shampoo comprising geranyl acetate may also comprise one or more of the following additional ingredients: purified water, sodium laureth sulfate, lauryl betaine, dipropylene glycol, lauramide DEA, glycol distearate, *Sanguisorba officinalis* root extract, *Rosa multiflora* fruit extract, *Swertia japonica* extract, chlorella vulgaris extract, *Moringa pterygosperma* seed extract, *Eucalyptus globulus* leaf extract, polyquaternium-64, polyquaternium-51, sodium lauroyl methylalanine, glycerol, polyquaternium-10, sorbitan stearate, polysorbate 80, PEG-5 stearate, dimethicone, laureth-2, citric acid, sodium citrate, butylene glycol, laureth-20, methylparaben, propylparaben, sodium salicylate, alcohol (ethanol), and fragrances.

### Example 13 - Shampoo formulation comprising l-carveol

This example describes a shampoo formulation comprising *l*-carveol.

An exemplary shampoo for use in accordance with the invention comprises *l*-carveol, in a substantially pure form or as a constituent of an essential oil such as from spearmint or caraway seed, as an active ingredient to inhibit FGF-5-dependent signalling in a hair follicle or part thereof. An exemplary shampoo comprising *l*-carveol may also comprise one or more of the following additional ingredients: purified water, sodium laureth sulfate, lauryl betaine, dipropylene glycol, lauramide DEA, glycol distearate, *Sanguisorba officinalis* root extract, *Rosa multiflora* fruit extract, *Swertia japonica* extract, chlorella vulgaris extract, *Moringa pterygosperma* seed extract, *Eucalyptus globulus* leaf extract, polyquaternium-64, polyquaternium-51, sodium lauroyl methylalanine, glycerol, polyquaternium-10, sorbitan stearate, polysorbate 80, PEG-5 stearate, dimethicone, laureth-2, citric acid, sodium citrate, butylene glycol, laureth-20, methylparaben, propylparaben, sodium salicylate, alcohol (ethanol), and fragrances.

### Reference Example 14 - Shampoo formulation comprising piperitone

This reference example describes a shampoo formulation comprising *piperitone.*

An exemplary shampoo for use in accordance with the present disclosure comprises piperitone, in a substantially pure form or as a constituent of an essential oil such as from *Eucalyptus dives* or lemon grass or mint, as an active ingredient to inhibit FGF-5-dependent signalling in a hair follicle or part thereof. An exemplary shampoo comprising piperitone may also comprise one or more of the following additional ingredients: purified water, sodium laureth sulfate, lauryl betaine, dipropylene glycol, lauramide DEA, glycol distearate, *Sanguisorba officinalis* root extract, *Rosa multiflora* fruit extract, *Swertia japonica* extract, chlorella vulgaris extract, *Moringa pterygosperma* seed extract, *Eucalyptus globulus* leaf extract, polyquaternium-64, polyquaternium-51, sodium lauroyl methylalanine, glycerol, polyquaternium-10, sorbitan stearate, polysorbate 80, PEG-5 stearate, dimethicone, laureth-2, citric acid, sodium citrate, butylene glycol, laureth-20, methylparaben, propylparaben, sodium salicylate, alcohol (ethanol), and fragrances.

### Reference Example 15 - Tonic formulation comprising (-)-terpinen-4-ol

This reference example describes a tonic formulation comprising (-)-terpinen-4-ol.

An exemplary tonic for use in accordance with the present disclosure comprises (-)-terpinen-4-ol, in a substantially pure form or as a constituent of an essential oil such as from *Eucalyptus dives,* as an active ingredient to inhibit FGF-5-dependent signalling in a hair follicle or part thereof. An exemplary tonic may also comprise one or more of the following additional ingredients: purified water, ethanol, butylene glycol, panthenyl ethyl ether, *Swertia japonica* (or *Swertia chirata*) extract, glycyrrhetinic acid, citric acid, sodium citrate, maltodextrin, *Ginkgo biloba* extract, *Eriobotrya japonica* leaf extract, *Poterium officinale* root extract and *Rosa multiflora* fruit extract.

For example, an exemplary tonic formulation of the disclosure comprises (-)-terpinen-4-ol, purified water, ethanol, butylene glycol, *Poterium officinale* root extract, *Rosa multiflora* fruit extract, panthenyl ethyl ether, *Swertia japonica* (or *Swertia chirata*) extract, glycyrrhetinic acid, citric acid, sodium citrate, and maltodextrin, wherein the (-)-terpinen-4-ol is present in an amount between 0.01-3.0% or 0.01-0.3% by weight of active compound per unit volume of topical formulation (w/v) or by volume of essential oil per unit volume of the topical formulation (v/v).

For example, a formulation comprising both (-)-terpinen-4-ol and piperitone in suitable concentration ranges is prepared as a dilution of the essential oil from *E. dives* in a dilution range from about 1:10,000 (v/v) to about 1:33 (v/v), including 1:1,000 (v/v) or 1:500 (v/v) or 1:100 (v/v) or 1:50 (v/v).

### Reference Example 16 - Tonic formulation comprising (±)-terpinen-4-ol

This reference example describes a tonic formulation comprising (±)-terpinen-4-ol.

An exemplary tonic for use in accordance with the present disclosure comprises (±)-terpinen-4-ol, in a substantially pure form or as a constituent of an essential oil such as from lavender or other suitable source shown in Table 1, as an active ingredient to inhibit FGF-5-dependent signalling in a hair follicle or part thereof. An exemplary tonic formulation may also comprise one or more of the following additional ingredients: purified water, ethanol, butylene glycol, panthenyl ethyl ether, *Swertia japonica* (or *Swertia chirata*) extract, glycyrrhetinic acid, citric acid, sodium citrate, maltodextrin, *Ginkgo biloba* extract, *Eriobotrya japonica* leaf extract, *Poterium officinale* root extract and *Rosa multiflora* fruit extract.

For example, an exemplary tonic formulation of the disclosure comprises (±)-terpinen-4-ol, purified water, ethanol, butylene glycol, *Poterium officinale* root extract, *Rosa multiflora* fruit extract, panthenyl ethyl ether, *Swertia japonica* (or *Swertia chirata*) extract, glycyrrhetinic acid, citric acid, sodium citrate, and maltodextrin, wherein the (±)-terpinen-4-ol is present in an amount between 0.01-3.0% or 0.01-0.3% by weight of active compound per unit volume of topical formulation (w/v) or by volume of essential oil per unit volume of the topical formulation (v/v).

### Reference Example 17 - Tonic formulation comprising α-Terpineol

This reference example describes a tonic formulation comprising α-Terpineol.

An exemplary tonic for use in accordance with the present disclosure comprises α-Terpineol, in a substantially pure form or as a constituent of an essential oil such as from clary sage or other suitable source shown in Table 1, as an active ingredient to inhibit FGF-5-dependent signalling in a hair follicle or part thereof. An exemplary tonic formulation may also comprise one or more of the following additional ingredients: purified water, ethanol, butylene glycol, panthenyl ethyl ether, *Swertia japonica* (or *Swertia chirata*) extract, glycyrrhetinic acid, citric acid, sodium citrate, maltodextrin, *Ginkgo biloba* extract, *Eriobotrya japonica* leaf extract, *Poterium officinale* root extract and *Rosa multiflora* fruit extract.

For example, an exemplary tonic formulation of the disclosure comprises (α)-Terpineol, purified water, ethanol, butylene glycol, *Poterium officinale* root extract, *Rosa multiflora* fruit extract, panthenyl ethyl ether, *Swertia japonica* (or *Swertia chirata*) extract, glycyrrhetinic acid, citric acid, sodium citrate, and maltodextrin, wherein the (α)-Terpineol is present in an amount between 0.01-3.0% or 0.01-0.3% by weight of active compound per unit volume of topical formulation (w/v) or by volume of essential oil per unit volume of the topical formulation (v/v).

### Reference Example 18- Tonic formulation comprising (+)-terpinen-4-ol

This reference example describes a tonic formulation comprising (+)-terpinen-4-ol.

An exemplary tonic for use in accordance with the present disclosure comprises (+)-terpinen-4-ol, in a substantially pure form or as a constituent of an essential oil such as from tea tree or other suitable source shown in Table 1, as an active ingredient to inhibit FGF-5-dependent signalling in a hair follicle or part thereof. An exemplary tonic formulation may also comprise one or more of the following additional ingredients: purified water, ethanol, butylene glycol, panthenyl ethyl ether, *Swertia japonica* (or *Swertia chirata*) extract, glycyrrhetinic acid, citric acid, sodium citrate, maltodextrin, *Ginkgo biloba* extract, *Eriobotrya japonica* leaf extract, *Poterium officinale* root extract and *Rosa multiflora* fruit extract.

For example, an exemplary tonic formulation of the disclosure comprises (+)-terpinen-4-ol, purified water, ethanol, butylene glycol, *Poterium officinale* root extract, *Rosa multiflora* fruit extract, panthenyl ethyl ether, *Swertia japonica* (or *Swertia chirata*) extract, glycyrrhetinic acid, citric acid, sodium citrate, and maltodextrin, wherein the (+)-terpinen-4-ol is present in an amount between 0.01-3.0% or 0.01-0.3% by weight of active compound per unit volume of topical formulation (w/v) or by volume of essential oil per unit volume of the topical formulation (v/v).

### Reference Example 19- Tonic formulation comprising linalyl acetate

This reference example describes a tonic formulation comprising linalyl acetate.

An exemplary tonic for use in accordance with the present disclosure comprises linalyl acetate, in a substantially pure form or as a constituent of an essential oil such as from lavender or other suitable source shown in Table 1, as an active ingredient to inhibit FGF-5-dependent signalling in a hair follicle or part thereof. An exemplary tonic may also comprise one or more of the following additional ingredients: purified water, ethanol, butylene glycol, panthenyl ethyl ether, *Swertia japonica* (or *Swertia chirata*) extract, glycyrrhetinic acid, citric acid, sodium citrate, maltodextrin, *Ginkgo biloba* extract, *Eriobotrya japonica* leaf extract, *Poterium officinale* root extract and *Rosa multiflora* fruit extract.

For example, an exemplary tonic formulation of the disclosure comprises linalyl acetate, purified water, ethanol, butylene glycol, *Poterium officinale* root extract, *Rosa multiflora* fruit extract, panthenyl ethyl ether, *Swertia japonica* (or *Swertia chirata*) extract, glycyrrhetinic acid, citric acid, sodium citrate, and maltodextrin, wherein the linalyl acetate is present in an amount between 0.01-3.0% or 0.01-0.3% by weight of active compound per unit volume of topical formulation (w/v) or by volume of essential oil per unit volume of the topical formulation (v/v).

### Reference Example 20- Tonic formulation comprising linalool

This reference example describes a tonic formulation comprising linalool.

An exemplary tonic for use in accordance with the disclosure comprises linalool, in a substantially pure form or as a constituent of an essential oil such as from lavender or other suitable source shown in Table 1, as an active ingredient to inhibit FGF-5-dependent signalling in a hair follicle or part thereof. An exemplary tonic formulation may also comprise one or more of the following additional ingredients: purified water, ethanol, butylene glycol, panthenyl ethyl ether, *Swertia japonica* (or *Swertia chirata*) extract, glycyrrhetinic acid, citric acid, sodium citrate, maltodextrin, *Ginkgo biloba* extract, *Eriobotrya japonica* leaf extract, *Poterium officinale* root extract and *Rosa multiflora* fruit extract.

For example, an exemplary tonic formulation of the disclosure comprises linalool, purified water, ethanol, butylene glycol, *Poterium officinale* root extract, *Rosa multiflora* fruit extract, panthenyl ethyl ether, *Swertia japonica* (or *Swertia chirata*) extract, glycyrrhetinic acid, citric acid, sodium citrate, and maltodextrin, wherein the linalool is present in an amount between 0.01-3.0% or 0.01-0.3% by weight of active compound per unit volume of topical formulation (w/v) or by volume of essential oil per unit volume of the topical formulation (v/v).

### Reference Example 21- Tonic formulation comprising geranyl acetate

This reference example describes a tonic formulation comprising geranyl acetate.

An exemplary tonic for use in accordance with the present disclosure comprises geranyl acetate, in a substantially pure form or as a constituent of an essential oil such as from carrot seed or other suitable source shown in Table 1, as an active ingredient to inhibit FGF-5-dependent signalling in a hair follicle or part thereof. An exemplary tonic formulation may also comprise one or more of the following additional ingredients: purified water, ethanol, butylene glycol, panthenyl ethyl ether, *Swertia japonica* (or *Swertia chirata*) extract, glycyrrhetinic acid, citric acid, sodium citrate, maltodextrin, *Ginkgo biloba* extract, *Eriobotrya japonica* leaf extract, *Poterium officinale* root extract and *Rosa multiflora* fruit extract.

For example, an exemplary tonic formulation of the disclosure comprises geranyl acetate, purified water, ethanol, butylene glycol, *Poterium officinale* root extract, *Rosa multiflora* fruit extract, panthenyl ethyl ether, *Swertia japonica* (or *Swertia chirata*) extract, glycyrrhetinic acid, citric acid, sodium citrate, and maltodextrin, wherein the geranyl acetate is present in an amount between 0.01-3.0% or 0.01-0.3% by weight of active compound per unit volume of topical formulation (w/v) or by volume of essential oil per unit volume of the topical formulation (v/v).

### Example 22- Tonic formulation comprising l-carveol

This example describes a tonic formulation comprising *l*-carveol.

An exemplary tonic for use in accordance with the invention comprises *l*-carveol, in a substantially pure form or as a constituent of an essential oil such as from spearmint or other suitable source shown in Table 1, as an active ingredient to inhibit FGF-5-dependent signalling in a hair follicle or part thereof. An exemplary tonic formulation may also comprise one or more of the following additional ingredients: purified water, ethanol, butylene glycol, panthenyl ethyl ether, *Swertia japonica* (or *Swertia chirata*) extract, glycyrrhetinic acid, citric acid, sodium citrate, maltodextrin, *Ginkgo biloba* extract, *Eriobotrya japonica* leaf extract, *Poterium officinale* root extract and *Rosa multiflora* fruit extract.

For example, an exemplary tonic formulation of the invention comprises *l*-carveol, purified water, ethanol, butylene glycol, *Poterium officinale* root extract, *Rosa multiflora* fruit extract, panthenyl ethyl ether, *Swertia japonica* (or *Swertia chirata*) extract, glycyrrhetinic acid, citric acid, sodium citrate, and maltodextrin, wherein the *l-*carveol is present in an amount between 0.01-3.0% or 0.01-0.3% by weight of active compound per unit volume of topical formulation (w/v) or by volume of essential oil per unit volume of the topical formulation (v/v).

### Reference Example 23- Tonic formulation comprising piperitone

This reference example describes a tonic formulation comprising *piperitone.*

An exemplary tonic for use in accordance with the present disclosure comprises piperitone, in a substantially pure form or as a constituent of an essential oil such as from *Eucalyptus dives,* as an active ingredient to inhibit FGF-5-dependent signalling in a hair follicle or part thereof. An exemplary tonic formulation may also comprise one or more of the following additional ingredients: purified water, ethanol, butylene glycol, panthenyl ethyl ether, *Swertia japonica* (or *Swertia chirata*) extract, glycyrrhetinic acid, citric acid, sodium citrate, maltodextrin, *Ginkgo biloba* extract, *Eriobotrya japonica* leaf extract, *Poterium officinale* root extract and *Rosa multiflora* fruit extract.

For example, an exemplary tonic formulation of the disclosure comprises piperitone, purified water, ethanol, butylene glycol, *Poterium officinale* root extract, *Rosa multiflora* fruit extract, panthenyl ethyl ether, *Swertia japonica* (or *Swertia chirata*) extract, glycyrrhetinic acid, citric acid, sodium citrate, and maltodextrin, wherein the piperitone is present in an amount between 0.01-3.0% or 0.01-0.3% by weight of active compound per unit volume of topical formulation (w/v) or by volume of essential oil per unit volume of the topical formulation (v/v).

For example, a formulation comprising both (-)-terpinen-4-ol and piperitone in suitable concentration ranges is prepared as a dilution of the essential oil from *E. dives* in a dilution range from about 1:10,000 (v/v) to about 1:33 (v/v), including 1:1,000 (v/v) or 1:500 (v/v) or 1:100 (v/v) or 1:50 (v/v).

### Reference Example 24 - Perfume formulation comprising (-)-terpinen-4-ol

This reference example describes a perfume formulation comprising (-)-terpinen-4-ol.

An exemplary perfume for use in accordance with the present disclosure may comprise (-)-terpinen-4-ol, in a substantially pure form or as a constituent of an essential oil such as from *Eucalyptus dives,* formulated in an ethanol base comprising between 10% and 60% ethanol and purified water. The essential oil will be present in an amount in a range of 0.01% -10% (v/v) of the perfume formulation.

For example, a perfume comprising both (-)-terpinen-4-ol and piperitone in suitable concentration ranges is prepared as a dilution of the essential oil from *E. dives* in a dilution range from about 1:10,000 (v/v) to about 1:10 (v/v), including 1:1,000 (v/v) or 1:500 (v/v) or 1:100 (v/v) or 1:50 (v/v) or 1:20 (v/v).

A preferred perfume formulation will comprise (-)-terpinen-4-ol in an amount of at least about 0.01% by weight of active compound per unit volume of topical formulation (w/v) or by volume of oil per unit volume of the topical formulation (v/v), such as about 0.095% by weight of active compound per unit volume of topical formulation (w/v) or by volume of oil per unit volume of the topical formulation (v/v).

A perfume in accordance with the disclosure may also comprise one or more additional ingredients, such as one or more aromatic compounds and/or *Swertia japonica* (or *Swertia chirata*) extract.

### Reference Example 25 - Perfume formulation comprising (±)-terpinen-4-ol

This reference example describes a perfume formulation comprising (±)-terpinen-4-ol.

An exemplary perfume for use in accordance with the present disclosure may comprise (±)-terpinen-4-ol, in a substantially pure form or as a constituent of an essential oil such as tea tree oil, formulated in an ethanol base comprising between 10% and 60% ethanol and purified water. The essential oil will be present in an amount in a range of about 0.01% to about 10% (v/v) of the perfume formulation.

A preferred perfume formulation will comprise (±)-terpinen-4-ol in an amount of at least 0.01% by weight of active compound per unit volume of topical formulation (w/v) or by volume of oil per unit volume of the topical formulation (v/v), such as about 0.095% by weight of active compound per unit volume of topical formulation (w/v) or by volume of oil per unit volume of the topical formulation (v/v).

A perfume in accordance with the disclosure may also comprise one or more additional ingredients, such as one or more aromatic compounds and/or *Swertia japonica* (or *Swertia chirata*) extract.

### Reference Example 26 - Perfume formulation comprising α-Terpineol

This reference example describes a perfume formulation comprising α-Terpineol.

An exemplary perfume for use in accordance with the present disclosure may comprise α-Terpineol, in a substantially pure form or as a constituent of an essential oil such as clary sage oil, formulated in an ethanol base comprising between 10% and 60% ethanol and purified water. The essential oil will be present in an amount in a range of 0.01%-10% v/v of the perfume formulation.

A preferred perfume formulation will comprise (*α*)-Terpineol in an amount of at least about 0.01% by weight of active compound per unit volume of topical formulation (w/v) or by volume of oil per unit volume of the topical formulation (v/v), such as about 0.095% by weight of active compound per unit volume of topical formulation (w/v) or by volume of oil per unit volume of the topical formulation (v/v). A perfume in accordance with the disclosure may also comprise one or more additional ingredients, such as one or more aromatic compounds and/or *Swertia japonica* (or *Swertia chirata*) extract.

### Reference Example 27- Perfume formulation comprising (+)-terpinen-4-ol

This reference example describes a perfume formulation comprising (+)-terpinen-4-ol.

An exemplary perfume for use in accordance with the present disclosure may comprise (+)-terpinen-4-ol, in a substantially pure form or as a constituent of an essential oil such as tea tree oil, formulated in an ethanol base comprising between 10% and 60% ethanol and purified water. The essential oil will be present in an amount in a range of 0.01%-10% (v/v) of the perfume formulation.

A preferred perfume formulation will comprise (+)-terpinen-4-ol in an amount of at least about 0.01% by weight of active compound per unit volume of topical formulation (w/v) or by volume of oil per unit volume of the topical formulation (v/v), such as about 0.095% by weight of active compound per unit volume of topical formulation (w/v) or by volume of oil per unit volume of the topical formulation (v/v).

A perfume in accordance with the disclosure may also comprise one or more additional ingredients, such as one or more aromatic compounds and/or *Swertia japonica* (or *Swertia chirata*) extract.

### Reference Example 28- Perfume formulation comprising linalyl acetate

This reference example describes a perfume formulation comprising linalyl acetate.

An exemplary perfume for use in accordance with the present disclosure may comprise linalyl acetate, in a substantially pure form or as a constituent of an essential oil such as lavender oil, formulated in an ethanol base comprising between 10% and 60% ethanol and purified water. The essential oil will be present in an amount in a range of 0.01%-10% (v/v) of the perfume formulation.

A preferred perfume formulation will comprise linalyl acetate in an amount of at least about 0.01% by weight of active compound per unit volume of topical formulation (w/v) or by volume of oil per unit volume of the topical formulation (v/v), such as about 0.095% by weight of active compound per unit volume of topical formulation (w/v) or by volume of oil per unit volume of the topical formulation (v/v).

A perfume in accordance with the disclosure may also comprise one or more additional ingredients, such as one or more aromatic compounds and/or *Swertia japonica* (or *Swertia chirata*) extract.

### Reference Example 29- Perfume formulation comprising linalool

This reference example describes a perfume formulation comprising linalool.

An exemplary perfume for use in accordance with the disclosure may comprise linalool, in a substantially pure form or as a constituent of an essential oil such as lavender oil, formulated in an ethanol base comprising between 10% and 60% ethanol and purified water. The essential oil will be present in an amount in a range of 0.01%-10% (v/v) of the perfume formulation.

A preferred perfume formulation will comprise linalool in an amount of at least about 0.01% by weight of active compound per unit volume of topical formulation (w/v) or by volume of oil per unit volume of the topical formulation (v/v), such as about 0.095% by weight of active compound per unit volume of topical formulation (w/v) or by volume of oil per unit volume of the topical formulation (v/v).

A perfume in accordance with the disclosure may also comprise one or more additional ingredients, such as one or more aromatic compounds and/or *Swertia japonica* (or *Swertia chirata*) extract.

### Reference Example 30- Perfume formulation comprising geranyl acetate

This reference example describes a perfume formulation comprising geranyl acetate.

An exemplary perfume for use in accordance with the present disclosure may comprise geranyl acetate, in a substantially pure form or as a constituent of an essential oil such as carrot seed oil, formulated in an ethanol base comprising between 10% and 60% ethanol and purified water. The geranyl acetate or essential oil comprising same will be present in an amount in a range of 0.01%-10% (v/v) of the perfume formulation.

A preferred perfume formulation will comprise geranyl acetate in an amount of at least about 0.01% by weight of active compound per unit volume of topical formulation (w/v) or by volume of oil per unit volume of the topical formulation (v/v), such as about 0.095% by weight of active compound per unit volume of topical formulation (w/v) or by volume of oil per unit volume of the topical formulation (v/v).

A perfume in accordance with the disclosure may also comprise one or more additional ingredients, such as one or more aromatic compounds and/or *Swertia japonica* (or *Swertia chirata*) extract.

### Example 31- Perfume formulation comprising l-carveol

This example describes a perfume formulation comprising *l*-carveol.

An exemplary perfume for use in accordance with the invention may comprise *l-*carveol, in a substantially pure form or as a constituent of an essential oil such as spearmint oil, formulated in an ethanol base comprising between 10% and 60% ethanol and purified water. The essential oil will be present in an amount in a range of 0.01%-10% (v/v) of the perfume formulation.

A preferred perfume formulation will comprise *l*-carveol in an amount of at least about 0.01% by weight of active compound per unit volume of topical formulation (w/v) or by volume of oil per unit volume of the topical formulation (v/v), such as about 0.095% by weight of active compound per unit volume of topical formulation (w/v) or by volume of oil per unit volume of the topical formulation (v/v).

A perfume in accordance with the invention may also comprise one or more additional ingredients, such as one or more aromatic compounds and/or *Swertia japonica* (or *Swertia chirata*) extract.

### Reference Example 32- Perfume formulation comprising piperitone

This reference example describes a perfume formulation comprising piperitone.

An exemplary perfume for use in accordance with the present disclosure may comprise piperitone, in a substantially pure form or as a constituent of an essential oil from *Eucalyptus dives,* formulated in an ethanol base comprising between 10% and 60% ethanol and purified water. The essential oil will be present in an amount in a range of 0.01%-10% (v/v) of the perfume formulation.

For example, a perfume comprising both (-)-terpinen-4-ol and piperitone in suitable concentration ranges is prepared as a dilution of the essential oil from *E. dives* in a dilution range from about 1:10,000 (v/v) to about 1:10 (v/v), including 1:1,000 (v/v) or 1:500 (v/v) or 1:100 (v/v) or 1:50 (v/v) or 1:20 (v/v).

A preferred perfume formulation will comprise piperitone in an amount of at least about 0.01% by weight of active compound per unit volume of topical formulation (w/v) or by volume of oil per unit volume of the topical formulation (v/v), such as about 0.095% by weight of active compound per unit volume of topical formulation (w/v) or by volume of oil per unit volume of the topical formulation (v/v).

A perfume in accordance with the disclosure may also comprise one or more additional ingredients, such as one or more aromatic compounds and/or *Swertia japonica* (or *Swertia chirata*) extract.

### Example 33 - Testing of topical formulations in a rodent model of androgenic alopecia

This example shows exemplary means for testing efficacy of topical formulations used in the invention on therapy of androgenic alopecia.

One or more topical formulations described in the preceding examples is administered to a rodent model of androgenic alopecia described by Crabtree et al., Endocrinology, 151(5):2373-2380, 2010 (test groups). The topical formulations are applied twice daily to the dermis of mice in the respective test group for a period of 30 days or 60 days or 90 days or 120 days. Hair/fur loss and hair/fur growth is monitored throughout the application period to determine the effect of the topical formulations comprising test compound(s) used in the invention on hair loss and/or hair thinning in mice to which the formulation has been administered relative to a control group to which a placebo or control has been administered.

### Example 34 - Testing of formulations in a primate model of androgenic alopecia

This example shows exemplary means for testing efficacy of topical formulations used in the invention on therapy of androgenic alopecia.

One or more topical formulations described in the preceding examples is administered to a primate model of androgenic alopecia described by Brigham et al., Clinical Dermatology 6:177-187, 1998 and/or Sundberg et al., Experimental and Molecular Pathology 67:118-130, 1999. A topical formulation is applied to the dermis of the animal in the respective test group for a period of 30 days or 60 days or 90 days or 120 days. For example, a shampoo formulation as described may be administered to wet fur, massaged into the animal's skin and left for a period of 2-3 minutes, and washed off. Alternatively, a tonic formulation as described may be administered to fur 1-2 times per day, e.g., morning and evening, and massaged into the skin. Hair/fur loss and hair/fur growth is monitored throughout the test period to determine the effect of the topical formulation(s) on hair loss and/or hair thinning in those animals to which a topical formulation has been administered relative to a control group to which a placebo or control has been administered.

### Example 35 - Testing of topical formulations in a rodent model of alopecia areata

This example shows exemplary means for testing efficacy of topical formulations used in the invention on therapy of androgenic areata.

One or more topical formulations described in the preceding examples is administered to a C3H/HeJ mouse model of alopecia areata described by Sundberg et al., Journal of Investigative Dermatology, 102:847-56, 1994. A topical formulation is applied to the dermis of the animal in the respective test group for a period of 30 days or 60 days or 90 days or 120 days. For example, a shampoo formulation as described may be administered to wet fur, massaged into the animal's skin and left for a period of 2-3 minutes, and washed off. Alternatively, a tonic formulation as described is administered to fur 1-2 times per day, *e.g*., morning and evening, and massaged into the skin. Hair/fur loss and hair/fur growth is monitored throughout the test period to determine the effect of the topical formulation(s) on hair loss and/or hair thinning in those animals to which a topical formulation has been administered relative to a control group to which a placebo or control has been administered.

### Example 36 - Testing of topical formulations in a rodent model of acute alopecia

This example shows exemplary means for testing efficacy of topical formulations used in the invention on therapy of acute alopecia.

One or more topical formulations described in the preceding examples is administered to a C57 BL/6 mouse model of acute alopecia described by Paus et al., American Journal of Pathology 144:719-734, 1994. Adolescent, 6- to 8-week-old, female, syngeneic C 57 BL/6 mice (15 g to 20 g weight) with normal, black fur are housed in community cages with 12-hour light cycles and fed mouse chow and water *ad libitum.* Mice in telogen, as determined homogeneously pink color of their back skin, are depilated to induce a synchronized anagen, by applying a melted wax/rosin mixture to the back skin and by peeling off this mixture after hardening. About 9 days following depilation, the mice are then injected once intraperitoneally with 150 mg/kg body weight aqueous solution of cyclophosphamide, optionally with cyclosporine A administered intraperitoneally in 0.5 ml corn oil, at each of 7, 9 and 11 days post-depilation (250 mg/kg per dose) to prolong the testing period by delaying recovery from cyclophosphamide-induced alopecia. If required, animals are given a further injection of cyclophosphamide to extend the testing period. A topical formulation used in the invention as described according to any example hereof is applied to the dermis of the animal in the respective test group for a period of up to 30 days or up to 60 days or up to 90 days or up to 120 days. For example, a shampoo formulation as described may be administered to wet fur, massaged into the animal's skin and left for a period of 2-3 minutes, and washed off. Alternatively, a tonic formulation as described is administered to fur 1-2 times per day, e.g., morning and evening, and massaged into the skin. Skin color changes indicating the effect of test drugs on hair cycling and follicle melanogenesis, and hair regrowth, are monitored throughout the test period to determine the effect of the topical formulation(s) on hair loss and/or hair thinning in those animals to which a topical formulation has been administered relative to a control group to which a placebo or control has been administered. Mice are also sacrificed to permit histological analysis of follicle responses and recovery (morphometry) in the presence and absence of the topical formulations. Together, these data define a pattern of hair follicle response to the topical formulation(s), and monoterpenoid-mediated recovery from acute alopecia.

### Example 37 - Testing of topical formulations for non-therapeutic or cosmetic purposes

This example shows exemplary means for testing efficacy of topical formulations used in the invention on hair loss and/or hair thinning and/or hair volume when applied to the dermis of a human subject not suffering from alopecia.

One or more topical formulations described in the preceding examples is administered to a male or female subject (as appropriate) who is not suffering from alopecia. The topical formulation is applied to the scalp twice daily *e.g*., morning and evening, for a period of up to four months. For example, a shampoo formulation as described is administered to a male or female subject (as appropriate) who is not suffering from alopecia. To administer, the shampoo formulation is applied to wet hair, massaged into the scalp with fingertips and left on the scalp for a period of 2-3 minutes, after which time the shampoo is rinsed thoroughly from hair. This process is performed once daily throughout the test period. Alternatively, a tonic formulation is applied to the scalp twice daily *e.g*., morning and evening, throughout the test period, and after each application the tonic is massaged gently into the scalp. Hair loss, hair growth and hair volume is monitored throughout the four month period to determine the effect of the shampoo formulation on hair loss and/or hair thinning and/or hair volume in the subject to which the shampoo formulation is administered.

Hair growth rate, anagen/catagen ratio, hair shaft diameter are determined by Phototrichogram.

### Example 38 - Efficacy of topical formulations for treating hair loss in humans

This example demonstrates the efficacy of an exemplary topical formulation to: (1) reduce hair fall/loss; (2) increase hair growth; and/or (3) increase anagen:catagen ratio, in males and females suffering from male and female pattern baldness respectively.

### Trial design

The trial was designed as a randomised, single-blinded, placebo controlled clinical trial of a topically applied FGF-5 inhibiting lotion for treating hair loss.

### Trial cohort

A total of 20 adult subjects between the age of 25-55 having mild to moderate male and female pattern baldness were included in the trial.

The inclusion criteria for subjects were as follows:
- Subjects exhibited pattern baldness on Hamilton-Norwood scale 2 to 4 for men (Figure 11) or Ludwig scale 1-2 to II-2 for women (Figure 12) which was not complicated with other crucial hair disorders, such as alopecia areata (cyclic alopecia), loose anagen syndrome, acute anagen or telogen effluvium and *trichotillomania* etc. Based on confirmatory visual assessment by suitably trained medical practicitioners *e.g*., Staff MD's from AMA Laboratory Inc.;
- Subjects were healthy, non-obese and not undergoing or recently completing any medical interventions or using any medications, and not utilising any other hair loss treatment; and
- Subjects were within a healthy weight range for height *i.e.,* body mass index (BMI) between 19-26.

Subjects were excluded from the trial if they:
- were suffering from scalp inflammation or a skin condition, had known allergies to any lotion ingredients, were receiving any hair loss treatments currently or in the last 6 weeks prior to enrolment, were pregnant, breastfeeding or planning a pregnancy in next 6 months; and/or
- had undergone hypothyroidism or thyroid hormone treatment in the 6 weeks prior to enrolment.

### Trial methodology

Patients were randomised into two (2) sex- and age-matched groups (n=10/group; sex ratio was set to 1:1, *i.e.,* 5 males and 5 females, but skewed as far as 7:3 based on ability to recruit suitable subjects). Group 1 received a Placebo Formulation and Group 2 received a Test Formulation. In each case, the formulation was self-applied twice daily for two weeks.

The formulations were as follows:

**Placebo Formulation:**

| **Ingredient** | **% (w/v)** | **Amount (mg/ml)** |
|---|---|---|
| **Ethanol** | 60.0 | 600.00 |
| **Purified water** | q.s. | |

**Test Formulation: 0.095% (v/v) Piperitone**

| **Ingredient** | **% (w/v)** | **Amount (mg/ml)** |
|---|---|---|
| **(-)-piperitone** | 0.088 | |
| ***Rosa multiflora* fruit extract** | 1.0 (in solution) | 1.67 |
| ***Poterium officinale* root extract** | 1.0 (in solution) | 2.50 |
| ***Swertia chirata* whole plant extract** | 0.03 (in solution) | 3.60 |
| **Ethanol** | 60.0 | 600.00 |
| **1,3-Butylene Glycol** | 3.0 | 30.00 |
| **Panthenyl ethyl ether** | 0.3 | 3.00 |
| **Glycyrrhetinic acid** | 0.1 | 1.00 |
| **Citric acid anhydrous** | 0.025 | 0.25 |
| **Sodium citrate** | 0.024 | 0.24 |
| **Purified water** | q.s. | |

Subjects agreed to use the same shampoo and to maintain the same hair style, hair length and hair colour throughout the duration of the study, and to refrain from cutting the scalp hair shorter than 1 inch in length during that time. Subjects were evaluated for compliance by phone/email contact after the first week.

Self-assessment was performed by questionnaire prior to treatment and at days 7 and 14 of the study.

### Results

At day 7 of the study, subjects in Group 2 applying the Test Formulation perceived improved hair volume, reduced hair loss, stronger hair, thicker hair, improved hair density and strengthening of fine hair to a greater extent than those subjects in Group 1 applying the Placebo Formulation (Figures 13-16, 18 and 19 respectively). This trend continued at day 14, with the additional observation that subjects in in the Group 2 applying the Test Formulation perceived that hair fall was prevented to a greater extent relative to those subjects in Group 1 applying the Placebo Formulation (Figure 17). Based on the foregoing data, it was found that the topical application of Test Formulation 1 was effective for treating hair loss in subjects suffering from male pattern baldness and female pattern baldness.

### Example 39 - Efficacy of piperitone to increase hair growth in hair follicles from murine vibrissae ex vivo

### Methodology

### Preparation of follicles

Five week old male C3H mice (supplied by Japan SLC, Inc., Hamamatsu, Japan) were used for isolation of vibrissae follicles. The mice were sacrificed and the vibrissae follicles were carefully dissected from the mystacial pad. Briefly, the mystacial pad was cut into two sides (left and right). The skin cut, picked at the edge by tweezers, was washed in (i) 70% Ethanol for 30 seconds, (ii) PBS for 10 seconds, (iii) fresh PBS for 10 seconds, and (iv) another fresh PBS for 10 seconds. This washing process was repeated twice. After washing, the skin cut was placed inverted to expose the vibrissa follicles in Dulbecco's Modified Eagle's Medium (DMEM; Wako Pure Chemical, Osaka, Japan) at 37°C. Under a dissecting microscope, the surrounding tissue was removed from the follicles using tweezers, carefully so as not to destroy the structure of the follicles. The isolated follicles were then placed immediately into Williams' E medium (Life Technologies, Carlsbad, USA). Those follicles that exhibited fine growing fibers were then transected leaving 0.5 mm of the hair shaft from the frontier with the hair bulb.

From the isolated mouse vibrissae follicles, only early anagen follicles were selected and randomized into groups with 30 follicles per group. The anagen phase follicles were laid individually on 0.7 mm x 0.7 mm Gelfoam (Pfizer, New York, USA) submerged in 0.5 ml Williams' E medium supplemented with 30 µg/mL Insulin (Wako Pure Chemical), 10 ng/mL Hydrocortisone (Sigma-Aldrich, St. Louis, USA) and 2 mM GlutaMAX (Life Technologies) without any preservatives in a 24-well plate.

### FGF-5 cultures

A stock of FGF-5 solution (100 µg/mL) was prepared by dissolving 100 µg of FGF-5 protein (R&D Systems, Minneapolis, USA) in 1 ml of PBS (Takara Bio, Otsu, Japan). The stock solution was diluted further in culture medium to yield a culture medium with a final FGF-5 concentration of 300ng/mL. This culture medium was used for culturing follicles in the FGF-5 treatment group. In contrast, the follicles cultured in medium without the addition of FGF-5 served as controls.

Follicles in each of the treatment and control groups were incubated at 37°C at 5% CO₂ for 8 days, while exchanging the respective culture mediums every 2 days. From Day 1, elongation of hair shafts was observed and the elongation length was measured for each hair shaft every 24 hours from Day 1 to Day 8 using a micrometer under microscope. The follicles which showed apparently abnormal growth (extremely low or no) growth were excluded from the data set. Among the early anagen phase follicles selected for culture, almost 30% of them were qualified up to Day 8.

### Piperitone cultures

A stock of Piperitone solution with a concentration of 100 mg/ml was prepared by dissolving 100mg of Piperitone (Tokyo Chemical Industry, Tokyo, Japan) in 1 ml of Ethanol. The stock solution was diluted further in culture medium to yield a culture medium with a final Piperitone concentration of 0.1mg/mL. This culture medium was used for culturing follicles in the Piperitone treatment group. In contrast, the follicles cultured in medium without the addition of Piperitone served as controls.

Follicles in each of the treatment and control groups were incubated at 37°C at 5% CO₂ for 5 days, while exchanging the culture medium every 3 days. From Day 1, elongation of hair shafts was observed and the elongation length was measured for each hair shaft every 24 hours from Day 1 to Day 5, using a micrometer under microscope. The follicles which showed apparently abnormal growth (extremely low or no) growth were excluded from data set. Among the early anagen phase follicles selected for the culture, almost 30% of them were qualified up to Day 5.

### Results

### FGF-5 cultures

The addition of exogenous FGF-5 to follicle cultures was shown to slow the rate of hair shaft elongation over time (Figures 20 and 21). This is particularly apparent from the growth curves presented in Figures 20 and 21, which show that the rate of hair growth diminishes over time for those follicles cultured in the presence of FGF-5 relative to those follicles in the control group, particularly from days 5-8. This observation supports the inventor's theory that FGF-5-dependent signalling is important in the processes that lead to hair loss and/or thinning.

### Piperitone cultures

As is apparent from Figure 22, hair shaft elongation continued steadily and consistently throughout the culture period for those follicles cultured in medium containing piperitone. In contrast, the rate of hair shaft elongation for follicles in the control group declined from days 3-5 due to the uninhibited activity of endogenous FGF-5 secreted by the follicles. Based on these results, the inventors observed that the addition of piperitone to the culture medium increased hair growth by inhibiting the activity of endogenous FGF-5 secreted by the follicles.

It will be appreciated by persons skilled in the art that numerous variations and/or modifications may be made to the invention as shown in the specific embodiments without departing from the scope of the invention as broadly described. The present embodiments are, therefore, to be considered in all respects as illustrative and not restrictive.

## Claims

1. A topical formulation for use in a therapeutic method of treating or preventing hair loss in a subject, said topical formulation comprising 2-Methyl-5-(1-methylethenyl)-2-cyclohexen-1-ol (carveol) or an isolated enantiomer thereof or an isolated ester thereof with a carboxylic acid in an amount sufficient to reduce or inhibit fibroblast growth factor 5 (FGF5)-dependent signalling in a hair follicle cell of a subject to which the formulation is applied.

2. The topical formulation for use according to claim 1 comprising a carboxylic acid monoester of 2-Methyl-5-(1-methylethenyl)-2-cyclohexen-1-ol (carveol).

3. The topical formulation for use according to claim 2 wherein the carboxylic acid monoester of 2-Methyl-5-(1-methylethenyl)-2-cyclohexen-1-ol (carveol) is a monoester with a carboxylic acid selected from acetic acid, propionic acid and formic acid.

4. The topical formulation for use according to claim 3 wherein the carboxylic acid monoester of 2-Methyl-5-(1-methylethenyl)-2-cyclohexen-1-ol (carveol) is 5-Isopropenyl-2-methyl-2-cyclohexen-1-yl acetate (carvyl acetate).

5. The topical formulation for use according to claim 1 comprising an isolated enantiomer of 2-Methyl-5-(1-methylethenyl)-2-cyclohexen-1-ol (carveol).

6. The topical formulation for use according to claim 5, wherein the isolated enantiomer of 2-Methyl-5-(1-methylethenyl)-2-cyclohexen-1-ol (carveol) is selected from the group consisting of:
(*1R,5R*)-2-Methyl-5-(1-methylethenyl)-2-cyclohexen-1-ol [(-)-*cis*-carveol];
(*1S,5S*)-2-Methyl-5-(1-methylethenyl)-2-cyclohexen-1-ol [(+)-*cis*-carveol];
(*1R,5S*)-2-Methyl-5-(1-methylethenyl)-2-cyclohexen-1-ol [(+)-*trans*-carveol]; and
(*1S,5R*)-2-Methyl-5-(1-methylethenyl)-2-cyclohexen-1-ol [(-)-*trans*-carveol].

7. The topical formulation for use according to any one of claims 1 to 6 comprising a topical carrier, excipient or emollient and/or further comprising one or more adjunctive agents effective for treatment or prevention of hair loss.

8. The topical formulation for use according to any one of claims 1 to 7 wherein the method of treating or preventing hair loss comprises administering the topical formulation to an area of the dermis or skin of the subject comprising one or more hair follicle cells comprising terminal hair(s), or an area of dermis adjacent or surrounding thereto, to thereby extend an anagen phase of a hair follicle cell comprising the hair and/or delay the hair follicle cell comprising the hair from entering a catagen phase.

9. The topical formulation for use according to claim 8, wherein:
(i) the hair is scalp hair and the method of treating or preventing hair loss comprises administering the topical formulation to the scalp of the subject;
(ii) the hair is eyelash hair and the method of treating or preventing hair loss comprises administering the topical formulation to the eyelid or eyelash of the subject; and/or
(iii) the hair is eyebrow hair and the method of treating or preventing hair loss comprises administering the topical formulation to the face or forehead or eyebrow of the subject.

10. The topical formulation for use according to claim 8 or 9, wherein hair growth is promoted or enhanced.

11. The topical formulation for use according to any one of claims 1 to 7, wherein the hair loss is associated with alopecia.

12. The topical formulation for use according to claim 11, wherein:
(i) the alopecia involves scalp hair and the method comprises administering the topical formulation to the scalp of the subject;
(ii) the alopecia involves eyelash hair and the method comprises administering the topical formulation to the eyelid or eyelash of the subject; and/or
(iii) the alopecia involves eyebrow hair and the method comprises administering the topical formulation to the face or forehead or eyebrow of the subject.

13. 2-Methyl-5-(1-methylethenyl)-2-cyclohexen-1-ol (carveol) or an isolated enantiomer thereof or an isolated ester thereof with a carboxylic acid as defined in any one of claims 1 to 6 for use in the treatment or prevention of alopecia in a subject.

14. The 2-Methyl-5-(1-methylethenyl)-2-cyclohexen-1-ol (carveol) or the isolated enantiomer thereof or the isolated ester thereof with a carboxylic acid for use according to claim 13, wherein:
(i) the 2-Methyl-5-(1-methylethenyl)-2-cyclohexen-1-ol (carveol) or isolated enantiomer thereof or isolated ester thereof with a carboxylic acid is provided an amount sufficient to reduce or inhibit FGF-5 activity in the hair follicle or part thereof;
(ii) the 2-Methyl-5-(1-methylethenyl)-2-cyclohexen-1-ol (carveol) or isolated enantiomer thereof or isolated ester thereof with a carboxylic acid is provided in an amount sufficient to reduce or inhibit FGF-5 binding to a cognate fibroblast growth factor receptor (FGFR) in the hair follicle or part thereof; and/or
(iii) the 2-Methyl-5-(1-methylethenyl)-2-cyclohexen-1-ol (carveol) or isolated enantiomer thereof or isolated ester thereof with a carboxylic acid is provided with one or more adjunctive agents effective for treatment or prevention of hair loss.

15. A cosmetic method for treating or preventing hair loss comprising administering a topical formulation to a subject, wherein said topical formulation comprises 2-Methyl-5-(1-methylethenyl)-2-cyclohexen-1-ol (carveol) or an isolated enantiomer thereof or an isolated ester thereof with a carboxylic acid in an amount sufficient to reduce or inhibit fibroblast growth factor 5 (FGF5)-dependent signalling in a hair follicle cell of a subject to which the formulation is applied.

16. The cosmetic method of claim 15, wherein the topical formulation is a topical formulation as defined in any one of claims 2 to 7.

## Patentansprüche

1. Topische Formulierung zur Verwendung in einem therapeutischen Verfahren zur Behandlung oder Prävention von Haarausfall bei einem Patienten, wobei die topische Formulierung 2-Methyl-5-(1-methylethenyl)-2-cyclohexen-1-ol (Carveol) oder ein isoliertes Enantiomer davon oder einen isolierten Ester davon mit einer Carbonsäure in einer Menge umfasst, die ausreicht, um die Fibroblasten-Wachstumsfaktor 5- (FGF5-) abhängige Signalisierung in einer Haarfollikelzelle eines Patienten zu reduzieren oder zu inhibieren, bei dem die Formulierung angewendet wird.

2. Topische Formulierung zur Verwendung nach Anspruch 1, umfassend einen Carbonsäuremonoester von 2-Methyl-5-(1-methylethenyl)-2-cyclohexen-1-ol (Carveol).

3. Topische Formulierung zur Verwendung nach Anspruch 2, wobei der Carbonsäuremonoester von 2-Methyl-5-(1-methylethenyl)-2-cyclohexen-1-ol (Carveol) ein Monoester mit einer Carbonsäure ist, die aus Essigsäure, Propionsäure und Ameisensäure ausgewählt ist.

4. Topische Formulierung zur Verwendung nach Anspruch 3, wobei der Carbonsäuremonoester von 2-Methyl-5-(1-methylethenyl)-2-cyclohexen-1-ol (Carveol) 5-Isopropenyl-2-methyl-2-cyclohexen-1-ylacetat (Carvylacetat) ist.

5. Topische Formulierung zur Verwendung nach Anspruch 1, umfassend ein isoliertes Enantiomer von 2-Methyl-5-(1-methylethenyl)-2-cyclohexen-1-ol (Carveol) .

6. Topische Formulierung zur Verwendung nach Anspruch 5, wobei das isolierte Enantiomer von 2-Methyl-5-(1-methylethenyl)-2-cyclohexen-1-ol (Carveol) aus der Gruppe ausgewählt ist, bestehend aus:
(1R,5R)-2-Methyl-5-(1-methylethenyl)-2-cyclohexen-1-ol [(-)-cis-Carveol];
(1S,5S)-2-Methyl-5-(1-methylethenyl)-2-cyclohexen-1-ol [(+)-cis-Carveol];
(1R,5S)-2-Methyl-5-(1-methylethenyl)-2-cyclohexen-1-ol [(+)-trans-Carveol]; und
(1S,5R)-2-Methyl-5-(1-methylethenyl)-2-cyclohexen-1-ol [(-)-trans-Carveol].

7. Topische Formulierung zur Verwendung nach einem der Ansprüche 1 bis 6, umfassend einen topischen Träger, Exzipienten oder Weichmacher, und/oder ferner umfassend einen oder mehrere Zusatzstoffe, die zur Behandlung oder Prävention von Haarausfall wirksam sind.

8. Topische Formulierung zur Verwendung nach einem der Ansprüche 1 bis 7, wobei das Verfahren zur Behandlung oder Prävention von Haarausfall umfasst: Verabreichen der topischen Formulierung an einen Bereich der Dermis oder Haut des Patienten, umfassend eine oder mehrere Haarfollikelzellen, umfassend Endhaar(e), oder einen dort benachbarten oder umgebenden Bereich der Dermis, um dadurch eine Anagenphase einer Haarfollikelzelle, umfassend das Haar, zu verlängern, und/oder um zu verzögern dass die Haarfollikelzelle, umfassend das Haar, in eine Katagenphase eintritt.

9. Topische Formulierung zur Verwendung nach Anspruch 8, wobei:
(i) das Haar Kopfhaar ist, und das Verfahren zur Behandlung oder Prävention von Haarausfall das Verabreichen der topischen Formulierung an die Kopfhaut des Patienten umfasst;
(ii) das Haar Wimpernhaar ist, und das Verfahren zur Behandlung oder Prävention von Haarausfall das Verabreichen der topischen Formulierung an die Augenlider oder die Wimpern des Patienten umfasst; und/oder
(iii)das Haar Augenbrauenhaar ist, und das Verfahren zur Behandlung oder Prävention von Haarausfall das Verabreichen der topischen Formulierung an das Gesicht oder die Stirn oder die Augenbrauen des Patienten umfasst.

10. Topische Formulierung zur Verwendung nach Anspruch 8 oder 9, wobei das Haarwachstum gefördert oder verstärkt wird.

11. Topische Formulierung zur Verwendung nach einem der Ansprüche 1 bis 7, wobei der Haarverlust mit Alopecia assoziiert ist.

12. Topische Formulierung zur Verwendung nach Anspruch 11, wobei:
(i) die Alopecia das Kopfhaar betrifft, und das Verfahren das Verabreichen der topischen Formulierung an die Kopfhaut des Patienten umfasst;
(ii) die Alopecia Wimperhaar betrifft, und das Verfahren das Verabreichen der topischen Formulierung an die Augenlider oder Wimpern des Patienten umfasst; und/oder
(iii)die Alopecia Augenbrauenhaar betrifft, und das Verfahren das Verabreichen der topischen Formulierung an das Gesicht oder die Stirn oder die Augenbrauen des Patienten umfasst.

13. 2-Methyl-5-(1-methylethenyl)-2-cyclohexen-1-ol (Carveol) oder isoliertes Enantiomer davon oder isolierter Ester davon mit einer Carbonsäure, wie in einem der Ansprüche 1 bis 6 definiert, zur Verwendung bei der Behandlung oder Prävention von Alopecia bei einem Patienten.

14. 2-Methyl-5-(1-methylethenyl)-2-cyclohexen-1-ol (Carveol) oder isoliertes Enantiomer davon oder isolierter Ester davon mit einer Carbonsäure zur Verwendung nach Anspruch 13, wobei:
(i) das 2-Methyl-5-(1-methylethenyl)-2-cyclohexen-1-ol (Carveol) oder das isolierte Enantiomer davon oder der isolierter Ester davon mit einer Carbonsäure in einer Menge bereitgestellt wird, die ausreicht, um die FGF-5-Aktivität in dem Haarfollikel oder einem Teil davon zu reduzieren oder zu inhibieren;
(ii) das 2-Methyl-5-(1-methylethenyl)-2-cyclohexen-1-ol (Carveol) oder das isolierte Enantiomer davon oder der isolierter Ester davon mit einer Carbonsäure in einer Menge bereitgestellt wird, die ausreicht, um die FGF-5-Bindung an einen verwandten Fibroblastenwachstumsfaktor-Rezeptor (FGFR) in dem Haarfollikel oder einem Teil davon zu reduzieren oder zu inhibieren; und/oder
(iii)das 2-Methyl-5-(1-methylethenyl)-2-cyclohexen-1-ol (Carveol) oder das isolierte Enantiomer davon oder der isolierter Ester davon mit einer Carbonsäure mit einem oder mehreren Zusatzstoffen bereitgestellt wird, die zur Behandlung oder Prävention von Haarausfall wirksam sind.

15. Kosmetisches Verfahren zur Behandlung oder Prävention von Haarausfall, umfassend das Verabreichen der topischen Formulierung an einen Patienten, wobei die topische Formulierung 2-Methyl-5-(1-methylethenyl)-2-cyclohexen-1-ol (Carveol) oder ein isoliertes Enantiomer davon oder einen isolierten Ester davon mit einer Carbonsäure in einer Menge umfasst, die ausreicht, um die Fibroblasten-Wachstumsfaktor 5- (FGF5-) abhängige Signalisierung in einer Haarfollikelzelle eines Patienten zu reduzieren oder zu inhibieren, bei dem die Formulierung angewendet wird.

16. Kosmetisches Verfahren nach Anspruch 15, wobei die topische Formulierung eine topische Formulierung ist, wie in einem der Ansprüche 2 bis 7 definiert.

## Revendications

1. Formule topique pour utilisation dans une méthode thérapeutique de traitement ou de prévention de la chute de cheveux chez un sujet, ladite formule topique comprenant du 2-Méthyl-5-(1-méthyléthényl)-2-cyclohexén-1-ol (carvéol) ou un énantiomère isolé de celui-ci, ou un ester isolé de celui-ci avec un acide carboxylique à une teneur suffisante pour réduire ou inhiber la signalisation dépendant du facteur de croissance des fibroblastes 5 (FGF5) dans une cellule de follicule capillaire d'un sujet auquel la formule est appliquée.

2. Formule topique pour utilisation selon la revendication 1 comprenant un monoester d'acide carboxylique du 2-Méthyl-5-(1-méthyléthényl)-2-cyclohexén-1-ol (carvéol).

3. Formule topique pour utilisation selon la revendication 2 où le monoester d'acide carboxylique du 2-Méthyl-5-(1-méthyléthényl)-2-cyclohexén-1-ol (carvéol) est un monoester avec un acide carboxylique choisi parmi l'acide acétique, l'acide propionique et l'acide formique.

4. Formule topique pour utilisation selon la revendication 3 où le monoester d'acide carboxylique du 2-Méthyl-5-(1-méthyléthényl)-2-cyclohexén-1-ol (carvéol) est l'Acétate de 5-isopropényl-2-méthyl-2-cyclohexén-1-yle (acétate de carvyle).

5. Formule topique pour utilisation selon la revendication 1 comprenant un énantiomère isolé de 2-Méthyl-5-(1-méthyléthényl)-2-cyclohexén-1-ol (carvéol).

6. Formule topique pour utilisation selon la revendication 5, où l'énantiomère isolé de 2-Méthyl-5-(1-méthyléthényl)-2-cyclohexén-1-ol (carvéol) est choisi dans le groupe constitué par les suivants :
(*1R,5R*)-2-Méthyl-5-(1-méthyléthényl)-2-cyclohexén-1-ol [(-)-*cis*-carvéol] ;
(*1S,5S*)-2-Méthyl-5-(1-méthyléthényl)-2-cyclohexén-1-ol [(-)-*cis*-carvéol] ;
(*1R,5S*)-2-Méthyl-5-(1-méthyléthényl)-2-cyclohexén-1-ol [(+)-*trans*-carvéol] ;
et
(*1S,5R*)-2-Méthyl-5-(1-méthyléthényl)-2-cyclohexén-1-ol [(+)-*trans*-carvéol].

7. Formule topique pour utilisation selon l'une quelconque des revendications 1 à 6 comprenant un véhicule, excipient ou émollient topique et/ou comprenant en outre un ou plusieurs adjuvants efficaces dans le traitement ou la prévention de la chute de cheveux.

8. Formule topique pour utilisation selon l'une quelconque des revendications 1 à 7, où le procédé de traitement ou de prévention de la chute de cheveux comprend l'administration de la formule topique au niveau d'une zone du derme ou de la peau du sujet comprenant une ou plusieurs cellules de follicule capillaire comprenant un ou plusieurs cheveux terminaux, ou d'une zone du derme adjacente ou entourant celle-ci, pour prolonger de cette façon une phase anagénétique d'une cellule de follicule capillaire comprenant le cheveu et/ou retarder l'entrée dans une phase catagénétique de la cellule de follicule capillaire comprenant le cheveu.

9. Formule topique pour utilisation selon la revendication 8, où :
(i) le cheveu est un cheveu de cuir chevelu et la méthode de traitement ou de prévention de la chute de cheveux comprend l'administration de la formule topique au niveau du cuir chevelu du sujet ;
(ii) le cheveu est un cil et la méthode de traitement ou de prévention de la chute de cheveux comprend l'administration de la formule topique au niveau de la paupière ou du cil du sujet ; et/ou
(iii) le cheveu est un sourcil et la méthode de traitement ou de prévention de la chute de cheveux comprend l'administration de la formule topique au niveau du visage, du front ou du sourcil du sujet.

10. Formule topique pour utilisation selon la revendication 8 ou 9, où la croissance capillaire est favorisée ou améliorée.

11. Formule topique pour utilisation selon l'une quelconque des revendications 1 à 7, où la chute de cheveux est associée à l'alopécie.

12. Formule topique pour utilisation selon la revendication 11, où :
(i) l'alopécie implique les cheveux du cuir chevelu et la méthode comprend l'administration de la formule topique au cuir chevelu du sujet ;
(ii) l'alopécie implique les cils et la méthode comprend l'administration de la formule topique à la paupière ou au cil du sujet ; et/ou
(iii) l'alopécie implique les sourcils et la méthode comprend l'administration de la formule topique au visage, au front ou au sourcil du sujet.

13. 2-Méthyl-5-(1-méthyléthényl)-2-cyclohexén-1-ol (carvéol) ou énantiomère isolé de celui-ci ou ester isolé de celui-ci avec un acide carboxylique selon l'une quelconque des revendications 1 à 6 pour utilisation dans le traitement ou la prévention de l'alopécie chez un sujet.

14. 2-Méthyl-5-(1-méthyléthényl)-2-cyclohexén-1-ol (carvéol) ou énantiomère isolé de celui-ci ou ester isolé de celui-ci avec un acide carboxylique pour utilisation selon la revendication 13, où :
(i) le 2-Méthyl-5-(1-méthyléthényl)-2-cyclohexén-1-ol (carvéol) ou l'énantiomère isolé de celui-ci ou l'ester isolé de celui-ci avec un acide carboxylique est fourni à une teneur suffisante pour réduire ou inhiber l'activité de FGF-5 au niveau du follicule capillaire ou d'une partie de celui-ci ;
(ii) le 2-Méthyl-5-(1-méthyléthényl)-2-cyclohexén-1-ol (carvéol) ou l'énantiomère isolé de celui-ci ou l'ester isolé de celui-ci avec un acide carboxylique est fourni à une teneur suffisante pour réduire ou inhiber la liaison de FGF-5 à un récepteur de facteur de croissance des fibroblastes (FGFR) correspondant au niveau du follicule capillaire ou d'une partie de celui-ci ; et/ou
(iii) le 2-Méthyl-5-(1-méthyléthényl)-2-cyclohexén-1-ol (carvéol) ou l'énantiomère isolé de celui-ci ou l'ester isolé de celui-ci avec un acide carboxylique est fourni avec un ou plusieurs adjuvants efficaces dans le traitement ou la prévention de la chute de cheveux.

15. Méthode cosmétique de traitement ou de prévention de la chute de cheveux comprenant l'administration d'une formule topique à un sujet, où ladite formule topique comprend du 2-Méthyl-5-(1-méthyléthényl)-2-cyclohexén-1-ol (carvéol) ou un énantiomère isolé de celui-ci, ou un ester isolé de celui-ci avec un acide carboxylique à une teneur suffisante pour réduire ou inhiber la signalisation dépendant du facteur de croissance des fibroblastes 5 (FGF5) dans une cellule de follicule capillaire d'un sujet auquel la formule est appliquée.

16. Méthode cosmétique selon la revendication 15, où la formule topique est une formule topique selon l'une quelconque des revendications 2 à 7.
